# EUROPEAN PATENT SPECIFICATION

(11) **EP 2 155 243 B1**
(45) Date of publication and mention of the grant of the patent: **03.12.2014**
(21) Application number: 08754370.8
(22) Date of filing: 12.05.2008
(51) Int. Cl.: A61K 39/00, A61K 39/02, A61P 35/00, C07K 14/47, C07K 19/00

(54) **COMPOSITIONS AND METHODS COMPRISING KLK3, PSCA, OR FOLH1 ANTIGEN**
ZUSAMMENSETZUNGEN UND VERFAHREN MIT KLK3; PSCA ODER FOLH1-ANTIGEN
COMPOSITIONS ET PROCÉDÉS COMPRENANT DES ANTIGÈNES KLK3, PSCA OU FOLH1

(30) Priority: 10.05.2007 US 798177
(43) Date of publication of application: 24.02.2010
(73) Proprietor: The Trustees of the University of Pennsylvania, Philadelphia, PA 19104-6283 (US); Advaxis, North Brunswick, NJ 08902 (US)
(72) Inventor: PATERSON, Yvonne, Philadelphia, PA 19143 (US); ROTHMAN, John, Lebanon, NJ 08833 (US); SHAHABI, Vafa, Valley Forge, PA 19481 (US)
(74) Representative: Korn, Richard Mervyn
(86) International application number: PCT/US2008/006048
(87) International publication number: WO 2008/140812

(56) References cited:
- WO-A2-2005/071088
- WO-A2-2006/036550
- US-A1- 2003 220 239
- US-A1- 2004 058 342
- US-A1- 2006 104 991
- US-A1- 2006 210 540
- US-A1- 2006 223 835
- SEWELL D A ET AL: "REGRESSION OF HPV-POSITIVE TUMORS TREATED WITH A NEW LISTERIA MONOCYTOGENES VACCINE", ARCHIVES OF OTOLARYNGOLOGY HEAD AND NECK SURGERY, AMERICAN MEDICAL ASSOCIATION, US, vol. 130, no. 1, 1 January 2004 (2004-01-01), pages 92-97, XP009031081, ISSN: 0886-4470, DOI: DOI:10.1001/ARCHOTOL.130.1.92
- SEWELL D A ET AL: "Recombinant Listeria Vaccines Containing PEST Sequences Are Potent Immune Adjuvants for the Tumor-Associated Antigen Human Papillomavirus-16 E7", CANCER RESEARCH, AMERICAN ASSOCIATION FOR CANCER REREARCH, US, vol. 64, no. 24, 15 December 2004 (2004-12-15), pages 8821-8825, XP008119032, ISSN: 0008-5472
- FARZANA HUSSAIN S ET AL: "What is needed for effective antitumor immunotherapy? Lessons learned using Listeria monocytogenes as a live vector for HPV-associated tumors", CANCER IMMUNOLOGY, IMMUNOTHERAPY, SPRINGER, BERLIN, DE, vol. 54, no. 6, 1 June 2005 (2005-06-01), pages 577-586, XP019333127, ISSN: 1432-0851, DOI: DOI:10.1007/S00262-004-0600-2
- VAFA SHAHABI ET AL: "Development of a Listeria monocytogenes based vaccine against prostate cancer", CANCER IMMUNOLOGY, IMMUNOTHERAPY, SPRINGER, BERLIN, DE, vol. 57, no. 9, 14 February 2008 (2008-02-14), pages 1301-1313, XP019624387, ISSN: 1432-0851

### SEQUENCE LISTING

<110> The Trustees of the university of Pennsylvania
   ADVAXIS
<120> COMPOSITIONS AND METHODS COMPRISING KLK3, PSCA, OR FOLH1 ANTIGEN
<130> P-7769-PC2
<150> 11/798,177
   <151> 2007-05-10
<160> 64
<170> PatentIn version 3.3
<210> 1
   <211> 32
   <212> PRT
   <213> Listeria monocytogenes
<400> 1
   Lys Glu Asn Ser Ile Ser Ser Met Ala Pro Pro Ala Ser Pro Pro Ala
   1 5 10 15
   Ser Pro Lys Thr Pro Ile Glu Lys Lys His Ala Asp Glu Ile Asp Lys
   20 25 30
<210> 2
   <211> 14
   <212> PRT
   <213> Listeria monocytogenes
<400> 2
   Lys Thr Glu Glu Gln Pro Ser Glu Val Asn Thr Gly Pro Arg
   1 5 10
<210> 3
   <211> 28
   <212> PRT
   <213> Listeria monocytogenes
<400> 3
   Lys Ala Ser Val Thr Asp Thr Ser Glu Gly Asp Leu Asp Ser Ser Met
   1 5 10 15
   Gln Ser Ala Asp Glu Ser Thr Pro Gln Pro Leu Lys
   20 25
<210> 4
   <211> 20
   <212> PRT
   <213> Listeria monocytogenes
<400> 4
   Lys Asn Glu Glu Val Asn Ala Ser Asp Phe Pro Pro Pro Pro Thr Asp
   1 5 10 15
   Glu Glu Leu Arg
   20
<210> 5
   <211> 33
   <212> PRT
   <213> Listeria monocytogenes
<400> 5
   Arg Gly Gly Ile Pro Thr Ser Glu Glu Phe Ser Ser Leu Asn Ser Gly
   1               5                   10                  15
   Asp Phe Thr Asp Asp Glu Asn Ser Glu Thr Thr Glu Glu Glu Ile Asp
               20                  25                  30
   Arg
<210> 6
   <211> 17
   <212> PRT
   <213> Streptococcus pyogenes
<400> 6
   Lys Gln Asn Thr Ala Ser Thr Glu Thr Thr Thr Thr Asn Glu Gln Pro
   1               5                   10                  15
   Lys
<210> 7
   <211> 17
   <212> PRT
   <213> Streptococcus equisimilis
<400> 7
   Lys Gln Asn Thr Ala Asn Thr Glu Thr Thr Thr Thr Asn Glu Gln Pro
   1               5                   10                  15
   Lys
<210> 8
   <211> 22
   <212> DNA
   <213> Artificial
<220>
   <223> chemically synthesized
<400> 8
   ggctcgagca tggagataca cc 22
<210> 9
   <211> 28
   <212> DNA
   <213> Artificial
<220>
   <223> chemically synthesized
<400> 9
   ggggactagt ttatggtttc tgagaaca 28
<210> 10
   <211> 31
   <212> DNA
   <213> Artificial
<220>
   <223> chemically synthesized
<400> 10
   gggggctagc cctcctttga ttagtatatt c 31
<210> 11
   <211> 28
   <212> DNA
   <213> Artificial
<220>
   <223> chemically synthesized
<400> 11
   ctccctcgag atcataattt acttcatc 28
<210> 12
   <211> 55
   <212> DNA
   <213> Artificial
<220>
   <223> chemically synthesized
<400> 12
   gactacaagg acgatgaccg acaagtgata acccgggatc taaataaatc cgttt 55
<210> 13
   <211> 27
   <212> DNA
   <213> Artificial
<220>
   <223> chemically synthesized
<400> 13
   cccgtcgacc agctcttctt ggtgaag 27
<210> 14
   <211> 100
   <212> PRT
   <213> Listeria monocytogenes
<400> 14
   Met Arg Ala Met Met Val Val Phe Ile Thr Ala Asn Cys Ile Thr Ile
   1 5 10 15
   Asn Pro Asp Ile Ile Phe Ala Ala Thr Asp Ser Glu Asp Ser Ser Leu
   20 25 30
   Asn Thr Asp Glu Trp Glu Glu Glu Lys Thr Glu Glu Gln Pro Ser Glu
   35 40 45
   Val Asn Thr Gly Pro Arg Tyr Glu Thr Ala Arg Glu Val Ser Ser Arg
   50 55 60
   Asp Ile Lys Glu Leu Glu Lys Ser Asn Lys Val Arg Asn Thr Asn Lys
   65 70 75 80
   Ala Asp Leu Ile Ala Met Leu Lys Glu Lys Ala Glu Lys Gly Pro Asn
   85 90 95
   Ile Asn Asn Asn
   100
<210> 15
   <211> 390
   <212> PRT
   <213> Listeria monocytogenes
<400> 15
   Met Arg Ala Met Met Val Val Phe Ile Thr Ala Asn Cys Ile Thr Ile
   1               5                   10                  15
   Asn Pro Asp Ile Ile Phe Ala Ala Thr Asp Ser Glu Asp Ser Ser Leu
               20                  25                  30
   Asn Thr Asp Glu Trp Glu Glu Glu Lys Thr Glu Glu Gln Pro Ser Glu
           35                  40                  45
   Val Asn Thr Gly Pro Arg Tyr Glu Thr Ala Arg Glu Val Ser Ser Arg
       50                  55                  60
   Asp Ile Lys Glu Leu Glu Lys Ser Asn Lys Val Arg Asn Thr Asn Lys
   65                  70                  75                  80
   Ala Asp Leu Ile Ala Met Leu Lys Glu Lys Ala Glu Lys Gly Pro Asn
                   85                  90                  95
   Ile Asn Asn Asn Asn Ser Glu Gln Thr Glu Asn Ala Ala Ile Asn Glu
               100                 105                 110
   Glu Ala Ser Gly Ala Asp Arg Pro Ala Ile Gln Val Glu Arg Arg His
           115                 120                 125
   Pro Gly Leu Pro Ser Asp Ser Ala Ala Glu Ile Lys Lys Arg Arg Lys
       130                 135                 140
   Ala Ile Ala Ser Ser Asp Ser Glu Leu Glu Ser Leu Thr Tyr Pro Asp
   145 150 155 160
   Lys Pro Thr Lys Val Asn Lys Lys Lys Val Ala Lys Glu Ser Val Ala
   165 170 175
   Asp Ala Ser Glu Ser Asp Leu Asp Ser Ser Met Gln Ser Ala Asp Glu
   180 185 190
   Ser Ser Pro Gln Pro Leu Lys Ala Asn Gln Gln Pro Phe Phe Pro Lys
   195 200 205
   Val Phe Lys Lys Ile Lys Asp Ala Gly Lys Trp Val Arg Asp Lys Ile
   210 215 220
   Asp Glu Asn Pro Glu Val Lys Lys Ala Ile Val Asp Lys Ser Ala Gly
   225 230 235 240
   Leu Ile Asp Gln Leu Leu Thr Lys Lys Lys Ser Glu Glu Val Asn Ala
   245 250 255
   Ser Asp Phe Pro Pro Pro Pro Thr Asp Glu Glu Leu Arg Leu Ala Leu
   260 265 270
   Pro Glu Thr Pro Met Leu Leu Gly Phe Asn Ala Pro Ala Thr Ser Glu
   275 280 285
   Pro Ser Ser Phe Glu Phe Pro Pro Pro Pro Thr Asp Glu Glu Leu Arg
   290 295 300
   Leu Ala Leu Pro Glu Thr Pro Met Leu Leu Gly Phe Asn Ala Pro Ala
   305 310 315 320
   Thr Ser Glu Pro Ser Ser Phe Glu Phe Pro Pro Pro Pro Thr Glu Asp
   325 330 335
   Glu Leu Glu Ile Ile Arg Glu Thr Ala Ser Ser Leu Asp Ser Ser Phe
   340 345 350
   Thr Arg Gly Asp Leu Ala Ser Leu Arg Asn Ala Ile Asn Arg His Ser
   355 360 365
   Gln Asn Phe Ser Asp Phe Pro Pro Ile Pro Thr Glu Glu Glu Leu Asn
   370 375 380
   Gly Arg Gly Gly Arg Pro
   385 390
<210> 16
   <211> 1170
   <212> DNA
   <213> Listeria monocytogenes
<400> 16
   atgcgtgcga tgatggtggt tttcattact gccaattgca ttacgattaa ccccgacata      60
   atatttgcag cgacagatag cgaagattct agtctaaaca cagatgaatg ggaagaagaa     120
   aaaacagaag agcaaccaag cgaggtaaat acgggaccaa gatacgaaac tgcacgtgaa     180
   gtaagttcac gtgatattaa agaactagaa aaatcgaata aagtgagaaa tacgaacaaa     240
   gcagacctaa tagcaatgtt gaaagaaaaa gcagaaaaag gtccaaatat caataataac     300
   aacagtgaac aaactgagaa tgcggctata aatgaagagg cttcaggagc cgaccgacca     360
   gctatacaag tggagcgtcg tcatccagga ttgccatcgg atagcgcagc ggaaattaaa     420
   aaaagaagga aagccatagc atcatcggat agtgagcttg aaagccttac ttatccggat     480
   aaaccaacaa aagtaaataa gaaaaaagtg gcgaaagagt cagttgcgga tgcttctgaa     540
   agtgacttag attctagcat gcagtcagca gatgagtctt caccacaacc tttaaaagca     600
   aaccaacaac catttttccc taaagtattt aaaaaaataa aagatgcggg gaaatgggta     660
   cgtgataaaa tcgacgaaaa tcctgaagta aagaaagcga ttgttgataa aagtgcaggg     720
   ttaattgacc aattattaac caaaaagaaa agtgaagagg taaatgcttc ggacttcccg     780
   ccaccaccta cggatgaaga gttaagactt gctttgccag agacaccaat gcttcttggt     840
   tttaatgctc ctgctacatc agaaccgagc tcattcgaat ttccaccacc acctacggat     900
   gaagagttaa gacttgcttt gccagagacg ccaatgcttc ttggttttaa tgctcctgct     960
   acatcggaac cgagctcgtt cgaatttcca ccgcctccaa cagaagatga actagaaatc    1020
   atccgggaaa cagcatcctc gctagattct agttttacaa gaggggattt agctagtttg    1080
   agaaatgcta ttaatcgcca tagtcaaaat ttctctgatt tcccaccaat cccaacagaa    1140
   gaagagttga acgggagagg cggtagacca                                     1170
<210> 17
   <211> 529
   <212> PRT
   <213> Listeria monocytogenes
<400> 17
   Met Lys Lys Ile Met Leu Val Phe Ile Thr Leu Ile Leu Val Ser Leu
   1               5                   10                  15
   Pro Ile Ala Gln Gln Thr Glu Ala Lys Asp Ala Ser Ala Phe Asn Lys
               20                  25                  30
   Glu Asn Ser Ile Ser Ser Met Ala Pro Pro Ala Ser Pro Pro Ala Ser
           35                  40                  45
   Pro Lys Thr Pro Ile Glu Lys Lys His Ala Asp Glu Ile Asp Lys Tyr
       50                  55                  60
   Ile Gln Gly Leu Asp Tyr Asn Lys Asn Asn Val Leu Val Tyr His Gly
   65                  70                  75                  80
   Asp Ala Val Thr Asn Val Pro Pro Arg Lys Gly Tyr Lys Asp Gly Asn
   85 90 95
   Glu Tyr Ile Val Val Glu Lys Lys Lys Lys Ser Ile Asn Gln Asn Asn
   100 105 110
   Ala Asp Ile Gln Val Val Asn Ala Ile Ser Ser Leu Thr Tyr Pro Gly
   115 120 125
   Ala Leu Val Lys Ala Asn Ser Glu Leu Val Glu Asn Gln Pro Asp Val
   130 135 140
   Leu Pro Val Lys Arg Asp Ser Leu Thr Leu Ser Ile Asp Leu Pro Gly
   145 150 155 160
   Met Thr Asn Gln Asp Asn Lys Ile Val Val Lys Asn Ala Thr Lys Ser
   165 170 175
   Asn Val Asn Asn Ala Val Asn Thr Leu Val Glu Arg Trp Asn Glu Lys
   180 185 190
   Tyr Ala Gln Ala Tyr Pro Asn Val Ser Ala Lys Ile Asp Tyr Asp Asp
   195 200 205
   Glu Met Ala Tyr Ser Glu Ser Gln Leu Ile Ala Lys Phe Gly Thr Ala
   210 215 220
   Phe Lys Ala Val Asn Asn Ser Leu Asn Val Asn Phe Gly Ala Ile Ser
   225 230 235 240
   Glu Gly Lys Met Gln Glu Glu Val Ile Ser Phe Lys Gln Ile Tyr Tyr
   245 250 255
   Asn Val Asn Val Asn Glu Pro Thr Arg Pro Ser Arg Phe Phe Gly Lys
   260 265 270
   Ala Val Thr Lys Glu Gln Leu Gln Ala Leu Gly Val Asn Ala Glu Asn
   275 280 285
   Pro Pro Ala Tyr Ile Ser Ser Val Ala Tyr Gly Arg Gln Val Tyr Leu
   290 295 300
   Lys Leu Ser Thr Asn Ser His Ser Thr Lys Val Lys Ala Ala Phe Asp
   305 310 315 320
   Ala Ala Val Ser Gly Lys Ser Val Ser Gly Asp Val Glu Leu Thr Asn
   325 330 335
   Ile Ile Lys Asn Ser Ser Phe Lys Ala Val Ile Tyr Gly Gly Ser Ala
   340 345 350
   Lys Asp Glu Val Gln Ile Ile Asp Gly Asn Leu Gly Asp Leu Arg Asp
   355 360 365
   Ile Leu Lys Lys Gly Ala Thr Phe Asn Arg Glu Thr Pro Gly Val Pro
   370 375 380
   Ile Ala Tyr Thr Thr Asn Phe Leu Lys Asp Asn Glu Leu Ala Val Ile
   385 390 395 400
   Lys Asn Asn Ser Glu Tyr Ile Glu Thr Thr Ser Lys Ala Tyr Thr Asp
   405 410 415
   Gly Lys Ile Asn Ile Asp His Ser Gly Gly Tyr Val Ala Gln Phe Asn
   420 425 430
   Ile Ser Trp Asp Glu Val Asn Tyr Asp Pro Glu Gly Asn Glu Ile Val
   435 440 445
   Gln His Lys Asn Trp Ser Glu Asn Asn Lys Ser Lys Leu Ala His Phe
   450 455 460
   Thr Ser Ser Ile Tyr Leu Pro Gly Asn Ala Arg Asn Ile Asn Val Tyr
   465 470 475 480
   Ala Lys Glu Cys Thr Gly Leu Ala Trp Glu Trp Trp Arg Thr Val Ile
   485 490 495
   Asp Asp Arg Asn Leu Pro Leu Val Lys Asn Arg Asn Ile Ser Ile Trp
   500 505 510
   Gly Thr Thr Leu Tyr Pro Lys Tyr Ser Asn Lys Val Asp Asn Pro Ile
   515 520 525
   Glu
<210> 18
   <211> 441
   <212> PRT
   <213> Listeria monocytogenes
<400> 18
   Met Lys Lys Ile Met Leu Val Phe Ile Thr Leu Ile Leu Val Ser Leu
   1 5 10 15
   Pro Ile Ala Gln Gln Thr Glu Ala Lys Asp Ala Ser Ala Phe Asn Lys
   20 25 30
   Glu Asn Ser Ile Ser Ser Val Ala Pro Pro Ala Ser Pro Pro Ala Ser
   35 40 45
   Pro Lys Thr Pro Ile Glu Lys Lys His Ala Asp Glu Ile Asp Lys Tyr
       50                  55                  60
   Ile Gln Gly Leu Asp Tyr Asn Lys Asn Asn Val Leu Val Tyr His Gly
   65                  70                  75                  80
   Asp Ala Val Thr Asn Val Pro Pro Arg Lys Gly Tyr Lys Asp Gly Asn
                   85                  90                  95
   Glu Tyr Ile Val Val Glu Lys Lys Lys Lys Ser Ile Asn Gln Asn Asn
               100                 105                 110
   Ala Asp Ile Gln Val Val Asn Ala Ile Ser Ser Leu Thr Tyr Pro Gly
           115                 120                 125
   Ala Leu Val Lys Ala Asn Ser Glu Leu Val Glu Asn Gln Pro Asp Val
       130                 135                 140
   Leu Pro Val Lys Arg Asp Ser Leu Thr Leu Ser Ile Asp Leu Pro Gly
   145                 150                 155                 160
   Met Thr Asn Gln Asp Asn Lys Ile Val Val Lys Asn Ala Thr Lys Ser
                   165                 170                 175
   Asn Val Asn Asn Ala Val Asn Thr Leu Val Glu Arg Trp Asn Glu Lys
               180                 185                 190
   Tyr Ala Gln Ala Tyr Ser Asn Val Ser Ala Lys Ile Asp Tyr Asp Asp
           195                 200                 205
   Glu Met Ala Tyr Ser Glu Ser Gln Leu Ile Ala Lys Phe Gly Thr Ala
       210                 215                 220
   Phe Lys Ala Val Asn Asn Ser Leu Asn Val Asn Phe Gly Ala Ile Ser
   225                 230                 235                 240
   Glu Gly Lys Met Gln Glu Glu Val Ile Ser Phe Lys Gln Ile Tyr Tyr
                   245                 250                 255
   Asn Val Asn Val Asn Glu Pro Thr Arg Pro Ser Arg Phe Phe Gly Lys
               260                 265                 270
   Ala Val Thr Lys Glu Gln Leu Gln Ala Leu Gly Val Asn Ala Glu Asn
           275                 280                 285
   Pro Pro Ala Tyr Ile Ser Ser Val Ala Tyr Gly Arg Gln Val Tyr Leu
       290                 295                 300
   Lys Leu Ser Thr Asn Ser His Ser Thr Lys Val Lys Ala Ala Phe Asp
   305                 310                 315                 320
   Ala Ala Val Ser Gly Lys Ser Val Ser Gly Asp Val Glu Leu Thr Asn
   325 330 335
   Ile Ile Lys Asn Ser Ser Phe Lys Ala Val Ile Tyr Gly Gly Ser Ala
   340 345 350
   Lys Asp Glu Val Gln Ile Ile Asp Gly Asn Leu Gly Asp Leu Arg Asp
   355 360 365
   Ile Leu Lys Lys Gly Ala Thr Phe Asn Arg Glu Thr Pro Gly Val Pro
   370 375 380
   Ile Ala Tyr Thr Thr Asn Phe Leu Lys Asp Asn Glu Leu Ala Val Ile
   385 390 395 400
   Lys Asn Asn Ser Glu Tyr Ile Glu Thr Thr Ser Lys Ala Tyr Thr Asp
   405 410 415
   Gly Lys Ile Asn Ile Asp His Ser Gly Gly Tyr Val Ala Gln Phe Asn
   420 425 430
   Ile Ser Trp Asp Glu Val Asn Tyr Asp
   435 440
<210> 19
   <211> 416
   <212> PRT
   <213> Listeria monocytogenes
<400> 19
   Met Lys Lys Ile Met Leu Val Phe Ile Thr Leu Ile Leu Val Ser Leu
   1 5 10 15
   Pro Ile Ala Gln Gln Thr Glu Ala Lys Asp Ala Ser Ala Phe Asn Lys
   20 25 30
   Glu Asn Ser Ile Ser Ser Val Ala Pro Pro Ala Ser Pro Pro Ala Ser
   35 40 45
   Pro Lys Thr Pro Ile Glu Lys Lys His Ala Asp Glu Ile Asp Lys Tyr
   50 55 60
   Ile Gln Gly Leu Asp Tyr Asn Lys Asn Asn Val Leu Val Tyr His Gly
   65 70 75 80
   Asp Ala Val Thr Asn Val Pro Pro Arg Lys Gly Tyr Lys Asp Gly Asn
   85 90 95
   Glu Tyr Ile Val Val Glu Lys Lys Lys Lys Ser Ile Asn Gln Asn Asn
   100 105 110
   Ala Asp Ile Gln Val Val Asn Ala Ile Ser Ser Leu Thr Tyr Pro Gly
               115                 120                 125
   Ala Leu Val Lys Ala Asn Ser Glu Leu Val Glu Asn Gln Pro Asp Val
       130                 135                 140
   Leu Pro Val Lys Arg Asp Ser Leu Thr Leu Ser Ile Asp Leu Pro Gly
   145                 150                 155                 160
   Met Thr Asn Gln Asp Asn Lys Ile Val Val Lys Asn Ala Thr Lys Ser
                   165                 170                 175
   Asn Val Asn Asn Ala Val Asn Thr Leu Val Glu Arg Trp Asn Glu Lys
               180                 185                 190
   Tyr Ala Gln Ala Tyr Ser Asn Val Ser Ala Lys Ile Asp Tyr Asp Asp
           195                 200                 205
   Glu Met Ala Tyr Ser Glu Ser Gln Leu Ile Ala Lys Phe Gly Thr Ala
       210                 215                 220
   Phe Lys Ala Val Asn Asn Ser Leu Asn Val Asn Phe Gly Ala Ile Ser
   225                 230                 235                 240
   Glu Gly Lys Met Gln Glu Glu Val Ile Ser Phe Lys Gln Ile Tyr Tyr
                   245                 250                 255
   Asn Val Asn Val Asn Glu Pro Thr Arg Pro Ser Arg Phe Phe Gly Lys
               260                 265                 270
   Ala Val Thr Lys Glu Gln Leu Gln Ala Leu Gly Val Asn Ala Glu Asn
           275                 280                 285
   Pro Pro Ala Tyr Ile Ser Ser Val Ala Tyr Gly Arg Gln Val Tyr Leu
       290                 295                 300
   Lys Leu Ser Thr Asn Ser His Ser Thr Lys Val Lys Ala Ala Phe Asp
   305                 310                 315                 320
   Ala Ala Val Ser Gly Lys Ser Val Ser Gly Asp Val Glu Leu Thr Asn
                   325                 330                 335
   Ile Ile Lys Asn Ser Ser Phe Lys Ala Val Ile Tyr Gly Gly Ser Ala
               340                 345                 350
   Lys Asp Glu Val Gln Ile Ile Asp Gly Asn Leu Gly Asp Leu Arg Asp
           355                 360                 365
   Ile Leu Lys Lys Gly Ala Thr Phe Asn Arg Glu Thr Pro Gly Val Pro
       370                 375                 380
   Ile Ala Tyr Thr Thr Asn Phe Leu Lys Asp Asn Glu Leu Ala Val Ile
   385 390 395 400
   Lys Asn Asn Ser Glu Tyr Ile Glu Thr Thr Ser Lys Ala Tyr Thr Asp
   405 410 415
<210> 20
   <211> 19
   <212> PRT
   <213> Listeria seeligeri
<400> 20
   Arg Ser Glu Val Thr Ile Ser Pro Ala Glu Thr Pro Glu Ser Pro Pro
   1 5 10 15
   Ala Thr Pro
<210> 21
   <211> 19
   <212> PRT
   <213> Listeria monocytogenes
<400> 21
   Lys Glu Asn Ser Ile Ser Ser Met Ala Pro Pro Ala Ser Pro Pro Ala
   1 5 10 15
   Ser Pro Lys
<210> 22
   <211> 25
   <212> DNA
   <213> Artificial
<220>
   <223> chemically synthesized
<400> 22
   gcggatccca tggagataca cctac 25
<210> 23
   <211> 25
   <212> DNA
   <213> Artificial
<220>
   <223> chemically synthesized
<400> 23
   gcggatccca tggagataca cctac 25
<210> 24
   <211> 9
   <212> PRT
   <213> Human papillomavirus
<400> 24
   Arg Ala His Tyr Asn Ile Val Thr Phe
   1               5
<210> 25
   <211> 261
   <212> PRT
   <213> Homo sapiens
<400> 25
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
               100                 105                 110
   Pro Gly Asp Asp Ser Ser His Asp Leu Met Leu Leu Arg Leu Ser Glu
           115                 120                 125
   Pro Ala Glu Leu Thr Asp Ala Val Lys Val Met Asp Leu Pro Thr Gln
       130                 135                 140
   Glu Pro Ala Leu Gly Thr Thr Cys Tyr Ala Ser Gly Trp Gly Ser Ile
   145                 150                 155                 160
   Glu Pro Glu Glu Phe Leu Thr Pro Lys Lys Leu Gln Cys Val Asp Leu
                   165                 170                 175
   His Val Ile Ser Asn Asp Val Cys Ala Gln Val His Pro Gln Lys Val
               180                 185                 190
   Thr Lys Phe Met Leu Cys Ala Gly Arg Trp Thr Gly Gly Lys Ser Thr
           195                 200                 205
   Cys Ser Gly Asp Ser Gly Gly Pro Leu Val Cys Asn Gly Val Leu Gln
       210                 215                 220
   Gly Ile Thr Ser Trp Gly Ser Glu Pro Cys Ala Leu Pro Glu Arg Pro
   225                 230                 235                 240
   Ser Leu Tyr Thr Lys Val Val His Tyr Arg Lys Trp Ile Lys Asp Thr
                   245                 250                 255
   Ile Val Ala Asn Pro
               260
<210> 26
   <211> 5873
   <212> DNA
   <213> Homo sapiens
<400> 26
   ggtgtcttag gcacactggt cttggagtgc aaaggatcta ggcacgtgag gctttgtatg      60
   aagaatcggg gatcgtaccc accccctgtt tctgtttcat cctgggcatg tctcctctgc     120
   ctttgtcccc tagatgaagt ctccatgagc tacaagggcc tggtgcatcc agggtgatct     180
   agtaattgca gaacagcaag tgctagctct ccctcccctt ccacagctct gggtgtggga     240
   gggggttgtc cagcctccag cagcatgggg agggccttgg tcagcctctg ggtgccagca     300
   gggcaggggc ggagtcctgg ggaatgaagg ttttataggg ctcctggggg aggctcccca     360
   gccccaagct taccacctgc acccggagag ctgtgtcacc atgtgggtcc cggttgtctt     420
   cctcaccctg tccgtgacgt ggattggtga gaggggccat ggttgggggg atgcaggaga     480
   gggagccagc cctgactgtc aagctgaggc tctttccccc ccaacccagc accccagccc     540
   agacagggag ctgggctctt ttctgtctct cccagcccca cttcaagccc atacccccag     600
   tcccctccat attgcaacag tcctcactcc cacaccaggt ccccgctccc tcccacttac     660
   cccagaactt tcttcccatt tgcccagcca gctccctgct cccagctgct ttactaaagg     720
   ggaagttcct gggcatctcc gtgtttctct ttgtggggct caaaacctcc aaggacctct     780
   ctcaatgcca ttggttcctt ggaccgtatc actggtccat ctcctgagcc cctcaatcct     840
   atcacagtct actgactttt cccattcagc tgtgagtgtc caaccctatc ccagagacct     900
   tgatgcttgg cctcccaatc ttgccctagg atacccagat gccaaccaga cacctccttc     960
   tttcctagcc aggctatctg gcctgagaca acaaatgggt ccctcagtct ggcaatggga    1020
   ctctgagaac tcctcattcc ctgactctta gccccagact cttcattcag tggcccacat    1080
   tttccttagg aaaaacatga gcatccccag ccacaactgc cagctctctg agtccccaaa    1140
   tctgcatcct tttcaaaacc taaaaacaaa aagaaaaaca aataaaacaa aaccaactca    1200
   gaccagaact gttttctcaa cctgggactt cctaaacttt ccaaaacctt cctcttccag    1260
   caactgaacc tcgccataag gcacttatcc ctggttccta gcacccctta tcccctcaga    1320
   atccacaact tgtaccaagt ttcccttctc ccagtccaag accccaaatc accacaaagg    1380
   acccaatccc cagactcaag atatggtctg ggcgctgtct tgtgtctcct accctgatcc    1440
   ctgggttcaa ctctgctccc agagcatgaa gcctctccac cagcaccagc caccaacctg    1500
   caaacctagg gaagattgac agaattccca gcctttccca gctccccctg cccatgtccc     1560
   aggactccca gccttggttc tctgcccccg tgtcttttca aacccacatc ctaaatccat 1620
   ctcctatccg agtcccccag ttccccctgt caaccctgat tcccctgatc tagcaccccc 1680
   tctgcaggcg ctgcgcccct catcctgtct cggattgtgg gaggctggga gtgcgagaag 1740
   cattcccaac cctggcaggt gcttgtggcc tctcgtggca gggcagtctg cggcggtgtt 1800
   ctggtgcacc cccagtgggt cctcacagct gcccactgca tcaggaagtg agtaggggcc 1860
   tggggtctgg ggagcaggtg tctgtgtccc agaggaataa cagctgggca ttttccccag 1920
   gataacctct aaggccagcc ttgggactgg gggagagagg gaaagttctg gttcaggtca 1980
   catggggagg cagggttggg gctggaccac cctccccatg gctgcctggg tctccatctg 2040
   tgtccctcta tgtctctttg tgtcgctttc attatgtctc ttggtaactg gcttcggttg 2100
   tgtctctccg tgtgactatt ttgttctctc tctccctctc ttctctgtct tcagtctcca 2160
   tatctccccc tctctctgtc cttctctggt ccctctctag ccagtgtgtc tcaccctgta 2220
   tctctctgcc aggctctgtc tctcggtctc tgtctcacct gtgccttctc cctactgaac 2280
   acacgcacgg gatgggcctg ggggaccctg agaaaaggaa gggctttggc tgggcgcggt 2340
   ggctcacacc tgtaatccca gcactttggg aggccaaggc aggtagatca cctgaggtca 2400
   ggagttcgag accagcctgg ccaactggtg aaaccccatc tctactaaaa atacaaaaaa 2460
   ttagccaggc gtggtggcgc atgcctgtag tcccagctac tcaggagctg agggaggaga 2520
   attgcattga acctggaggt tgaggttgca gtgagccgag accgtgccac tgcactccag 2580
   cctgggtgac agagtgagac tccgcctcaa aaaaaaaaaa aaaaaaaaaa aaaaaaaaga 2640
   aaagaaaaga aaagaaaagg aagtgtttta tccctgatgt gtgtgggtat gagggtatga 2700
   gagggcccct ctcactccat tccttctcca ggacatccct ccactcttgg gagacacaga 2760
   gaagggctgg ttccagctgg agctgggagg ggcaattgag ggaggaggaa ggagaagggg 2820
   gaaggaaaac agggtatggg ggaaaggacc ctggggagcg aagtggagga tacaaccttg 2880
   ggcctgcagg caggctacct acccacttgg aaacccacgc caaagccgca tctacagctg 2940
   agccactctg aggcctcccc tccccggcgg tccccactca gctccaaagt ctctctccct 3000
   tttctctccc acactttatc atcccccgga ttcctctcta cttggttctc attcttcctt 3060
   tgacttcctg cttccctttc tcattcatct gtttctcact ttctgcctgg ttttgttctt 3120
   ctctctctct ttctctggcc catgtctgtt tctctatgtt tctgtctttt ctttctcatc 3180
   ctgtgtattt tcggctcacc ttgtttgtca ctgttctccc ctctgccctt tcattctctc 3240
   tgccctttta ccctcttcct tttcccttgg ttctctcagt tctgtatctg cccttcaccc 3300
   tctcacactg ctgtttccca actcgttgtc tgtattttgg cctgaactgt gtcttcccaa 3360
   ccctgtgttt tctcactgtt tctttttctc ttttggagcc tcctccttgc tcctctgtcc 3420
   cttctctctt tccttatcat cctcgctcct cattcctgcg tctgcttcct ccccagcaaa 3480
   agcgtgatct tgctgggtcg gcacagcctg tttcatcctg aagacacagg ccaggtattt 3540
   caggtcagcc acagcttccc acacccgctc tacgatatga gcctcctgaa gaatcgattc 3600
   ctcaggccag gtgatgactc cagccacgac ctcatgctgc tccgcctgtc agagcctgcc 3660
   gagctcacgg atgctgtgaa ggtcatggac ctgcccaccc aggagccagc actggggacc 3720
   acctgctacg cctcaggctg gggcagcatt gaaccagagg agtgtacgcc tgggccagat 3780
   ggtgcagccg ggagcccaga tgcctgggtc tgagggagga ggggacagga ctcctgggtc 3840
   tgagggagga gggccaagga accaggtggg gtccagccca caacagtgtt tttgcctggc 3900
   ccgtagtctt gaccccaaag aaacttcagt gtgtggacct ccatgttatt tccaatgacg 3960
   tgtgtgcgca agttcaccct cagaaggtga ccaagttcat gctgtgtgct ggacgctgga 4020
   cagggggcaa aagcacctgc tcggtgagtc atccctactc ccaagatctt gagggaaagg 4080
   tgagtgggac cttaattctg ggctggggtc tagaagccaa caaggcgtct gcctcccctg 4140
   ctccccagct gtagccatgc cacctccccg tgtctcatct cattccctcc ttccctcttc 4200
   tttgactccc tcaaggcaat aggttattct tacagcacaa ctcatctgtt cctgcgttca 4260
   gcacacggtt actaggcacc tgctatgcac ccagcactgc cctagagcct gggacatagc 4320
   agtgaacaga cagagagcag cccctccctt ctgtagcccc caagccagtg aggggcacag 4380
   gcaggaacag ggaccacaac acagaaaagc tggagggtgt caggaggtga tcaggctctc 4440
   ggggagggag aaggggtggg gagtgtgact gggaggagac atcctgcaga aggtgggagt 4500
   gagcaaacac ctgcgcaggg gaggggaggg cctgcggcac ctgggggagc agagggaaca 4560
   gcatctggcc aggcctggga ggaggggcct agagggcgtc aggagcagag aggaggttgc 4620
   ctggctggag tgaaggatcg gggcagggtg cgagagggaa caaaggaccc ctcctgcagg 4680
   gcctcacctg ggccacagga ggacactgct tttcctctga ggagtcagga actgtggatg 4740
   gtgctggaca gaagcaggac agggcctggc tcaggtgtcc agaggctgcg ctggcctcct 4800
   atgggatcag actgcaggga gggagggcag cagggatgtg gagggagtga tgatggggct 4860
   gacctggggg tggctccagg cattgtcccc acctgggccc ttacccagcc tccctcacag 4920
   gctcctggcc ctcagtctct cccctccact ccattctcca cctacccaca gtgggtcatt 4980
   ctgatcaccg aactgaccat gccagccctg ccgatggtcc tccatggctc cctagtgccc 5040
   tggagaggag gtgtctagtc agagagtagt cctggaaggt ggcctctgtg aggagccacg 5100
   gggacagcat cctgcagatg gtcctggccc ttgtcccacc gacctgtcta caaggactgt 5160
   cctcgtggac cctcccctct gcacaggagc tggaccctga agtcccttcc taccggccag 5220
   gactggagcc cctacccctc tgttggaatc cctgcccacc ttcttctgga agtcggctct 5280
   ggagacattt ctctcttctt ccaaagctgg gaactgctat ctgttatctg cctgtccagg 5340
   tctgaaagat aggattgccc aggcagaaac tgggactgac ctatctcact ctctccctgc 5400
   ttttaccctt agggtgattc tgggggccca cttgtctgta atggtgtgct tcaaggtatc 5460
   acgtcatggg gcagtgaacc atgtgccctg cccgaaaggc cttccctgta caccaaggtg 5520
   gtgcattacc ggaagtggat caaggacacc atcgtggcca acccctgagc acccctatca 5580
   agtccctatt gtagtaaact tggaaccttg gaaatgacca ggccaagact caagcctccc 5640
   cagttctact gacctttgtc cttaggtgtg aggtccaggg ttgctaggaa aagaaatcag    5700
   cagacacagg tgtagaccag agtgtttctt aaatggtgta attttgtcct ctctgtgtcc    5760
   tggggaatac tggccatgcc tggagacata tcactcaatt tctctgagga cacagttagg    5820
   atggggtgtc tgtgttattt gtgggataca gagatgaaag aggggtggga tcc           5873
<210> 27
   <211> 238
   <212> PRT
   <213> Homo sapiens
<400> 27
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
               100                 105                 110
   Pro Gly Asp Asp Ser Ser His Asp Leu Met Leu Leu Arg Leu Ser Glu
           115                 120                 125
   Pro Ala Glu Leu Thr Asp Ala Val Lys Val Met Asp Leu Pro Thr Gln
       130                 135                 140
   Glu Pro Ala Leu Gly Thr Thr Cys Tyr Ala Ser Gly Trp Gly Ser Ile
   145                 150                 155                 160
   Glu Pro Glu Glu Phe Leu Thr Pro Lys Lys Leu Gln Cys Val Asp Leu
                   165                 170                 175
   His Val Ile Ser Asn Asp Val Cys Ala Gln Val His Pro Gln Lys Val
               180                 185                 190
   Thr Lys Phe Met Leu Cys Ala Gly Arg Trp Thr Gly Gly Lys Ser Thr
           195                 200                 205
   Cys Ser Trp Val Ile Leu Ile Thr Glu Leu Thr Met Pro Ala Leu Pro
   210 215 220
   Met Val Leu His Gly Ser Leu Val Pro Trp Arg Gly Gly Val
   225 230 235
<210> 28
   <211> 1906
   <212> DNA
   <213> Homo sapiens
<400> 28
   agccccaagc ttaccacctg cacccggaga gctgtgtcac catgtgggtc ccggttgtct 60
   tcctcaccct gtccgtgacg tggattggtg ctgcacccct catcctgtct cggattgtgg 120
   gaggctggga gtgcgagaag cattcccaac cctggcaggt gcttgtggcc tctcgtggca 180
   gggcagtctg cggcggtgtt ctggtgcacc cccagtgggt cctcacagct gcccactgca 240
   tcaggaacaa aagcgtgatc ttgctgggtc ggcacagcct gtttcatcct gaagacacag 300
   gccaggtatt tcaggtcagc cacagcttcc cacacccgct ctacgatatg agcctcctga 360
   agaatcgatt cctcaggcca ggtgatgact ccagccacga cctcatgctg ctccgcctgt 420
   cagagcctgc cgagctcacg gatgctgtga aggtcatgga cctgcccacc caggagccag 480
   cactggggac cacctgctac gcctcaggct ggggcagcat tgaaccagag gagttcttga 540
   ccccaaagaa acttcagtgt gtggacctcc atgttatttc caatgacgtg tgtgcgcaag 600
   ttcaccctca gaaggtgacc aagttcatgc tgtgtgctgg acgctggaca gggggcaaaa 660
   gcacctgctc gtgggtcatt ctgatcaccg aactgaccat gccagccctg ccgatggtcc 720
   tccatggctc cctagtgccc tggagaggag gtgtctagtc agagagtagt cctggaaggt 780
   ggcctctgtg aggagccacg gggacagcat cctgcagatg gtcctggccc ttgtcccacc 840
   gacctgtcta caaggactgt cctcgtggac cctcccctct gcacaggagc tggaccctga 900
   agtcccttcc ccaccggcca ggactggagc ccctacccct ctgttggaat ccctgcccac 960
   cttcttctgg aagtcggctc tggagacatt tctctcttct tccaaagctg ggaactgcta 1020
   tctgttatct gcctgtccag gtctgaaaga taggattgcc caggcagaaa ctgggactga 1080
   cctatctcac tctctccctg cttttaccct tagggtgatt ctgggggccc acttgtctgt 1140
   aatggtgtgc ttcaaggtat cacgtcatgg ggcagtgaac catgtgccct gcccgaaagg 1200
   ccttccctgt acaccaaggt ggtgcattac cggaagtgga tcaaggacac catcgtggcc 1260
   aacccctgag cacccctatc aaccccctat tgtagtaaac ttggaacctt ggaaatgacc 1320
   aggccaagac tcaagcctcc ccagttctac tgacctttgt ccttaggtgt gaggtccagg 1380
   gttgctagga aaagaaatca gcagacacag gtgtagacca gagtgtttct taaatggtgt 1440
   aattttgtcc tctctgtgtc ctggggaata ctggccatgc ctggagacat atcactcaat 1500
   ttctctgagg acacagatag gatggggtgt ctgtgttatt tgtggggtac agagatgaaa 1560
   gaggggtggg atccacactg agagagtgga gagtgacatg tgctggacac tgtccatgaa 1620
   gcactgagca gaagctggag gcacaacgca ccagacactc acagcaagga tggagctgaa 1680
   aacataaccc actctgtcct ggaggcactg ggaagcctag agaaggctgt gagccaagga 1740
   gggagggtct tcctttggca tgggatgggg atgaagtaag gagagggact ggaccccctg 1800
   gaagctgatt cactatgggg ggaggtgtat tgaagtcctc cagacaaccc tcagatttga 1860
   tgatttccta gtagaactca cagaaataaa gagctgttat actgtg 1906
<210> 29
   <211> 69
   <212> PRT
   <213> Homo sapiens
<400> 29
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Lys
   65
<210> 30
   <211> 220
   <212> PRT
   <213> Homo sapiens
<400> 30
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
   100 105 110
   Pro Gly Asp Asp Ser Ser Ile Glu Pro Glu Glu Phe Leu Thr Pro Lys
   115 120 125
   Lys Leu Gln Cys Val Asp Leu His Val Ile Ser Asn Asp Val Cys Ala
   130 135 140
   Gln Val His Pro Gln Lys Val Thr Lys Phe Met Leu Cys Ala Gly Arg
   145 150 155 160
   Trp Thr Gly Gly Lys Ser Thr Cys Ser Gly Asp Ser Gly Gly Pro Leu
   165 170 175
   Val Cys Asn Gly Val Leu Gln Gly Ile Thr Ser Trp Gly Ser Glu Pro
   180 185 190
   Cys Ala Leu Pro Glu Arg Pro Ser Leu Tyr Thr Lys Val Val His Tyr
   195 200 205
   Arg Lys Trp Ile Lys Asp Thr Ile Val Ala Asn Pro
   210 215 220
<210> 31
   <211> 218
   <212> PRT
   <213> Homo sapiens
<400> 31
   Met Trp Val Pro Val Val Phe Leu Thr Leu Ser Val Thr Trp Ile Gly
   1 5 10 15
   Ala Ala Pro Leu Ile Leu Ser Arg Ile Val Gly Gly Trp Glu Cys Glu
   20 25 30
   Lys His Ser Gln Pro Trp Gln Val Leu Val Ala Ser Arg Gly Arg Ala
   35 40 45
   Val Cys Gly Gly Val Leu Val His Pro Gln Trp Val Leu Thr Ala Ala
   50 55 60
   His Cys Ile Arg Lys Pro Gly Asp Asp Ser Ser His Asp Leu Met Leu
   65 70 75 80
   Leu Arg Leu Ser Glu Pro Ala Glu Leu Thr Asp Ala Val Lys Val Met
   85 90 95
   Asp Leu Pro Thr Gln Glu Pro Ala Leu Gly Thr Thr Cys Tyr Ala Ser
   100 105 110
   Gly Trp Gly Ser Ile Glu Pro Glu Glu Phe Leu Thr Pro Lys Lys Leu
   115 120 125
   Gln Cys Val Asp Leu His Val Ile Ser Asn Asp Val Cys Ala Gln Val
   130 135 140
   His Pro Gln Lys Val Thr Lys Phe Met Leu Cys Ala Gly Arg Trp Thr
   145 150 155 160
   Gly Gly Lys Ser Thr Cys Ser Gly Asp Ser Gly Gly Pro Leu Val Cys
   165 170 175
   Asn Gly Val Leu Gln Gly Ile Thr Ser Trp Gly Ser Glu Pro Cys Ala
   180 185 190
   Leu Pro Glu Arg Pro Ser Leu Tyr Thr Lys Val Val His Tyr Arg Lys
   195 200 205
   Trp Ile Lys Asp Thr Ile Val Ala Asn Pro
   210 215
<210> 32
   <211> 261
   <212> PRT
   <213> Homo sapiens
<400> 32
   Met Trp Val Pro Val Val Phe Leu Thr Leu Ser Val Thr Trp Ile Gly
   1 5 10 15
   Ala Ala Pro Leu Ile Leu Ser Arg Ile Val Gly Gly Trp Glu Cys Glu
   20 25 30
   Lys His Ser Gln Pro Trp Gln Val Leu Val Ala Ser Arg Gly Arg Ala
   35 40 45
   Val Cys Gly Gly Val Leu Val His Pro Gln Trp Val Leu Thr Ala Ala
   50 55 60
   His Cys Ile Arg Asn Lys Ser Val Ile Leu Leu Gly Arg His Ser Leu
   65 70 75 80
   Phe His Pro Glu Asp Thr Gly Gln Val Phe Gln Val Ser His Ser Phe
   85 90 95
   Pro His Pro Leu Tyr Asp Met Ser Leu Leu Lys Asn Arg Phe Leu Arg
   100 105 110
   Pro Gly Asp Asp Ser Ser His Asp Leu Met Leu Leu Arg Leu Ser Glu
   115 120 125
   Pro Ala Glu Leu Thr Asp Ala Val Lys Val Met Asp Leu Pro Thr Gln
       130                 135                 140
   Glu Pro Ala Leu Gly Thr Thr Cys Tyr Ala Ser Gly Trp Gly Ser Ile
   145                 150                 155                 160
   Glu Pro Glu Glu Phe Leu Thr Pro Lys Lys Leu Gln Cys Val Asp Leu
                   165                 170                 175
   His Val Ile Ser Asn Asp Val Cys Ala Gln Val His Pro Gln Lys Val
               180                 185                 190
   Thr Lys Phe Met Leu Cys Ala Gly Arg Trp Thr Gly Gly Lys Ser Thr
           195                 200                 205
   Cys Ser Gly Asp Ser Gly Gly Pro Leu Val Cys Asn Gly Val Leu Gln
       210                 215                 220
   Gly Ile Thr Ser Trp Gly Ser Glu Pro Cys Ala Leu Pro Glu Arg Pro
   225                 230                 235                 240
   Ser Leu Tyr Thr Lys Val Val His Tyr Arg Lys Trp Ile Lys Asp Thr
                   245                 250                 255
   Ile Val Ala Asn Pro
               260
<210> 33
   <211> 1464
   <212> DNA
   <213> Homo sapiens
<400> 33
   agccccaagc ttaccacctg cacccggaga gctgtgtcac catgtgggtc ccggttgtct      60
   tcctcaccct gtccgtgacg tggattggtg ctgcacccct catcctgtct cggattgtgg     120
   gaggctggga gtgcgagaag cattcccaac cctggcaggt gcttgtggcc tctcgtggca     180
   gggcagtctg cggcggtgtt ctggtgcacc cccagtgggt cctcacagct gcccactgca     240
   tcaggaacaa aagcgtgatc ttgctgggtc ggcacagcct gtttcatcct gaagacacag     300
   gccaggtatt tcaggtcagc cacagcttcc cacacccgct ctacgatatg agcctcctga     360
   agaatcgatt cctcaggcca ggtgatgact ccagccacga cctcatgctg ctccgcctgt     420
   cagagcctgc cgagctcacg gatgctgtga aggtcatgga cctgcccacc caggagccag     480
   cactggggac cacctgctac gcctcaggct ggggcagcat tgaaccagag gagttcttga     540
   ccccaaagaa acttcagtgt gtggacctcc atgttatttc caatgacgtg tgtgcgcaag     600
   ttcaccctca gaaggtgacc aagttcatgc tgtgtgctgg acgctggaca gggggcaaaa     660
   gcacctgctc gggtgattct gggggcccac ttgtctgtaa tggtgtgctt caaggtatca     720
   cgtcatgggg cagtgaacca tgtgccctgc ccgaaaggcc ttccctgtac accaaggtgg     780
   tgcattaccg gaagtggatc aaggacacca tcgtggccaa cccctgagca cccctatcaa     840
   ccccctattg tagtaaactt ggaaccttgg aaatgaccag gccaagactc aagcctcccc     900
   agttctactg acctttgtcc ttaggtgtga ggtccagggt tgctaggaaa agaaatcagc     960
   agacacaggt gtagaccaga gtgtttctta aatggtgtaa ttttgtcctc tctgtgtcct    1020
   ggggaatact ggccatgcct ggagacatat cactcaattt ctctgaggac acagatagga    1080
   tggggtgtct gtgttatttg tggggtacag agatgaaaga ggggtgggat ccacactgag    1140
   agagtggaga gtgacatgtg ctggacactg tccatgaagc actgagcaga agctggaggc    1200
   acaacgcacc agacactcac agcaaggatg gagctgaaaa cataacccac tctgtcctgg    1260
   aggcactggg aagcctagag aaggctgtga gccaaggagg gagggtcttc ctttggcatg    1320
   ggatggggat gaagtaagga gagggactgg accccctgga agctgattca ctatgggggg    1380
   aggtgtattg aagtcctcca gacaaccctc agatttgatg atttcctagt agaactcaca    1440
   gaaataaaga gctgttatac tgtg                                           1464
<210> 34
   <211> 261
   <212> PRT
   <213> Homo sapiens
<400> 34
   Met Trp Val Pro Val Val Phe Leu Thr Leu Ser Val Thr Trp Ile Gly
   1 5 10 15
   Ala Ala Pro Leu Ile Leu Ser Arg Ile Val Gly Gly Trp Glu Cys Glu
   20 25 30
   Lys His Ser Gln Pro Trp Gln Val Leu Val Ala Ser Arg Gly Arg Ala
   35 40 45
   Val Cys Gly Gly Val Leu Val His Pro Gln Trp Val Leu Thr Ala Ala
   50 55 60
   His Cys Ile Arg Asn Lys Ser Val Ile Leu Leu Gly Arg His Ser Leu
   65 70 75 80
   Phe His Pro Glu Asp Thr Gly Gln Val Phe Gln Val Ser His Ser Phe
   85 90 95
   Pro His Pro Leu Tyr Asp Met Ser Leu Leu Lys Asn Arg Phe Leu Arg
   100 105 110
   Pro Gly Asp Asp Ser Ser His Asp Leu Met Leu Leu Arg Leu Ser Glu
   115 120 125
   Pro Ala Glu Leu Thr Asp Ala Val Lys Val Met Asp Leu Pro Thr Gln
   130 135 140
   Glu Pro Ala Leu Gly Thr Thr Cys Tyr Ala Ser Gly Trp Gly Ser Ile
   145 150 155 160
   Glu Pro Glu Glu Phe Leu Thr Pro Lys Lys Leu Gln Cys Val Asp Leu
   165 170 175
   His Val Ile Ser Asn Asp Val Cys Ala Gln Val His Pro Gln Lys Val
   180 185 190
   Thr Lys Phe Met Leu Cys Ala Gly Arg Trp Thr Gly Gly Lys Ser Thr
   195 200 205
   Cys Ser Gly Asp Ser Gly Gly Pro Leu Val Cys Asn Gly Val Leu Gln
   210 215 220
   Gly Ile Thr Ser Trp Gly Ser Glu Pro Cys Ala Leu Pro Glu Arg Pro
   225 230 235 240
   Ser Leu Tyr Thr Lys Val Val His Tyr Arg Lys Trp Ile Lys Asp Thr
   245 250 255
   Ile Val Ala Asn Pro
   260
<210> 35
   <211> 1495
   <212> DNA
   <213> Homo sapiens
<400> 35
   gggggagccc caagcttacc acctgcaccc ggagagctgt gtcaccatgt gggtcccggt     60
   tgtcttcctc accctgtccg tgacgtggat tggtgctgca cccctcatcc tgtctcggat    120
   tgtgggaggc tgggagtgcg agaagcattc ccaaccctgg caggtgcttg tggcctctcg    180
   tggcagggca gtctgcggcg gtgttctggt gcacccccag tgggtcctca cagctgccca    240
   ctgcatcagg aacaaaagcg tgatcttgct gggtcggcac agcctgtttc atcctgaaga    300
   cacaggccag gtatttcagg tcagccacag cttcccacac ccgctctacg atatgagcct    360
   cctgaagaat cgattcctca ggccaggtga tgactccagc cacgacctca tgctgctccg    420
   cctgtcagag cctgccgagc tcacggatgc tgtgaaggtc atggacctgc ccacccagga    480
   gccagcactg gggaccacct gctacgcctc aggctggggc agcattgaac cagaggagtt    540
   cttgacccca aagaaacttc agtgtgtgga cctccatgtt atttccaatg acgtgtgtgc    600
   gcaagttcac cctcagaagg tgaccaagtt catgctgtgt gctggacgct ggacaggggg    660
   caaaagcacc tgctcgggtg attctggggg cccacttgtc tgtaatggtg tgcttcaagg    720
   tatcacgtca tggggcagtg aaccatgtgc cctgcccgaa aggccttccc tgtacaccaa    780
   ggtggtgcat taccggaagt ggatcaagga caccatcgtg gccaacccct gagcacccct    840
   atcaactccc tattgtagta aacttggaac cttggaaatg accaggccaa gactcaggcc    900
   tccccagttc tactgacctt tgtccttagg tgtgaggtcc agggttgcta ggaaaagaaa    960
   tcagcagaca caggtgtaga ccagagtgtt tcttaaatgg tgtaattttg tcctctctgt   1020
   gtcctgggga atactggcca tgcctggaga catatcactc aatttctctg aggacacaga   1080
   taggatgggg tgtctgtgtt atttgtgggg tacagagatg aaagaggggt gggatccaca   1140
   ctgagagagt ggagagtgac atgtgctgga cactgtccat gaagcactga gcagaagctg   1200
   gaggcacaac gcaccagaca ctcacagcaa ggatggagct gaaaacataa cccactctgt   1260
   cctggaggca ctgggaagcc tagagaaggc tgtgagccaa ggagggaggg tcttcctttg   1320
   gcatgggatg gggatgaagt agggagaggg actggacccc ctggaagctg attcactatg   1380
   gggggaggtg tattgaagtc ctccagacaa ccctcagatt tgatgatttc ctagtagaac   1440
   tcacagaaat aaagagctgt tatactgcga aaaaaaaaaa aaaaaaaaaa aaaaa        1495
<210> 36
   <211> 218
   <212> PRT
   <213> Homo sapiens
<400> 36
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
               100                 105                 110
   Pro Gly Asp Asp Ser Ser Ile Glu Pro Glu Glu Phe Leu Thr Pro Lys
           115                 120                 125
   Lys Leu Gln Cys Val Asp Leu His Val Ile Ser Asn Asp Val Cys Ala
       130                 135                 140
   Gln Val His Pro Gln Lys Val Thr Lys Phe Met Leu Cys Ala Gly Arg
   145                 150                 155                 160
   Trp Thr Gly Gly Lys Ser Thr Cys Ser Gly Asp Ser Gly Gly Pro Leu
                   165                 170                 175
   Val Cys Asn Gly Val Leu Gln Gly Ile Thr Ser Trp Gly Ser Glu Pro
               180                 185                 190
   Cys Ala Leu Pro Glu Arg Pro Ser Leu Tyr Thr Lys Val Val His Tyr
           195                 200                 205
   Arg Lys Trp Ile Lys Asp Thr Ile Val Ala
       210                 215
<210> 37
   <211> 227
   <212> PRT
   <213> Homo sapiens
<400> 37
   Met Trp Val Pro Val Val Phe Leu Thr Leu Ser Val Thr Trp Ile Gly
   1               5                   10                  15
   Ala Ala Pro Leu Ile Leu Ser Arg Ile Val Gly Gly Trp Glu Cys Glu
               20                  25                  30
   Lys His Ser Gln Pro Trp Gln Val Leu Val Ala Ser Arg Gly Arg Ala
           35                  40                  45
   Val Cys Gly Gly Val Leu Val His Pro Gln Trp Val Leu Thr Ala Ala
       50                  55                  60
   His Cys Ile Arg Asn Lys Ser Val Ile Leu Leu Gly Arg His Ser Leu
   65                  70                  75                  80
   Phe His Pro Glu Asp Thr Gly Gln Val Phe Gln Val Ser His Ser Phe
                   85                  90                  95
   Pro His Pro Leu Tyr Asp Met Ser Leu Leu Lys Asn Arg Phe Leu Arg
               100                 105                 110
   Pro Gly Asp Asp Ser Ser His Asp Leu Met Leu Leu Arg Leu Ser Glu
           115                 120                 125
   Pro Ala Glu Leu Thr Asp Ala Val Lys Val Met Asp Leu Pro Thr Gln
       130                 135                 140
   Glu Pro Ala Leu Gly Thr Thr Cys Tyr Ala Ser Gly Trp Gly Ser Ile
   145                 150                 155                 160
   Glu Pro Glu Glu Phe Leu Thr Pro Lys Lys Leu Gln Cys Val Asp Leu
                   165                 170                 175
   His Val Ile Ser Asn Asp Val Cys Ala Gln Val His Pro Gln Lys Val
               180                 185                 190
   Thr Lys Phe Met Leu Cys Ala Gly Arg Trp Thr Gly Gly Lys Ser Thr
           195                 200                 205
   Cys Ser Val Ser His Pro Tyr Ser Gln Asp Leu Glu Gly Lys Gly Glu
       210                 215                 220
   Trp Gly Pro
   225
<210> 38
   <211> 104
   <212> PRT
   <213> Homo sapiens
<400> 38
   Met Trp Val Pro Val Val Phe Leu Thr Leu Ser Val Thr Trp Ile Gly
   1 5 10 15
   Glu Arg Gly His Gly Trp Gly Asp Ala Gly Glu Gly Ala Ser Pro Asp
   20 25 30
   Cys Gln Ala Glu Ala Leu Ser Pro Pro Thr Gln His Pro Ser Pro Asp
   35 40 45
   Arg Glu Leu Gly Ser Phe Leu Ser Leu Pro Ala Pro Leu Gln Ala His
   50 55 60
   Thr Pro Ser Pro Ser Ile Leu Gln Gln Ser Ser Leu Pro His Gln Val
   65 70 75 80
   Pro Ala Pro Ser His Leu Pro Gln Asn Phe Leu Pro Ile Ala Gln Pro
   85 90 95
   Ala Pro Cys Ser Gln Leu Leu Tyr
   100
<210> 39
   <211> 261
   <212> PRT
   <213> Homo sapiens
<400> 39
   Met Trp Val Pro Val Val Phe Leu Thr Leu Ser Val Thr Trp Ile Gly
   1 5 10 15
   Ala Ala Pro Leu Ile Leu Ser Arg Ile Val Gly Gly Trp Glu Cys Glu
   20 25 30
   Lys His Ser Gln Pro Trp Gln Val Leu Val Ala Ser Arg Gly Arg Ala
   35 40 45
   Val Cys Gly Gly Val Leu Val His Pro Gln Trp Val Leu Thr Ala Ala
   50 55 60
   His Cys Ile Arg Asn Lys Ser Val Ile Leu Leu Gly Arg His Ser Leu
   65 70 75 80
   Phe His Pro Glu Asp Thr Gly Gln Val Phe Gln Val Ser His Ser Phe
   85 90 95
   Pro His Pro Leu Tyr Asp Met Ser Leu Leu Lys Asn Arg Phe Leu Arg
   100 105 110
   Pro Gly Asp Asp Ser Ser His Asp Leu Met Leu Leu Arg Leu Ser Glu
   115 120 125
   Pro Ala Glu Leu Thr Asp Ala Val Lys Val Met Asp Leu Pro Thr Gln
   130 135 140
   Glu Pro Ala Leu Gly Thr Thr Cys Tyr Ala Ser Gly Trp Gly Ser Ile
   145 150 155 160
   Glu Pro Glu Glu Phe Leu Thr Pro Lys Lys Leu Gln Cys Val Asp Leu
   165 170 175
   His Val Ile Ser Asn Asp Val Cys Ala Gln Val His Pro Gln Lys Val
   180 185 190
   Thr Lys Phe Met Leu Cys Ala Gly Arg Trp Thr Gly Gly Lys Ser Thr
   195 200 205
   Cys Ser Gly Asp Ser Gly Gly Pro Leu Val Cys Asn Gly Val Leu Gln
   210 215 220
   Gly Ile Thr Ser Trp Gly Ser Glu Pro Cys Ala Leu Pro Glu Arg Pro
   225 230 235 240
   Ser Leu Tyr Thr Lys Val Val His Tyr Arg Lys Trp Ile Lys Asp Thr
   245 250 255
   Ile Val Ala Asn Pro
   260
<210> 40
   <211> 1729
   <212> DNA
   <213> Homo sapiens
<400> 40
   aagtttccct tctcccagtc caagacccca aatcaccaca aaggacccaa tccccagact 60
   caagatatgg tctgggcgct gtcttgtgtc tcctaccctg atccctgggt tcaactctgc 120
   tcccagagca tgaagcctct ccaccagcac cagccaccaa cctgcaaacc tagggaagat 180
   tgacagaatt cccagccttt cccagctccc cctgcccatg tcccaggact cccagccttg 240
   gttctctgcc cccgtgtctt ttcaaaccca catcctaaat ccatctccta tccgagtccc 300
   ccagttcctc ctgtcaaccc tgattcccct gatctagcac cccctctgca ggtgctgcac 360
   ccctcatcct gtctcggatt gtgggaggct gggagtgcga gaagcattcc caaccctggc 420
   aggtgcttgt agcctctcgt ggcagggcag tctgcggcgg tgttctggtg cacccccagt 480
   gggtcctcac agctacccac tgcatcagga acaaaagcgt gatcttgctg ggtcggcaca 540
   gcctgtttca tcctgaagac acaggccagg tatttcaggt cagccacagc ttcccacacc 600
   cgctctacga tatgagcctc ctgaagaatc gattcctcag gccaggtgat gactccagcc 660
   acgacctcat gctgctccgc ctgtcagagc ctgccgagct cacggatgct atgaaggtca 720
   tggacctgcc cacccaggag ccagcactgg ggaccacctg ctacgcctca ggctggggca 780
   gcattgaacc agaggagttc ttgaccccaa agaaacttca gtgtgtggac ctccatgtta 840
   tttccaatga cgtgtgtgcg caagttcacc ctcagaaggt gaccaagttc atgctgtgtg 900
   ctggacgctg gacagggggc aaaagcacct gctcgggtga ttctgggggc ccacttgtct 960
   gtaatggtgt gcttcaaggt atcacgtcat ggggcagtga accatgtgcc ctgcccgaaa 1020
   ggccttccct gtacaccaag gtggtgcatt accggaagtg gatcaaggac accatcgtgg 1080
   ccaacccctg agcaccccta tcaactccct attgtagtaa acttggaacc ttggaaatga 1140
   ccaggccaag actcaggcct ccccagttct actgaccttt gtccttaggt gtgaggtcca 1200
   gggttgctag gaaaagaaat cagcagacac aggtgtagac cagagtgttt cttaaatggt 1260
   gtaattttgt cctctctgtg tcctggggaa tactggccat gcctggagac atatcactca 1320
   atttctctga ggacacagat aggatggggt gtctgtgtta tttgtggggt acagagatga 1380
   aagaggggtg ggatccacac tgagagagtg gagagtgaca tgtgctggac actgtccatg 1440
   aagcactgag cagaagctgg aggcacaacg caccagacac tcacagcaag gatggagctg 1500
   aaaacataac ccactctgtc ctggaggcac tgggaagcct agagaaggct gtgaaccaag 1560
   gagggagggt cttcctttgg catgggatgg ggatgaagta aggagaggga ctgaccccct 1620
   ggaagctgat tcactatggg gggaggtgta ttgaagtcct ccagacaacc ctcagatttg 1680
   atgatttcct agtagaactc acagaaataa agagctgtta tactgtgaa 1729
<210> 41
   <211> 719
   <212> PRT
   <213> Homo sapiens
<400> 41
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
   35 40 45
   Ala Thr Asn Ile Thr Pro Lys His Asn Met Lys Ala Phe Leu Asp Glu
   50 55 60
   Leu Lys Ala Glu Asn Ile Lys Lys Phe Leu Tyr Asn Phe Thr Gln Ile
   65 70 75 80
   Pro His Leu Ala Gly Thr Glu Gln Asn Phe Gln Leu Ala Lys Gln Ile
   85 90 95
   Gln Ser Gln Trp Lys Glu Phe Gly Leu Asp Ser Val Glu Leu Ala His
   100 105 110
   Tyr Asp Val Leu Leu Ser Tyr Pro Asn Lys Thr His Pro Asn Tyr Ile
   115 120 125
   Ser Ile Ile Asn Glu Asp Gly Asn Glu Ile Phe Asn Thr Ser Leu Phe
   130 135 140
   Glu Pro Pro Pro Pro Gly Tyr Glu Asn Val Ser Asp Ile Val Pro Pro
   145 150 155 160
   Phe Ser Ala Phe Ser Pro Gln Gly Met Pro Glu Gly Asp Leu Val Tyr
   165 170 175
   Val Asn Tyr Ala Arg Thr Glu Asp Phe Phe Lys Leu Glu Arg Asp Met
   180 185 190
   Lys Ile Asn Cys Ser Gly Lys Ile Val Ile Ala Arg Tyr Gly Lys Val
   195 200 205
   Phe Arg Gly Asn Lys Val Lys Asn Ala Gln Leu Ala Gly Ala Lys Gly
   210 215 220
   Val Ile Leu Tyr Ser Asp Pro Ala Asp Tyr Phe Ala Pro Gly Val Lys
   225 230 235 240
   Ser Tyr Pro Asp Gly Trp Asn Leu Pro Gly Gly Gly Val Gln Arg Gly
   245 250 255
   Asn Ile Leu Asn Leu Asn Gly Ala Gly Asp Pro Leu Thr Pro Gly Tyr
   260 265 270
   Pro Ala Asn Glu Tyr Ala Tyr Arg Arg Gly Ile Ala Glu Ala Val Gly
   275 280 285
   Leu Pro Ser Ile Pro Val His Pro Ile Gly Tyr Tyr Asp Ala Gln Lys
   290 295 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385                 390                 395                 400
   Ser Phe Gly Thr Leu Lys Lys Glu Gly Trp Arg Pro Arg Arg Thr Ile
                   405                 410                 415
   Leu Phe Ala Ser Trp Asp Ala Glu Glu Phe Gly Leu Leu Gly Ser Thr
               420                 425                 430
   Glu Trp Ala Glu Glu Asn Ser Arg Leu Leu Gln Glu Arg Gly Val Ala
           435                 440                 445
   Tyr Ile Asn Ala Asp Ser Ser Ile Glu Gly Asn Tyr Thr Leu Arg Val
       450                 455                 460
   Asp Cys Thr Pro Leu Met Tyr Ser Leu Val His Asn Leu Thr Lys Glu
   465                 470                 475                 480
   Leu Lys Ser Pro Asp Glu Gly Phe Glu Gly Lys Ser Leu Tyr Glu Ser
                   485                 490                 495
   Trp Thr Lys Lys Ser Pro Ser Pro Glu Phe Ser Gly Met Pro Arg Ile
               500                 505                 510
   Ser Lys Leu Gly Ser Gly Asn Asp Phe Glu Val Phe Phe Gln Arg Leu
           515                 520                 525
   Gly Ile Ala Ser Gly Arg Ala Arg Tyr Thr Lys Asn Trp Glu Thr Asn
       530                 535                 540
   Lys Phe Ser Gly Tyr Pro Leu Tyr His Ser Val Tyr Glu Thr Tyr Glu
   545                 550                 555                 560
   Leu Val Glu Lys Phe Tyr Asp Pro Met Phe Lys Tyr His Leu Thr Val
                   565                 570                 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Lys His Val Ile Tyr Ala Pro Ser Ser His Asn Lys Tyr Ala Gly Glu
   660 665 670
   Ser Phe Pro Gly Ile Tyr Asp Ala Leu Phe Asp Ile Glu Ser Lys Val
   675 680 685
   Asp Pro Ser Lys Ala Trp Gly Glu Val Lys Arg Gln Ile Tyr Val Ala
   690 695 700
   Ala Phe Thr Val Gln Ala Ala Ala Glu Thr Leu Ser Glu Val Ala
   705 710 715
<210> 42
   <211> 2472
   <212> DNA
   <213> Homo sapiens
<400> 42
   ctggacccca ggtctggagc gaattccagc ctgcagggct gataagcgag gcattagtga 60
   gattgagaga gactttaccc cgccgtggtg gttggagggc gcgcagtaga gcagcagcac 120
   aggcgcgggt cccgggaggc cggctctgct cgcgccgaga tgtggaatct ccttcacgaa 180
   accgactcgg ctgtggccac cgcgcgccgc ccgcgctggc tgtgcgctgg ggcgctggtg 240
   ctggcgggtg gcttctttct cctcggcttc ctcttcgggt ggtttataaa atcctccaat 300
   gaagctacta acattactcc aaagcataat atgaaagcat ttttggatga attgaaagct 360
   gagaacatca agaagttctt atataatttt acacagatac cacatttagc aggaacagaa 420
   caaaactttc agcttgcaaa gcaaattcaa tcccagtgga aagaatttgg cctggattct 480
   gttgagctag cacattatga tgtcctgttg tcctacccaa ataagactca tcccaactac 540
   atctcaataa ttaatgaaga tggaaatgag attttcaaca catcattatt tgaaccacct 600
   cctccaggat atgaaaatgt ttcggatatt gtaccacctt tcagtgcttt ctctcctcaa 660
   ggaatgccag agggcgatct agtgtatgtt aactatgcac gaactgaaga cttctttaaa 720
   ttggaacggg acatgaaaat caattgctct gggaaaattg taattgccag atatgggaaa 780
   gttttcagag gaaataaggt taaaaatgcc cagctggcag gggccaaagg agtcattctc     840
   tactccgacc ctgctgacta ctttgctcct ggggtgaagt cctatccaga tggttggaat     900
   cttcctggag gtggtgtcca gcgtggaaat atcctaaatc tgaatggtgc aggagaccct     960
   ctcacaccag gttacccagc aaatgaatat gcttataggc gtggaattgc agaggctgtt    1020
   ggtcttccaa gtattcctgt tcatccaatt ggatactatg atgcacagaa gctcctagaa    1080
   aaaatgggtg gctcagcacc accagatagc agctggagag gaagtctcaa agtgccctac    1140
   aatgttggac ctggctttac tggaaacttt tctacacaaa aagtcaagat gcacatccac    1200
   tctaccaatg aagtgacaag aatttacaat gtgataggta ctctcagagg agcagtggaa    1260
   ccagacagat atgtcattct gggaggtcac cgggactcat gggtgtttgg tggtattgac    1320
   cctcagagtg gagcagctgt tgttcatgaa attgtgagga gctttggaac actgaaaaag    1380
   gaagggtgga gacctagaag aacaattttg tttgcaagct gggatgcaga agaatttggt    1440
   cttcttggtt ctactgagtg ggcagaggag aattcaagac tccttcaaga gcgtggcgtg    1500
   gcttatatta atgctgactc atctatagaa ggaaactaca ctctgagagt tgattgtaca    1560
   ccgctgatgt acagcttggt acacaaccta acaaaagagc tgaaaagccc tgatgaaggc    1620
   tttgaaggca aatctcttta tgaaagttgg actaaaaaaa gtccttcccc agagttcagt    1680
   ggcatgccca ggataagcaa attgggatct ggaaatgatt ttgaggtgtt cttccaacga    1740
   cttggaattg cttcaggcag agcacggtat actaaaaatt gggaaacaaa caaattcagc    1800
   ggctatccac tgtatcacag tgtctatgaa acatatgagt tggtggaaaa gttttatgat    1860
   ccaatgttta aatatcacct cactgtggcc caggttcgag gagggatggt gtttgagcta    1920
   gccaattcca tagtgctccc ttttgattgt cgagattatg ctgtagtttt aagaaagtat    1980
   gctgacaaaa tctacagtat ttctatgaaa catccacagg aaatgaagac atacagtgta    2040
   tcatttgatt cacttttttc tgcagtaaag aattttacag aaattgcttc caagttcagt    2100
   gagagactcc aggactttga caaaagcaag catgtcatct atgctccaag cagccacaac    2160
   aagtatgcag gggagtcatt cccaggaatt tatgatgctc tgtttgatat tgaaagcaaa    2220
   gtggacccct ccaaggcctg gggagaagtg aagagacaga tttatgttgc agccttcaca    2280
   gtgcaggcag ctgcagagac tttgagtgaa gtagcctaag aggattcttt agagaatccg    2340
   tattgaattt gtgtggtatg tcactcagaa agaatcgtaa tgggtatatt gataaatttt    2400
   aaaattggta tatttgaaat aaagttgaat attatatata aaaaaaaaaa aaaaaaaaaa    2460
   aaaaaaaaaa aa                                                        2472
<210> 43
   <211> 719
   <212> PRT
   <213> Homo sapiens
<400> 43
   Met Trp Asn Leu Leu His Glu Thr Asp Ser Ala Val Ala Thr Ala Arg
   1 5 10 15
   Arg Pro Arg Trp Leu Cys Ala Gly Ala Leu Val Leu Ala Gly Gly Phe
   20 25 30
   Phe Leu Leu Gly Phe Leu Phe Gly Trp Phe Ile Lys Ser Ser Asn Glu
   35 40 45
   Ala Thr Asn Ile Thr Pro Lys His Asn Met Lys Ala Phe Leu Asp Glu
   50 55 60
   Leu Lys Ala Glu Asn Ile Lys Lys Phe Leu Tyr Asn Phe Thr Gln Ile
   65 70 75 80
   Pro His Leu Ala Gly Thr Glu Gln Asn Phe Gln Leu Ala Lys Gln Ile
   85 90 95
   Gln Ser Gln Trp Lys Glu Phe Gly Leu Asp Ser Val Glu Leu Ala His
   100 105 110
   Tyr Asp Val Leu Leu Ser Tyr Pro Asn Lys Thr His Pro Asn Tyr Ile
   115 120 125
   Ser Ile Ile Asn Glu Asp Gly Asn Glu Ile Phe Asn Thr Ser Leu Phe
   130 135 140
   Glu Pro Pro Pro Pro Gly Tyr Glu Asn Val Ser Asp Ile Val Pro Pro
   145 150 155 160
   Phe Ser Ala Phe Ser Pro Gln Gly Met Pro Glu Gly Asp Leu Val Tyr
   165 170 175
   Val Asn Tyr Ala Arg Thr Glu Asp Phe Phe Lys Leu Glu Arg Asp Met
   180 185 190
   Lys Ile Asn Cys Ser Gly Lys Ile Val Ile Ala Arg Tyr Gly Lys Val
   195 200 205
   Phe Arg Gly Asn Lys Val Lys Asn Ala Gln Leu Ala Gly Ala Lys Gly
   210 215 220
   Val Ile Leu Tyr Ser Asp Pro Ala Asp Tyr Phe Ala Pro Gly Val Lys
   225 230 235 240
   Ser Tyr Pro Asp Gly Trp Asn Leu Pro Gly Gly Gly Val Gln Arg Gly
   245 250 255
   Asn Ile Leu Asn Leu Asn Gly Ala Gly Asp Pro Leu Thr Pro Gly Tyr
   260 265 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385                 390                 395                 400
   Ser Phe Gly Thr Leu Lys Lys Glu Gly Trp Arg Pro Arg Arg Thr Ile
                   405                 410                 415
   Leu Phe Ala Ser Trp Asp Ala Glu Glu Phe Gly Leu Leu Gly Ser Thr
               420                 425                 430
   Glu Trp Ala Glu Glu Asn Ser Arg Leu Leu Gln Glu Arg Gly Val Ala
           435                 440                 445
   Tyr Ile Asn Ala Asp Ser Ser Ile Glu Gly Asn Tyr Thr Leu Arg Val
       450                 455                 460
   Asp Cys Thr Pro Leu Met Tyr Ser Leu Val His Asn Leu Thr Lys Glu
   465                 470                 475                 480
   Leu Lys Ser Pro Asp Glu Gly Phe Glu Gly Lys Ser Leu Tyr Glu Ser
                   485                 490                 495
   Trp Thr Lys Lys Ser Pro Ser Pro Glu Phe Ser Gly Met Pro Arg Ile
               500                 505                 510
   Ser Lys Leu Gly Ser Gly Asn Asp Phe Glu Val Phe Phe Gln Arg Leu
           515                 520                 525
   Gly Ile Ala Ser Gly Arg Ala Arg Tyr Thr Lys Asn Trp Glu Thr Asn
       530                 535                 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Lys His Val Ile Tyr Ala Pro Ser Ser His Asn Lys Tyr Ala Gly Glu
   660 665 670
   Ser Phe Pro Gly Ile Tyr Asp Ala Leu Phe Asp Ile Glu Ser Lys Val
   675 680 685
   Asp Pro Ser Lys Ala Trp Gly Glu Val Lys Arg Gln Ile Tyr Val Ala
   690 695 700
   Ala Phe Thr Val Gln Ala Ala Ala Glu Thr Leu Ser Glu Val Ala
   705 710 715
<210> 45
   <211> 671
   <212> PRT
   <213> Homo sapiens
<400> 45
   Ile Pro His Leu Ala Gly Thr Glu Gln Asn Phe Gln Leu Ala Lys Gln
   1 5 10 15
   Ile Gln Ser Gln Trp Lys Glu Phe Gly Leu Asp Ser Val Glu Leu Ala
   20 25 30
   His Tyr Asp Val Leu Leu Ser Tyr Pro Asn Lys Thr His Pro Asn Tyr
   35 40 45
   Ile Ser Ile Ile Asn Glu Asp Gly Asn Glu Ile Phe Asn Thr Ser Leu
   50 55 60
   Phe Glu Pro Pro Pro Pro Gly Tyr Glu Asn Val Ser Asp Ile Val Pro
   65 70 75 80
   Pro Phe Ser Ala Phe Ser Pro Gln Gly Met Pro Glu Gly Asp Leu Val
   85 90 95
   Tyr Val Asn Tyr Ala Arg Thr Glu Asp Phe Phe Lys Leu Glu Arg Asp
   100 105 110
   Met Lys Ile Asn Cys Ser Gly Lys Ile Val Ile Ala Arg Tyr Gly Lys
   115 120 125
   Val Phe Arg Gly Asn Lys Val Lys Asn Ala Gln Leu Ala Gly Ala Lys
   130 135 140
   Gly Val Ile Leu Tyr Ser Asp Pro Ala Asp Tyr Phe Ala Pro Gly Val
   145 150 155 160
   Lys Ser Tyr Pro Asp Gly Trp Asn Leu Pro Gly Gly Gly Val Gln Arg
   165 170 175
   Gly Asn Ile Leu Asn Leu Asn Gly Ala Gly Asp Pro Leu Thr Pro Gly
   180 185 190
   Tyr Pro Ala Asn Glu Tyr Ala Tyr Arg Arg Gly Ile Ala Glu Ala Val
   195 200 205
   Gly Leu Pro Ser Ile Pro Val His Pro Ile Gly Tyr Tyr Asp Ala Gln
   210 215 220
   Lys Leu Leu Glu Lys Met Gly Gly Ser Ala Pro Pro Asp Ser Ser Trp
   225 230 235 240
   Arg Gly Ser Leu Lys Val Pro Tyr Asn Val Gly Pro Gly Phe Thr Gly
   245 250 255
   Asn Phe Ser Thr Gln Lys Val Lys Met His Ile His Ser Thr Asn Glu
   260 265 270
   Val Thr Arg Ile Tyr Asn Val Ile Gly Thr Leu Arg Gly Ala Val Glu
   275 280 285
   Pro Asp Arg Tyr Val Ile Leu Gly Gly His Arg Asp Ser Trp Val Phe
   290 295 300
   Gly Gly Ile Asp Pro Gln Ser Gly Ala Ala Val Val His Glu Ile Val
   305 310 315 320
   Arg Ser Phe Gly Thr Leu Lys Lys Glu Gly Trp Arg Pro Arg Arg Thr
   325 330 335
   Ile Leu Phe Ala Ser Trp Asp Ala Glu Glu Phe Gly Leu Leu Gly Ser
   340 345 350
   Thr Glu Trp Ala Glu Glu Asn Ser Arg Leu Leu Gln Glu Arg Gly Val
   355 360 365
   Ala Tyr Ile Asn Ala Asp Ser Ser Ile Glu Gly Asn Tyr Thr Leu Arg
   370 375 380
   Val Asp Cys Thr Pro Leu Met Tyr Ser Leu Val His Asn Leu Thr Lys
   385 390 395 400
   Glu Leu Lys Ser Pro Asp Glu Gly Phe Glu Gly Lys Ser Leu Tyr Glu
   405 410 415
   Ser Trp Thr Lys Lys Ser Pro Ser Pro Glu Phe Ser Gly Met Pro Arg
   420 425 430
   Ile Ser Lys Leu Gly Ser Gly Asn Asp Phe Glu Val Phe Phe Gln Arg
   435 440 445
   Leu Gly Ile Ala Ser Gly Arg Ala Arg Tyr Thr Lys Asn Trp Glu Thr
   450 455 460
   Asn Lys Phe Ser Gly Tyr Pro Leu Tyr His Ser Val Tyr Glu Thr Tyr
   465 470 475 480
   Glu Leu Val Glu Lys Phe Tyr Asp Pro Met Phe Lys Tyr His Leu Thr
   485 490 495
   Val Ala Gln Val Arg Gly Gly Met Val Phe Glu Leu Ala Asn Ser Ile
   500 505 510
   Val Leu Pro Phe Asp Cys Arg Asp Tyr Ala Val Val Leu Arg Lys Tyr
   515 520 525
   Ala Asp Lys Ile Tyr Ser Ile Ser Met Lys His Pro Gln Glu Met Lys
   530 535 540
   Thr Tyr Ser Val Ser Phe Asp Ser Leu Phe Ser Ala Val Lys Asn Phe
   545 550 555 560
   Thr Glu Ile Ala Ser Lys Phe Ser Glu Arg Leu Gln Asp Phe Asp Lys
   565 570 575
   Ser Asn Pro Ile Val Leu Arg Met Met Asn Asp Gln Leu Met Phe Leu
   580 585 590
   Glu Arg Ala Phe Ile Asp Pro Leu Gly Leu Pro Asp Arg Pro Phe Tyr
   595 600 605
   Arg His Val Ile Tyr Ala Pro Ser Ser His Asn Lys Tyr Ala Gly Glu
   610 615 620
   Ser Phe Pro Gly Ile Tyr Asp Ala Leu Phe Asp Ile Glu Ser Lys Val
   625 630 635 640
   Asp Pro Ser Lys Ala Trp Gly Glu Val Lys Arg Gln Ile Tyr Val Ala
   645 650 655
   Ala Phe Thr Val Gln Ala Ala Ala Glu Thr Leu Ser Glu Val Ala
   660 665 670
<210> 46
   <211> 31
   <212> DNA
   <213> Artificial
<220>
   <223> chemically synthesized
<400> 46
   ggggtctaga cctcctttga ttagtatatt c 31
<210> 47
   <211> 45
   <212> DNA
   <213> Artificial
<220>
   <223> chemically synthesized
<400> 47
   atcttcgcta tctgtcgccg cggcgcgtgc ttcagtttgt tgcgc 45
<210> 48
   <211> 45
   <212> DNA
   <213> Artificial
<220>
   <223> chemically synthesized
<400> 48
   gcgcaacaaa ctgaagcagc ggccgcggcg acagatagcg aagat 45
<210> 49
   <211> 42
   <212> DNA
   <213> Artificial
<220>
   <223> chemically synthesized
<400> 49
   tgtaggtgta tctccatgct cgagagctag gcgatcaatt tc 42
<210> 50
   <211> 42
   <212> DNA
   <213> Artificial
<220>
   <223> chemically synthesized
<400> 50
   ggaattgatc gcctagctct cgagcatgga gatacaccta ca 42
<210> 51
   <211> 42
   <212> DNA
   <213> Artificial
<220>
   <223> chemically synthesized
<400> 51
   aaacggattt atttagatcc cgggttatgg tttctgagaa ca 42
<210> 52
   <211> 42
   <212> DNA
   <213> Artificial
<220>
   <223> chemically synthesized
<400> 52
   tgttctcaga aaccataacc cgggatctaa ataaatccgt tt 42
<210> 53
   <211> 28
   <212> DNA
   <213> Artificial
<220>
   <223> chemically synthesized
<400> 53
   gggggtcgac cagctcttct tggtgaag 28
<210> 54
   <211> 680
   <212> PRT
   <213> Artificial
<220>
   <223> chemically synthesized
<400> 54
<210> 55
   <211> 13294
   <212> DNA
   <213> Artificial
<220>
   <223> chemically synthesized
<400> 55
   aattccggat gagcattcat caggcgggca agaatgtgaa taaaggccgg ataaaacttg 60
   tgcttatttt tctttacggt ctttaaaaag gccgtaatat ccagctgaac ggtctggtta 120
   taggtacatt gagcaactga ctgaaatgcc tcaaaatgtt ctttacgatg ccattgggat 180
   atatcaacgg tggtatatcc agtgattttt ttctccattt tagcttcctt agctcctgaa 240
   aatctcgata actcaaaaaa tacgcccggt agtgatctta tttcattatg gtgaaagttg 300
   gaacctctta cgtgccgatc aacgtctcat tttcgccaaa agttggccca gggcttcccg 360
   gtatcaacag ggacaccagg atttatttat tctgcgaagt gatcttccgt cacaggtatt 420
   tattcggcgc aaagtgcgtc gggtgatgct gccaacttac tgatttagtg tatgatggtg 480
   tttttgaggt gctccagtgg cttctgtttc tatcagctgt ccctcctgtt cagctactga 540
   cggggtggtg cgtaacggca aaagcaccgc cggacatcag cgctagcgga gtgtatactg 600
   gcttactatg ttggcactga tgagggtgtc agtgaagtgc ttcatgtggc aggagaaaaa 660
   aggctgcacc ggtgcgtcag cagaatatgt gatacaggat atattccgct tcctcgctca 720
   ctgactcgct acgctcggtc gttcgactgc ggcgagcgga aatggcttac gaacggggcg 780
   gagatttcct ggaagatgcc aggaagatac ttaacaggga agtgagaggg ccgcggcaaa 840
   gccgtttttc cataggctcc gcccccctga caagcatcac gaaatctgac gctcaaatca 900
   gtggtggcga aacccgacag gactataaag ataccaggcg tttccccctg gcggctccct 960
   cgtgcgctct cctgttcctg cctttcggtt taccggtgtc attccgctgt tatggccgcg 1020
   tttgtctcat tccacgcctg acactcagtt ccgggtaggc agttcgctcc aagctggact 1080
   gtatgcacga accccccgtt cagtccgacc gctgcgcctt atccggtaac tatcgtcttg 1140
   agtccaaccc ggaaagacat gcaaaagcac cactggcagc agccactggt aattgattta 1200
   gaggagttag tcttgaagtc atgcgccggt taaggctaaa ctgaaaggac aagttttggt 1260
   gactgcgctc ctccaagcca gttacctcgg ttcaaagagt tggtagctca gagaaccttc 1320
   gaaaaaccgc cctgcaaggc ggttttttcg ttttcagagc aagagattac gcgcagacca 1380
   aaacgatctc aagaagatca tcttattaat cagataaaat atttctagcc ctcctttgat 1440
   tagtatattc ctatcttaaa gttactttta tgtggaggca ttaacatttg ttaatgacgt   1500
   caaaaggata gcaagactag aataaagcta taaagcaagc atataatatt gcgtttcatc   1560
   tttagaagcg aatttcgcca atattataat tatcaaaaga gaggggtggc aaacggtatt   1620
   tggcattatt aggttaaaaa atgtagaagg agagtgaaac ccatgaaaaa aataatgcta   1680
   gtttttatta cacttatatt agttagtcta ccaattgcgc aacaaactga agcaaaggat   1740
   gcatctgcat tcaataaaga aaattcaatt tcatccatgg caccaccagc atctccgcct   1800
   gcaagtccta agacgccaat cgaaaagaaa cacgcggatg aaatcgataa gtatatacaa   1860
   ggattggatt acaataaaaa caatgtatta gtataccacg gagatgcagt gacaaatgtg   1920
   ccgccaagaa aaggttacaa agatggaaat gaatatattg ttgtggagaa aaagaagaaa   1980
   tccatcaatc aaaataatgc agacattcaa gttgtgaatg caatttcgag cctaacctat   2040
   ccaggtgctc tcgtaaaagc gaattcggaa ttagtagaaa atcaaccaga tgttctccct   2100
   gtaaaacgtg attcattaac actcagcatt gatttgccag gtatgactaa tcaagacaat   2160
   aaaatagttg taaaaaatgc cactaaatca aacgttaaca acgcagtaaa tacattagtg   2220
   gaaagatgga atgaaaaata tgctcaagct tatccaaatg taagtgcaaa aattgattat   2280
   gatgacgaaa tggcttacag tgaatcacaa ttaattgcga aatttggtac agcatttaaa   2340
   gctgtaaata atagcttgaa tgtaaacttc ggcgcaatca gtgaagggaa aatgcaagaa   2400
   gaagtcatta gttttaaaca aatttactat aacgtgaatg ttaatgaacc tacaagacct   2460
   tccagatttt tcggcaaagc tgttactaaa gagcagttgc aagcgcttgg agtgaatgca   2520
   gaaaatcctc ctgcatatat ctcaagtgtg gcgtatggcc gtcaagttta tttgaaatta   2580
   tcaactaatt cccatagtac taaagtaaaa gctgcttttg atgctgccgt aagcggaaaa   2640
   tctgtctcag gtgatgtaga actaacaaat atcatcaaaa attcttcctt caaagccgta   2700
   atttacggag gttccgcaaa agatgaagtt caaatcatcg acggcaacct cggagactta   2760
   cgcgatattt tgaaaaaagg cgctactttt aatcgagaaa caccaggagt tcccattgct   2820
   tatacaacaa acttcctaaa agacaatgaa ttagctgtta ttaaaaacaa ctcagaatat   2880
   attgaaacaa cttcaaaagc ttatacagat ggaaaaatta acatcgatca ctctggagga   2940
   tacgttgctc aattcaacat ttcttgggat gaagtaaatt atgatctcga gattgtggga   3000
   ggctgggagt gcgagaagca ttcccaaccc tggcaggtgc ttgtggcctc tcgtggcagg   3060
   gcagtctgcg gcggtgttct ggtgcacccc cagtgggtcc tcacagctgc ccactgcatc   3120
   aggaacaaaa gcgtgatctt gctgggtcgg cacagcctgt ttcatcctga agacacaggc   3180
   caggtatttc aggtcagcca cagcttccca cacccgctct acgatatgag cctcctgaag   3240
   aatcgattcc tcaggccagg tgatgactcc agccacgacc tcatgctgct ccgcctgtca   3300
   gagcctgccg agctcacgga tgctgtgaag gtcatggacc tgcccaccca ggagccagca   3360
   ctggggacca cctgctacgc ctcaggctgg ggcagcattg aaccagagga gttcttgacc   3420
   ccaaagaaac ttcagtgtgt ggacctccat gttatttcca atgacgtgtg tgcgcaagtt   3480
   caccctcaga aggtgaccaa gttcatgctg tgtgctggac gctggacagg gggcaaaagc   3540
   acctgctcgg gtgattctgg gggcccactt gtctgttatg gtgtgcttca aggtatcacg   3600
   tcatggggca gtgaaccatg tgccctgccc gaaaggcctt ccctgtacac caaggtggtg   3660
   cattaccgga agtggatcaa ggacaccatc gtggccaacc cctaaactag tgactacaag   3720
   gacgatgacg acaagtgata cccgggatct aaataaatcc gtttttaaat atgtatgcat   3780
   ttcttttgcg aaatcaaaat ttgtataata aaatcctata tgtaaaaaac atcatttagc   3840
   gtgactttct ttcaacagct aacaattgtt gttactgcct aatgttttta gggtatttta   3900
   aaaaagggcg ataaaaaacg attgggggat gagacatgaa cgctcaagca gaagaattca   3960
   aaaaatattt agaaactaac gggataaaac caaaacaatt tcataaaaaa gaacttattt   4020
   ttaaccaatg ggatccacaa gaatattgta ttttcctata tgatggtatc acaaagctca   4080
   cgagtattag cgagaacggg accatcatga atttacaata ctacaaaggg gctttcgtta   4140
   taatgtctgg ctttattgat acagaaacat cggttggcta ttataattta gaagtcatta   4200
   gcgagcaggc taccgcatac gttatcaaaa taaacgaact aaaagaacta ctgagcaaaa   4260
   atcttacgca ctttttctat gttttccaaa ccctacaaaa acaagtttca tacagcctag   4320
   ctaaatttaa tgatttttcg attaacggga agcttggctc tatttgcggt caacttttaa   4380
   tcctgaccta tgtgtatggt aaagaaactc ctgatggcat caagattaca ctggataatt   4440
   taacaatgca ggagttagga tattcaagtg gcatcgcaca tagctcagct gttagcagaa   4500
   ttatttccaa attaaagcaa gagaaagtta tcgtgtataa aaattcatgc ttttatgtac   4560
   aaaatcgtga ttatctcaaa agatatgccc ctaaattaga tgaatggttt tatttagcat   4620
   gtcctgctac ttggggaaaa ttaaattaaa tcaaaaacag tattcctcaa tgaggaatac   4680
   tgttttatat tttattcgaa taaagaactt acagaagcat tttcatgaac gcgtacgatt   4740
   gcttcaccaa gaagagctgg tcgaccgatg cccttgagag ccttcaaccc agtcagctcc   4800
   ttccggtggg cgcggggcat gactatcgtc gccgcactta tgactgtctt ctttatcatg   4860
   caactcgtag gacaggtgcc ggcagcgctc tgggtcattt tcggcgagga ccgctttcgc   4920
   tggagcgcga cgatgatcgg cctgtcgctt gcggtattcg gaatcttgca cgccctcgct   4980
   caagccttcg tcactggtcc cgccaccaaa cgtttcggcg agaagcaggc cattatcgcc   5040
   ggcatggcgg ccgacgcgct gggctacgtc ttgctggcgt tcgcgacgcg aggctggatg   5100
   gccttcccca ttatgattct tctcgcttcc ggcggcatcg ggatgcccgc gttgcaggcc   5160
   atgctgtcca ggcaggtaga tgacgaccat cagggacagc ttcaaggatc gctcgcggct   5220
   cttaccagcc taacttcgat cattggaccg ctgatcgtca cggcgattta tgccgcctcg   5280
   gcgagcacat ggaacgggtt ggcatggatt gtaggcgccg ccctatacct tgtctgcctc   5340
   cccgcgttgc gtcgcggtgc atggagccgg gccacctcga cctgaatgga agccggcggc   5400
   acctcgctaa cggattcacc actccaagaa ttggagccaa tcaattcttg cggagaactg   5460
   tgaatgcgca aaccaaccct tggcagaaca tatccatcgc gtccgccatc tccagcagcc   5520
   gcacgcggcg catctcggct ttcgatttgt ttttgaatgg tttatccgat aaagaagttg 5580
   aagaacaaac tggaatcaat cgccgaacgt ttagaaggta tcgagcaaga tataacgtga 5640
   cagtcgatca aagaaaaaac aatgaaaaga gggatagtta atgagtacgg ttattttagc 5700
   tgaaaaacca agccaggcat tagcctacgc aagtgcttta aaacaaagca ccaaaaaaga 5760
   cggttatttt gagatcaaag acccactatt tacagatgaa acgtttatca cctttggttt 5820
   tgggcattta gtggaattag cagaaccagg tcattatgac gaaaagtggc aaaattggaa 5880
   acttgaatct ttgccgattt ttcctgatcg atacgatttt gaagttgcaa aagataaggg 5940
   aaagcagttt aaaattgttg cagaacttct caaaaaggca aatacaatta ttgttgcaac 6000
   agatagcgac agagaaggtg aaaatatcgc ctggtcgatt atccataaag caaatgcctt 6060
   ttcaaaagat aaaacattta aaagactatg gatcaatagc ttagaaaaag atgtaatccg 6120
   aagcggtttt caaaatttgc aacctggaat gaattactat cccttttatc aagaagcgca 6180
   aacacgccaa attgccgatt ggttgatcgg catgaacgca agccctttgt atacgttaaa 6240
   tttacaacag aagggcgtac aaggtacatt ttcactagga cgtgttcaaa cgcccacctt 6300
   ataccttatt tttcagcgcc aggaagccat agagaatttt aaaaaagaac cttttttcga 6360
   ggtggaagct agtataaaag taaaccaagg gtcgtttaag ggcgttctaa gccccacaca 6420
   gcgttttaaa acccaagagg agcttttagc ttttgtttct tctaaacaag ctaaaatagg 6480
   caatcaagag gggataattg ctgatgttca aaccaaagag aagaaaacga atagtccgag 6540
   tttgttttct ttaagtagtt tgcaatcaaa agtcaatcag ctttataaag cgacagcgag 6600
   ccaaacttta aaagctattt cttttttaat aacttaaaaa taaacttaat gtaacagcaa 6660
   gcacagtcaa ggtatacacc tttgacaaaa aatagcacat tctctatcga aaatttttgc 6720
   ttatttttta aattattttg ggaaattttc ccaatccctt tttctaactc aaaaaatata 6780
   atcactcaaa atttaaaagg gcgcacttat acatcatttt aaaaaattga tgtaacgtgc 6840
   taagttcaaa acaaagggcg cacttataca cgattttcaa tcttgtatat ttctaacgaa 6900
   aagcgtgcgc caaaaaaccc ccttcgtcaa ttttgacagg gggctttttg atgtaaaaat 6960
   ttctatcgaa atttaaaaat tcgcttcact catgttataa agacttaaaa taaaataact 7020
   ctttaaaatc ttttgctagt tgttcttcaa tattttttat tcggtgcatc ttccaagtaa 7080
   agtataacac actagactta tttactacgt ttcataagtc attaatgcgt gtgctctgcg 7140
   aggctagttt ttgtgcaagc acaaaaaatg gactgaataa atcagtccat aagttcaaaa 7200
   ccaaattcaa aatcaaaacc acaagcaacc aaaaaatgtg gttgttatac gttcataaat 7260
   tttatgatca cttacgtgta taaaattaaa ttcactttca aaatctaaaa actaaatcca 7320
   atcatctacc ctatgaatta tatcttgaaa ttcattcata aatagtgaag catggtaacc 7380
   atcacataca gaatgatgaa gttgcagagc aactggtata taaattttat tattctcact 7440
   ataaaattta cctatcgtaa taataggcaa taaaaagctg ctattgttac caatatttaa 7500
   attaaatgaa ctaaaatcaa tccaaggaat cattgaaatc ggtatggtgt tttcaggtat 7560
   cggtttttta ggaaacattt cttctttatc tttatattca agcaagtcat ttttataatt 7620
   attataaaaa gaaatgaagt ttttatcaga ttcagtccaa atgttagtaa atttttcagt 7680
   ttgcttatta aaaactgtat acaaaggatt taacttatcc caataaccta atttattctc 7740
   actattaatt cctgttctaa acactttatt tttatttaca acttccataa ttgcataaat 7800
   taaagaggga taaatttcat atcctttctt ttttatcata tctttaaaca aagtaatatc 7860
   aatttcttta gtaatgctat aagtagtttg ctgattaaaa tagtgttcaa aatattcttt 7920
   tctatcccaa ttttctaatt caataatatt aaaagtcata tataacttcc tcctaaattt 7980
   taaattttta tatttaggag gaataatcct ctgatttttt catacgttat gtcacctcgt 8040
   aaatattaat tatactgaat tagcaatttt tatcaaataa aacttatttt acttccaaaa 8100
   cctaaattca cgttgccaaa aatcaatctg cttttgcaat tgtttttcgt tcgcttttaa 8160
   agtcgatttc attaattccg ttaaatcaat tggagatatt tctctaatca attttttaaa 8220
   tttagtctta gtattcttac ttagctttcc ccacatactt tcttcatgca acaaagtata 8280
   aaccatagct tgctcattaa ttttttctaa agtagcccac gcaggtttca agatgtgtaa 8340
   atcattaaaa caatcattcc agtaatcaac catatctctt tttaattcaa cttctacacg 8400
   ccataaatgt tcagacacaa cttcaacatc tgcgttatct ttacgttctt gttttttatt 8460
   ataaattcta ataaatctat cactatcacg gacaccaaaa tattttgttt ctggcttgcc 8520
   attacgacca taaaaaacag ttttcttaac tgctttatca gtcattgcat agtaatcgct 8580
   caaatcatct tcaaaatcaa aagctaagtc taatcttgta aaaccgtcat cttccatgta 8640
   gtcgataata ttttgtttta accaaatcat ttcttcatgt gtgagtttat tgggattaaa 8700
   ttcaacacgc atattacgtc tatcccaagt atctgctttt actttgtcat attcgatata 8760
   aactttttct tgaagtgcct tagctttaaa ctttgtttga agtatatccc aaagtcgtat 8820
   ttgtggctct acactcataa agtcagatag ctttttagca ttagttttgt tcaaatttcc 8880
   aacgattgtc atggcatcaa aacttaatgc gggttgagat tttcccaaag tttgaccact 8940
   taaccggcta ttacttaacc ggctattaga gacggaacta actcaacgct agtagtggat 9000
   ttaatcccaa atgagccaac agaaccagaa ccagaaacag aacaagtaac attggagtta 9060
   gaaatggaag aagaaaaaag caatgatttc gtgtgaataa tgcacgaaat cattgcttat 9120
   ttttttaaaa agcgatatac tagatataac gaaacaacga actgaataaa gaatacaaaa 9180
   aaagagccac gaccagttaa agcctgagaa actttaactg cgagccttaa ttgattacca 9240
   ccaatcaatt aaagaagtcg agacccaaaa tttggtaaag tatttaatta ctttattaat 9300
   cagatactta aatatctgta aacccattat atcgggtttt tgaggggatt tcaagtcttt 9360
   aagaagatac caggcaatca attaagaaaa acttagttga ttgccttttt tgttgtgatt 9420
   caactttgat cgtagcttct aactaattaa ttttcgtaag aaaggagaac agctgaatga 9480
   atatcccttt tgttgtagaa actgtgcttc atgacggctt gttaaagtac aaatttaaaa 9540
   atagtaaaat tcgctcaatc actaccaagc caggtaaaag taaaggggct atttttgcgt 9600
   atcgctcaaa aaaaagcatg attggcggac gtggcgttgt tctgacttcc gaagaagcga 9660
   ttcacgaaaa tcaagataca tttacgcatt ggacaccaaa cgtttatcgt tatggtacgt 9720
   atgcagacga aaaccgttca tacactaaag gacattctga aaacaattta agacaaatca 9780
   ataccttctt tattgatttt gatattcaca cggaaaaaga aactatttca gcaagcgata 9840
   ttttaacaac agctattgat ttaggtttta tgcctacgtt aattatcaaa tctgataaag 9900
   gttatcaagc atattttgtt ttagaaacgc cagtctatgt gacttcaaaa tcagaattta 9960
   aatctgtcaa agcagccaaa ataatctcgc aaaatatccg agaatatttt ggaaagtctt 10020
   tgccagttga tctaacgtgc aatcattttg ggattgctcg tataccaaga acggacaatg 10080
   tagaattttt tgatcccaat taccgttatt ctttcaaaga atggcaagat tggtctttca 10140
   aacaaacaga taataagggc tttactcgtt caagtctaac ggttttaagc ggtacagaag 10200
   gcaaaaaaca agtagatgaa ccctggttta atctcttatt gcacgaaacg aaattttcag 10260
   gagaaaaggg tttagtaggg cgcaatagcg ttatgtttac cctctcttta gcctacttta 10320
   gttcaggcta ttcaatcgaa acgtgcgaat ataatatgtt tgagtttaat aatcgattag 10380
   atcaaccctt agaagaaaaa gaagtaatca aaattgttag aagtgcctat tcagaaaact 10440
   atcaaggggc taatagggaa tacattacca ttctttgcaa agcttgggta tcaagtgatt 10500
   taaccagtaa agatttattt gtccgtcaag ggtggtttaa attcaagaaa aaaagaagcg 10560
   aacgtcaacg tgttcatttg tcagaatgga aagaagattt aatggcttat attagcgaaa 10620
   aaagcgatgt atacaagcct tatttagcga cgaccaaaaa agagattaga gaagtgctag 10680
   gcattcctga acggacatta gataaattgc tgaaggtact gaaggcgaat caggaaattt 10740
   tctttaagat taaaccagga agaaatggtg gcattcaact tgctagtgtt aaatcattgt 10800
   tgctatcgat cattaaatta aaaaaagaag aacgagaaag ctatataaag gcgctgacag 10860
   cttcgtttaa tttagaacgt acatttattc aagaaactct aaacaaattg gcagaacgcc 10920
   ccaaaacgga cccacaactc gatttgttta gctacgatac aggctgaaaa taaaacccgc 10980
   actatgccat tacatttata tctatgatac gtgtttgttt ttctttgctg tttagtgaat 11040
   gattagcaga aatatacaga gtaagatttt aattaattat tagggggaga aggagagagt 11100
   agcccgaaaa cttttagttg gcttggactg aacgaagtga gggaaaggct actaaaacgt 11160
   cgaggggcag tgagagcgaa gcgaacactt gatcttttaa gttgctatca tttataggtc 11220
   aatagagtat acctatttgt cctaatatga ttttagcagt ataattgact tggtgaatag 11280
   gtcatttaag ttgggcataa taggaggagt aaaatgaaaa aatttattta tcgagtttta 11340
   gaaaatgacg aagtggtggc tatttttaat gagcaacaat atgcgcaaga ttttatcgct 11400
   tacgaaaaga caatttctga taagcaattt gaaattgaaa aagtagatat tgctgattgg 11460
   ttattgcaac cgagagaatt ttagaggttg gttgaaaatg gctaaaattg gttatgcacg 11520
   tgtcagtagc aaagaacaga acttagatcg gcaattacaa gcgttacagg gcgtttctaa 11580
   ggtcttttca gacaaattaa gcggtcaatc ggtcgaacgc ccacaattac aagctatgct 11640
   taactatatt cgtgaagggg atattgttat tgttactgaa ttagatcgat taggacgaaa 11700
   taataaagaa ttaacagaat tgatgaatca aattcaaatt aagggggcaa ccctggaagt 11760
   cttaaattta ccctcaatga atggtattga agatgaaaat ttaaggcgtt tgattaatag 11820
   ccttgtcatt gaattgtaca agtatcaagc agaatcagaa cgaaaaaaaa ttaaggaacg 11880
   tcaggcacaa ggaatcgaaa ttgctaagaa aaaaggcaaa ttcaaaggtc gtcagcataa 11940
   atttaaagaa aatgatccac gtttaaagtc gggcagcgtt gggtcctggc cacgggtgcg 12000
   catgatcgtg ctcctgtcgt tgaggacccg gctaggctgg cggggttgcc ttactggtta 12060
   gcagaatgaa tcaccgatac gcgagcgaac gtgaagcgac tgctgctgca aaacgtctgc 12120
   gacctgagca acaacatgaa tggtcttcgg tttccgtgtt tcgtaaagtc tggaaacgcg 12180
   gaagtcccct acgtgctgct gaagttgccc gcaacagaga gtggaaccaa ccggtgatac 12240
   cacgatacta tgactgagag tcaacgccat gagcggcctc atttcttatt ctgagttaca 12300
   acagtccgca ccgctgccgg tagctccttc cggtgggcgc ggggcatgac tatcgtcgcc 12360
   gcacttatga ctgtcttctt tatcatgcaa ctcgtaggac aggtgccggc agcgcccaac 12420
   agtcccccgg ccacggggcc tgccaccata cccacgccga aacaagcgcc ctgcaccatt 12480
   atgttccgga tctgcatcgc aggatgctgc tggctaccct gtggaacacc tacatctgta 12540
   ttaacgaagc gctaaccgtt tttatcaggc tctgggaggc agaataaatg atcatatcgt 12600
   caattattac ctccacgggg agagcctgag caaactggcc tcaggcattt gagaagcaca 12660
   cggtcacact gcttccggta gtcaataaac cggtaaacca gcaatagaca taagcggcta 12720
   tttaacgacc ctgccctgaa ccgacgaccg ggtcgaattt gctttcgaat ttctgccatt 12780
   catccgctta ttatcactta ttcaggcgta gcaaccaggc gtttaagggc accaataact 12840
   gccttaaaaa aattacgccc cgccctgcca ctcatcgcag tactgttgta attcattaag 12900
   cattctgccg acatggaagc catcacaaac ggcatgatga acctgaatcg ccagcggcat 12960
   cagcaccttg tcgccttgcg tataatattt gcccatggtg aaaacggggg cgaagaagtt 13020
   gtccatattg gccacgttta aatcaaaact ggtgaaactc acccagggat tggctgagac 13080
   gaaaaacata ttctcaataa accctttagg gaaataggcc aggttttcac cgtaacacgc 13140
   cacatcttgc gaatatatgt gtagaaactg ccggaaatcg tcgtggtatt cactccagag 13200
   cgatgaaaac gtttcagttt gctcatggaa aacggtgtaa caagggtgaa cactatccca 13260
   tatcaccagc tcaccgtctt tcattgccat acgg 13294
<210> 56
   <211> 368
   <212> PRT
   <213> Listeria monocytogenes
<400> 56
   Met Val Thr Gly Trp His Arg Pro Thr Trp Ile Glu Ile Asp Arg Ala
   1               5                   10                  15
   Ala Ile Arg Glu Asn Ile Lys Asn Glu Gln Asn Lys Leu Pro Glu Ser
   20 25 30
   Val Asp Leu Trp Ala Val Val Lys Ala Asn Ala Tyr Gly His Gly Ile
   35 40 45
   Ile Glu Val Ala Arg Thr Ala Lys Glu Ala Gly Ala Lys Gly Phe Cys
   50 55 60
   Val Ala Ile Leu Asp Glu Ala Leu Ala Leu Arg Glu Ala Gly Phe Gln
   65 70 75 80
   Asp Asp Phe Ile Leu Val Leu Gly Ala Thr Arg Lys Glu Asp Ala Asn
   85 90 95
   Leu Ala Ala Lys Asn His Ile Ser Leu Thr Val Phe Arg Glu Asp Trp
   100 105 110
   Leu Glu Asn Leu Thr Leu Glu Ala Thr Leu Arg Ile His Leu Lys Val
   115 120 125
   Asp Ser Gly Met Gly Arg Leu Gly Ile Arg Thr Thr Glu Glu Ala Arg
   130 135 140
   Arg Ile Glu Ala Thr Ser Thr Asn Asp His Gln Leu Gln Leu Glu Gly
   145 150 155 160
   Ile Tyr Thr His Phe Ala Thr Ala Asp Gln Leu Glu Thr Ser Tyr Phe
   165 170 175
   Glu Gln Gln Leu Ala Lys Phe Gln Thr Ile Leu Thr Ser Leu Lys Lys
   180 185 190
   Arg Pro Thr Tyr Val His Thr Ala Asn Ser Ala Ala Ser Leu Leu Gln
   195 200 205
   Pro Gln Ile Gly Phe Asp Ala Ile Arg Phe Gly Ile Ser Met Tyr Gly
   210 215 220
   Leu Thr Pro Ser Thr Glu Ile Lys Thr Ser Leu Pro Phe Glu Leu Lys
   225 230 235 240
   Pro Ala Leu Ala Leu Tyr Thr Glu Met Val His Val Lys Glu Leu Ala
   245 250 255
   Pro Gly Asp Ser Val Ser Tyr Gly Ala Thr Tyr Thr Ala Thr Glu Arg
   260 265 270
   Glu Trp Val Ala Thr Leu Pro Ile Gly Tyr Ala Asp Gly Leu Ile Arg
   275 280 285
   His Tyr Ser Gly Phe His Val Leu Val Asp Gly Glu Pro Ala Pro Ile
   290 295 300
   Ile Gly Arg Val Cys Met Asp Gln Thr Ile Ile Lys Leu Pro Arg Glu
   305 310 315 320
   Phe Gln Thr Gly Ser Lys Val Thr Ile Ile Gly Lys Asp His Gly Asn
   325 330 335
   Thr Val Thr Ala Asp Asp Ala Ala Gln Tyr Leu Asp Thr Ile Asn Tyr
   340 345 350
   Glu Val Thr Cys Leu Leu Asn Glu Arg Ile Pro Arg Lys Tyr Ile His
   355 360 365
<210> 57
   <211> 289
   <212> PRT
   <213> Listeria monocytogenes
<400> 57
   Met Lys Val Leu Val Asn Asn His Leu Val Glu Arg Glu Asp Ala Thr
   1 5 10 15
   Val Asp Ile Glu Asp Arg Gly Tyr Gln Phe Gly Asp Gly Val Tyr Glu
   20 25 30
   Val Val Arg Leu Tyr Asn Gly Lys Phe Phe Thr Tyr Asn Glu His Ile
   35 40 45
   Asp Arg Leu Tyr Ala Ser Ala Ala Lys Ile Asp Leu Val Ile Pro Tyr
   50 55 60
   Ser Lys Glu Glu Leu Arg Glu Leu Leu Glu Lys Leu Val Ala Glu Asn
   65 70 75 80
   Asn Ile Asn Thr Gly Asn Val Tyr Leu Gln Val Thr Arg Gly Val Gln
   85 90 95
   Asn Pro Arg Asn His Val Ile Pro Asp Asp Phe Pro Leu Glu Gly Val
   100 105 110
   Leu Thr Ala Ala Ala Arg Glu Val Pro Arg Asn Glu Arg Gln Phe Val
   115 120 125
   Glu Gly Gly Thr Ala Ile Thr Glu Glu Asp Val Arg Trp Leu Arg Cys
   130 135 140
   Asp Ile Lys Ser Leu Asn Leu Leu Gly Asn Ile Leu Ala Lys Asn Lys
   145 150 155 160
   Ala His Gln Gln Asn Ala Leu Glu Ala Ile Leu His Arg Gly Glu Gln
   165 170 175
   Val Thr Glu Cys Ser Ala Ser Asn Val Ser Ile Ile Lys Asp Gly Val
   180 185 190
   Leu Trp Thr His Ala Ala Asp Asn Leu Ile Leu Asn Gly Ile Thr Arg
   195 200 205
   Gln Val Ile Ile Asp Val Ala Lys Lys Asn Gly Ile Pro Val Lys Glu
   210 215 220
   Ala Asp Phe Thr Leu Thr Asp Leu Arg Glu Ala Asp Glu Val Phe Ile
   225 230 235 240
   Ser Ser Thr Thr Ile Glu Ile Thr Pro Ile Thr His Ile Asp Gly Val
   245 250 255
   Gln Val Ala Asp Gly Lys Arg Gly Pro Ile Thr Ala Gln Leu His Gln
   260 265 270
   Tyr Phe Val Glu Glu Ile Thr Arg Ala Cys Gly Glu Leu Glu Phe Ala
   275 280 285
   Lys
<210> 58
   <211> 29
   <212> DNA
   <213> Artificial
<220>
   <223> chemically synthesized
<400> 58
   gtgctcgaga ttgtgggagg ctgggagtg 29
<210> 59
   <211> 29
   <212> DNA
   <213> Artificial
<220>
   <223> chemically synthesized
<400> 59
   gatactagtt taggggttgg ccacgatgg 29
<210> 60
   <211> 10
   <212> PRT
   <213> Homo sapiens
<400> 60
   His Cys Ile Arg Asn Lys Ser Val Ile Leu
   1 5 10
<210> 61
   <211> 390
   <212> PRT
   <213> Listeria monocytogenes
<400> 61
   Met Arg Ala Met Met Val Val Phe Ile Thr Ala Asn Cys Ile Thr Ile
   1               5                   10                  15
   Asn Pro Asp Ile Ile Phe Ala Ala Thr Asp Ser Glu Asp Ser Ser Leu
               20                  25                  30
   Asn Thr Asp Glu Trp Glu Glu Glu Lys Thr Glu Glu Gln Pro Ser Glu
           35                  40                  45
   Val Asn Thr Gly Pro Arg Tyr Glu Thr Ala Arg Glu Val Ser Ser Arg
       50                  55                  60
   Asp Ile Glu Glu Leu Glu Lys Ser Asn Lys Val Lys Asn Thr Asn Lys
   65                  70                  75                  80
   Ala Asp Leu Ile Ala Met Leu Lys Ala Lys Ala Glu Lys Gly Pro Asn
                   85                  90                  95
   Asn Asn Asn Asn Asn Gly Glu Gln Thr Gly Asn Val Ala Ile Asn Glu
               100                 105                 110
   Glu Ala Ser Gly Val Asp Arg Pro Thr Leu Gln Val Glu Arg Arg His
           115                 120                 125
   Pro Gly Leu Ser Ser Asp Ser Ala Ala Glu Ile Lys Lys Arg Arg Lys
       130                 135                 140
   Ala Ile Ala Ser Ser Asp Ser Glu Leu Glu Ser Leu Thr Tyr Pro Asp
   145                 150                 155                 160
   Lys Pro Thr Lys Ala Asn Lys Arg Lys Val Ala Lys Glu Ser Val Val
                   165                 170                 175
   Asp Ala Ser Glu Ser Asp Leu Asp Ser Ser Met Gln Ser Ala Asp Glu
               180                 185                 190
   Ser Thr Pro Gln Pro Leu Lys Ala Asn Gln Lys Pro Phe Phe Pro Lys
           195                 200                 205
   Val Phe Lys Lys Ile Lys Asp Ala Gly Lys Trp Val Arg Asp Lys Ile
       210                 215                 220
   Asp Glu Asn Pro Glu Val Lys Lys Ala Ile Val Asp Lys Ser Ala Gly
   225                 230                 235                 240
   Leu Ile Asp Gln Leu Leu Thr Lys Lys Lys Ser Glu Glu Val Asn Ala
                   245                 250                 255
   Ser Asp Phe Pro Pro Pro Pro Thr Asp Glu Glu Leu Arg Leu Ala Leu
               260                 265                 270
   Pro Glu Thr Pro Met Leu Leu Gly Phe Asn Ala Pro Thr Pro Ser Glu
           275                 280                 285
   Pro Ser Ser Phe Glu Phe Pro Pro Pro Pro Thr Asp Glu Glu Leu Arg
       290                 295                 300
   Leu Ala Leu Pro Glu Thr Pro Met Leu Leu Gly Phe Asn Ala Pro Ala
   305                 310                 315                 320
   Thr Ser Glu Pro Ser Ser Phe Glu Phe Pro Pro Pro Pro Thr Glu Asp
                   325                 330                 335
   Glu Leu Glu Ile Met Arg Glu Thr Ala Pro Ser Leu Asp Ser Ser Phe
               340                 345                 350
   Thr Ser Gly Asp Leu Ala Ser Leu Arg Ser Ala Ile Asn Arg His Ser
           355                 360                 365
   Glu Asn Phe Ser Asp Phe Pro Leu Ile Pro Thr Glu Glu Glu Leu Asn
       370                 375                 380
   Gly Arg Gly Gly Arg Pro
   385                 390
<210> 62
   <211> 1170
   <212> DNA
   <213> Listeria monocytogenes
<400> 62
   atgcgtgcga tgatggtagt tttcattact gccaactgca ttacgattaa ccccgacata      60
   atatttgcag cgacagatag cgaagattcc agtctaaaca cagatgaatg ggaagaagaa     120
   aaaacagaag agcagccaag cgaggtaaat acgggaccaa gatacgaaac tgcacgtgaa     180
   gtaagttcac gtgatattga ggaactagaa aaatcgaata aagtgaaaaa tacgaacaaa     240
   gcagacctaa tagcaatgtt gaaagcaaaa gcagagaaag gtccgaataa caataataac     300
   aacggtgagc aaacaggaaa tgtggctata aatgaagagg cttcaggagt cgaccgacca     360
   actctgcaag tggagcgtcg tcatccaggt ctgtcatcgg atagcgcagc ggaaattaaa     420
   aaaagaagaa aagccatagc gtcgtcggat agtgagcttg aaagccttac ttatccagat     480
   aaaccaacaa aagcaaataa gagaaaagtg gcgaaagagt cagttgtgga tgcttctgaa     540
   agtgacttag attctagcat gcagtcagca gacgagtcta caccacaacc tttaaaagca     600
   aatcaaaaac catttttccc taaagtattt aaaaaaataa aagatgcggg gaaatgggta     660
   cgtgataaaa tcgacgaaaa tcctgaagta aagaaagcga ttgttgataa aagtgcaggg     720
   ttaattgacc aattattaac caaaaagaaa agtgaagagg taaatgcttc ggacttcccg     780
   ccaccaccta cggatgaaga gttaagactt gctttgccag agacaccgat gcttctcggt     840
   tttaatgctc ctactccatc ggaaccgagc tcattcgaat ttccgccgcc acctacggat     900
   gaagagttaa gacttgcttt gccagagacg ccaatgcttc ttggttttaa tgctcctgct     960
   acatcggaac cgagctcatt cgaatttcca ccgcctccaa cagaagatga actagaaatt    1020
   atgcgggaaa cagcaccttc gctagattct agttttacaa gcggggattt agctagtttg    1080
   agaagtgcta ttaatcgcca tagcgaaaat ttctctgatt tcccactaat cccaacagaa    1140
   gaagagttga acgggagagg cggtagacca                                     1170
<210> 63
   <211> 123
   <212> PRT
   <213> Homo sapiens
<400> 63
   Met Lys Ala Val Leu Leu Ala Leu Leu Met Ala Gly Leu Ala Leu Gln
   1               5                   10                  15
   Pro Gly Thr Ala Leu Leu Cys Tyr Ser Cys Lys Ala Gln Val Ser Asn
               20                  25                  30
   Glu Asp Cys Leu Gln Val Glu Asn Cys Thr Gln Leu Gly Glu Gln Cys
           35                  40                  45
   Trp Thr Ala Arg Ile Arg Ala Val Gly Leu Leu Thr Val Ile Ser Lys
       50                  55                  60
   Gly Cys Ser Leu Asn Cys Val Asp Asp Ser Gln Asp Tyr Tyr Val Gly
   65                  70                  75                  80
   Lys Lys Asn Ile Thr Cys Cys Asp Thr Asp Leu Cys Asn Ala Ser Gly
                   85                  90                  95
   Ala His Ala Leu Gln Pro Ala Ala Ala Ile Leu Ala Leu Leu Pro Ala
               100                 105                 110
   Leu Gly Leu Leu Leu Trp Gly Pro Gly Gln Leu
           115                 120
<210> 64
   <211> 990
   <212> DNA
   <213> Homo sapiens
<220>
   <221> misc_feature
   <222> (543)..(543)
   <223> n is a, c, g, or t
<220>
   <221> misc_feature
   <222> (580)..(580)
   <223> n is a, c, g, or t
<220>
   <221> misc_feature
   <222> (584)..(584)
   <223> n is a, c, g, or t
<220>
   <221> misc_feature
   <222> (604)..(604)
   <223> n is a, c, g, or t
<220>
   <221> misc_feature
   <222> (608)..(608)
   <223> n is a, c, g, or t
<220>
   <221> misc_feature
   <222> (615)..(615)
   <223> n is a, c, g, or t
<220>
   <221> misc_feature
   <222> (636)..(636)
   <223> n is a, c, g, or t
<220>
   <221> misc_feature
   <222> (640)..(640)
   <223> n is a, c, g, or t
<220>
   <221> misc_feature
   <222> (646)..(646)
   <223> n is a, c, g, or t
<220>
   <221> misc_feature
   <222> (697)..(697)
   <223> n is a, c, g, or t
<220>
   <221> misc_feature
   <222> (926)..(926)
   <223> n is a, c, g, or t
<400> 64
   agggagaggc agtgaccatg aaggctgtgc tgcttgccct gttgatggca ggcttggccc      60
   tgcagccagg cactgccctg ctgtgctact cctgcaaagc ccaggtgagc aacgaggact     120
   gcctgcaggt ggagaactgc acccagctgg gggagcagtg ctggaccgcg cgcatccgcg     180
   cagttggcct cctgaccgtc atcagcaaag gctgcagctt gaactgcgtg gatgactcac     240
   aggactacta cgtgggcaag aagaacatca cgtgctgtga caccgacttg tgcaacgcca     300
   gcggggccca tgccctgcag ccggctgccg ccatccttgc gctgctccct gcactcggcc     360
   tgctgctctg gggacccggc cagctatagg ctctgggggg ccccgctgca gcccacactg     420
   ggtgtggtgc cccaggcctt tgtgccactc ctcacagaac ctggcccagt gggagcctgt     480
   cctggttcct gaggcacatc ctaacgcaag tttgaccatg tatgtttgca ccccttttcc    540
   ccnaaccctg accttcccat gggccttttc caggattccn accnggcaga tcagttttag    600
   tganacanat ccgcntgcag atggcccctc caaccntttn tgttgntgtt tccatggccc   660
   agcattttcc acccttaacc ctgtgttcag gcacttnttc ccccaggaag ccttccctgc   720
   ccaccccatt tatgaattga gccaggtttg gtccgtggtg tcccccgcac ccagcagggg   780
   acaggcaatc aggagggccc agtaaaggct gagatgaagt ggactgagta gaactggagg   840
   acaagagttg acgtgagttc ctgggagttt ccagagatgg ggcctggagg cctggaggaa   900
   ggggccaggc ctcacatttg tggggntccc gaatggcagc ctgagcacag cgtaggccct   960
   taataaacac ctgttggata agccaaaaaa                                    990

## Description

### FIELD OF THE INVENTION

The present invention provides recombinant KLK3 peptides, recombinant nucleotide molecules encoding same, recombinant *Listeria* strains comprising same, and immunogenic and therapeutic uses thereof.

### BACKGROUND OF THE INVENTION

Stimulation of an immune response is dependent upon the presence of antigens recognized as foreign by the host immune system. Bacterial antigens such as *Salmonella enterica* and *Mycobacterium bovis* BCG remain in the phagosome and stimulate CD4⁺ T-cells via antigen presentation through major histocompatibility class II molecules. In contrast, bacterial antigens such as *Listeria monocytogenes* exit the phagosome into the cytoplasm. The phagolysosomal escape of *L. monocytogenes* is a unique mechanism which facilitates major histocompatibility class I antigen presentation of listerial antigens. This escape is dependent upon the pore-forming sulfhydryl-activated cytolysin, listeriolysin O (LLO).

ActA is a surface-associated listerial protein, and acts as a scaffold in infected host cells to facilitate the polymerization, assembly and activation of host actin polymers in order to propel the *Listeria* organism through the cytoplasm. Shortly after entry into the mammalian cell cytosol, L. *monocytogenes* induces the polymerization of host actin filaments and uses the force generated by actin polymerization to move, first intracellularly and then from cell to cell. A single bacterial protein, ActA, is responsible for mediating actin nucleation and actin-based motility. The ActA protein provides multiple binding sites for host cytoskeletal components, thereby acting as a scaffold to assemble the cellular actin polymerization machinery. The NH₂ terminus of ActA binds to monomeric actin and acts as a constitutively active nucleation promoting factor by stimulating the intrinsic actin nucleation activity. ActA and hly are both members of the 10-kb gene cluster regulated by the transcriptional activator PrfA, and is up-regulated approximately 226-fold in the mammalian cytosol.

Prostate cancer is the most frequent type of cancer in American men and it is the second most important cause of cancer-related death in this population. Prostate Specific Antigen (PSA) is a marker for prostate cancer that is highly expressed by prostate tumors. There exists a long-felt need to develop compositions and methods to enhance the immunogenicity of antigens, especially antigens useful in the prevention and treatment of tumors and intracellular pathogens.

### SUMMARY OF THE INVENTION

The present invention provides a recombinant Listeria strain expressing a fusion peptide of a kallikrein-related peptidase 3 (KLK3) peptide which is operatively linked to an N-terminal non-hemolytic listeriolysin (LLO) peptide, said fusion peptide comprising the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence. In an embodiment, the recombinant *Listeria* strain is passaged through an animal host.

The recombinant *Listeria* strain may be an auxotrophic *Listeria* or a recombinant *Listeria monocytogenes* strain. The auxotrophic *Listeria* strain may be a dal/dat mutant or further comprise a deletion in the endogenous ActA gene. The auxotrophic *Listeria strain* may comprise an episomal expression vector comprising a metabolic enzyme that complements the auxotrophy of the auxotrophic *Listeria* strain, and the metabolic enzyme may be an alanine racemase enzyme or a D-amino acid transferase enzyme.

The present invention also provides a recombinant polypeptide comprising a fusion peptide of a KLK3 peptide which is operatively linked to an N-terminal LLO peptide, wherein said recombinant polypeptide comprises the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence. The invention also provides a nucleotide molecule encoding the recombinant polypeptide, and a recombinant vaccine vector encoding the recombinant polypeptide or comprising the nucleotide molecule encoding the recombinant polypeptide.

The present invention also provides a vaccine comprising the recombinant *Listeria* strain, the recombinant polypeptide or the nucleotide molecule encoding the recombinant polypeptide, and an adjuvant.

The invention provides the recombinant *Listeria* strain, or an immunogenic composition comprising the recombinant polypeptide, or the recombinant nucleotide, for use as a medicament.

The invention further provides the recombinant *Listeria* strain, or an immunogenic composition comprising the recombinant polypeptide or the recombinant nucleotide, for use in inducing an anti-KLK3 immune response in a subject.

The invention also provides the recombinant *Listeria* strain, the recombinant polypeptide, or the recombinant nucleotide, for use in treating, inhibiting or suppressing a kallikrein-related peptidase 3 (KLK3) protein-expressing prostate cancer in a subject, whereby the subject mounts an immune response against the KLK3 protein-expressing prostate cancer.

The recombinant polypeptide may be made by a process comprising the step of chemically conjugating a polypeptide comprising the KLK3 peptide to a polypeptide comprising the N-terminal LLO peptide.

### BRIEF DESCRIPTION OF THE DRAWINGS

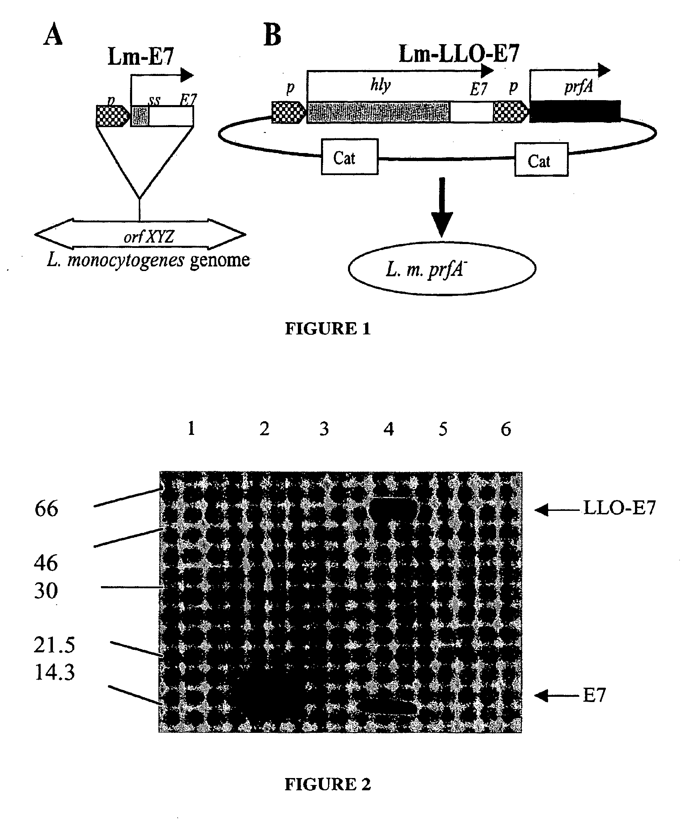

Figure 1. Lm-E7 and Lm-LLO-E7 use different expression systems to express and secrete E7. Lm-E7 was generated by introducing a gene cassette into the orfZ domain of the *L. monocytogenes* genome (A). The hly promoter drives expression of the hly signal sequence and the first five amino acids (AA) of LLO followed by HPV-16 E7. B), Lm-LLO-E7 was generated by transforming the prfA⁻ strain XFL-7 with the plasmid pGG-55. pGG-55 has the hly promoter driving expression of a non-hemolytic fusion of LLO-E7. pGG-55 also contains the prfA gene to select for retention of the plasmid by XFL-7 *in vivo.*
Figure 2. Lm-E7 and Lm-LLO-E7 secrete E7. Lm-Gag (lane 1), Lm-E7 (lane 2), Lm-LLO-NP (lane 3), Lm-LLO-E7 (lane 4), XFL-7 (lane 5), and 10403S (lane 6) were grown overnight at 37°C in Luria-Bertoni broth. Equivalent numbers of bacteria, as determined by OD at 600 nm absorbance, were pelleted and 18 ml of each supernatant was TCA-precipitated. E7 expression was analyzed by Western blot. The blot was probed with an anti-E7 mAb, followed by HRP-conjugated anti-mouse (Amersham), then developed using ECL detection reagents.

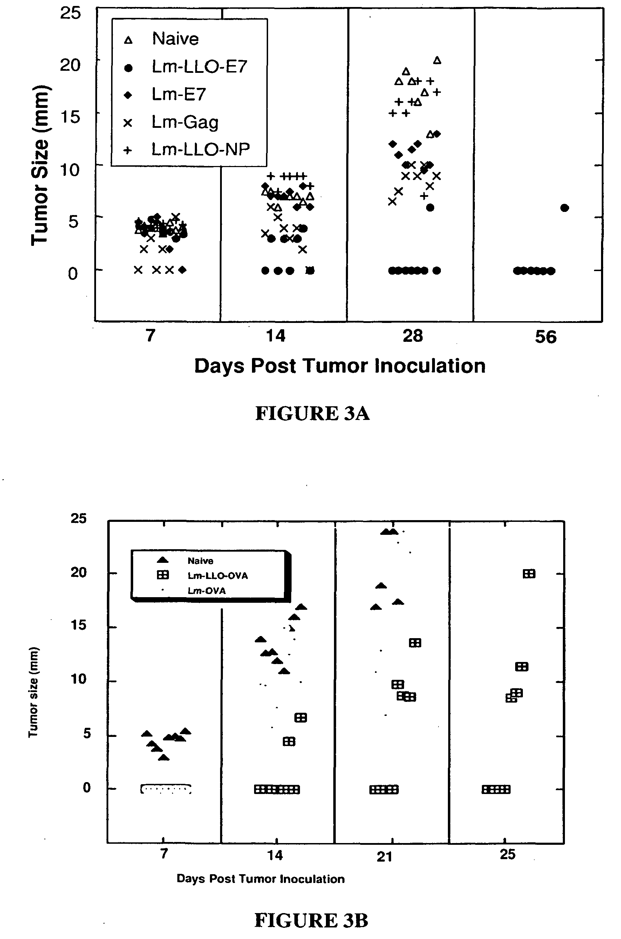

Figure 3. A. Tumor immunotherapeutic efficacy of LLO-E7 fusions. Tumor size in millimeters in mice is shown at 7, 14, 21, 28 and 56 days post-tumor inoculation. Naive mice: open triangles; Lm-LLO-E7: filled circles; Lm-E7: filled diamonds; Lm-Gag: X's; and Lm-LLO-NP: + signs. B. Tumor immunotherapeutic efficacy of LLO-Ova fusions.

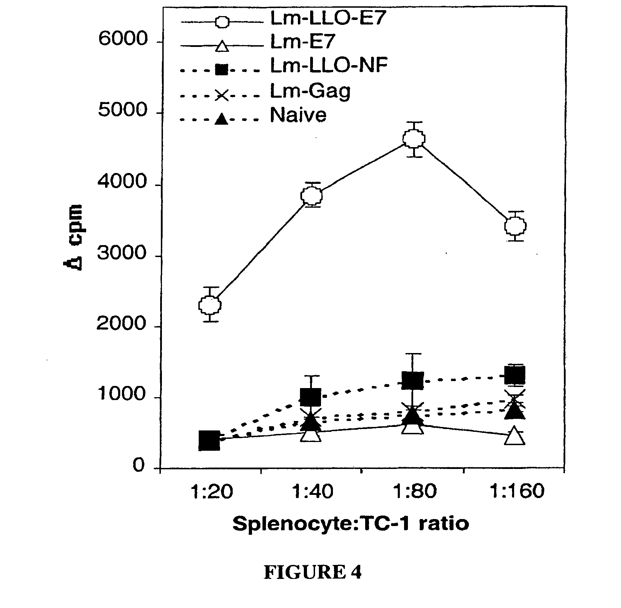

Figure 4. Splenocytes from Lm-LLO-E7-immunized mice proliferate when exposed to TC-1 cells. C57BL/6 mice were immunized and boosted with Lm-LLO-E7, Lm-E7, or control rLm strains. Splenocytes were harvested 6 days after the boost and plated with irradiated TC-1 cells at the ratios shown. The cells were pulsed with ³H thymidine and harvested. Cpm is defined as (experimental cpm) - (no-TC-1 control).

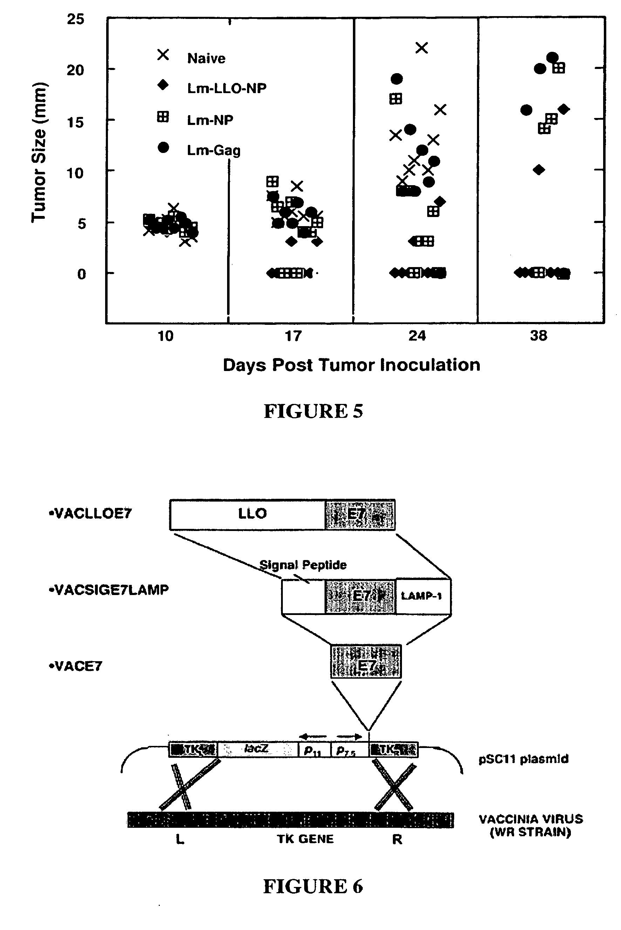

Figure 5. Tumor immunotherapeutic efficacy of NP antigen expressed in LM. Tumor size in millimeters in mice is shown at 10, 17, 24, and 38 days post-tumor inoculation. Naive mice: X's; mice administered Lm-LLO-NP: filled diamonds; Lm-NP: squares; Lm-Gag: filled circles.
Figure 6. Depiction of vaccinia virus constructs expressing different forms of HPV16 E7 protein.

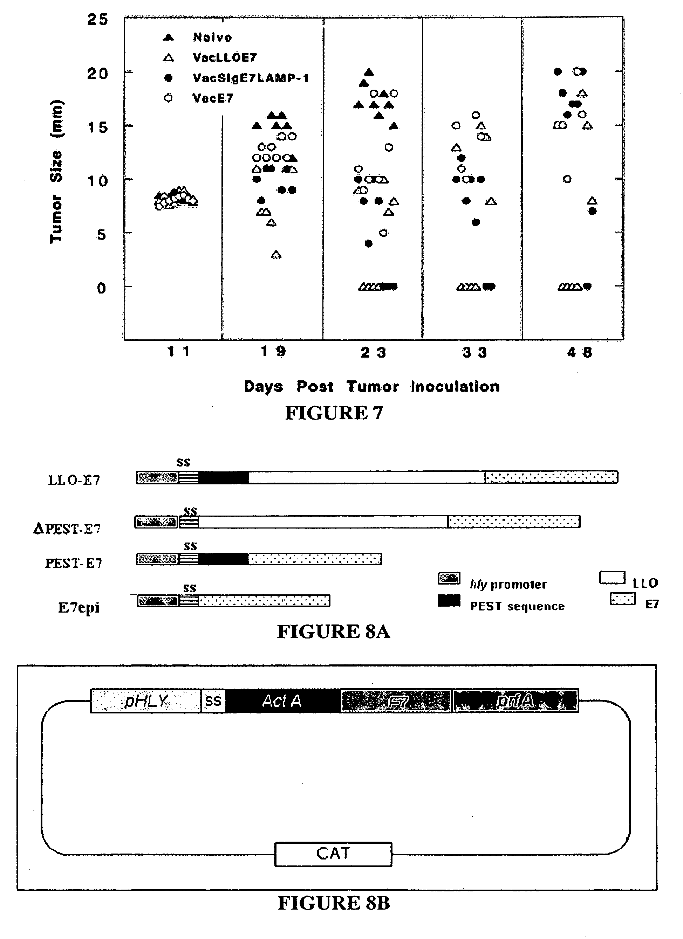

Figure 7. VacLLOE7 causes long-term regression of tumors established from 2 × 10⁵ TC-1 cells injected s.c. into C57BL/6 mice. Mice were injected 11 and 18 days after tumor challenge with 10⁷ PFU of VacLLOE7, VacSigE7LAMP-1, or VacE7/mouse i.p. or were left untreated (naive). Eight mice per treatment group were used, and the cross section for each tumor (average of two measurements) is shown for the indicated days after tumor inoculation.

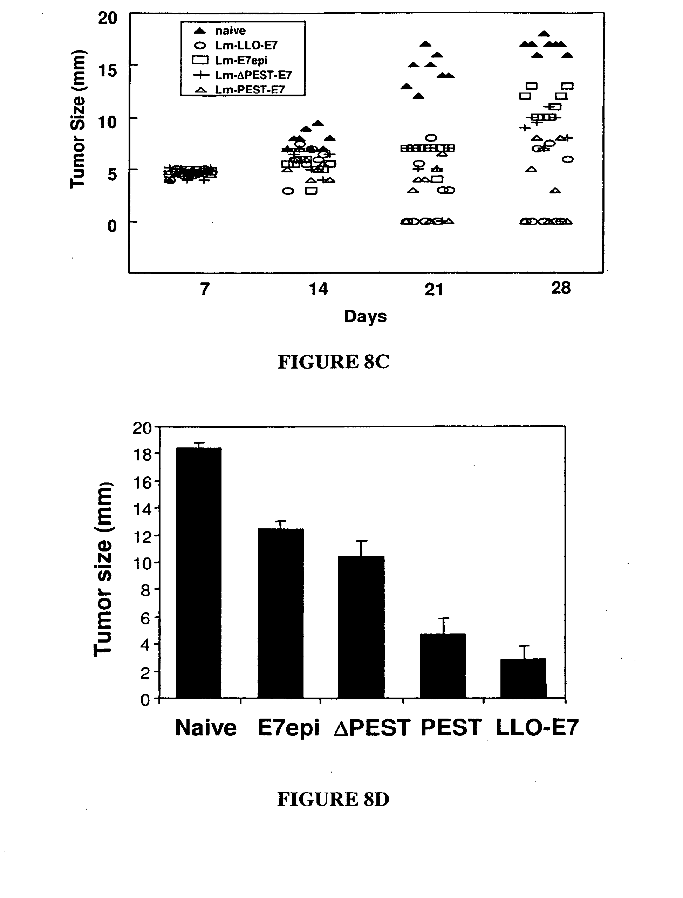

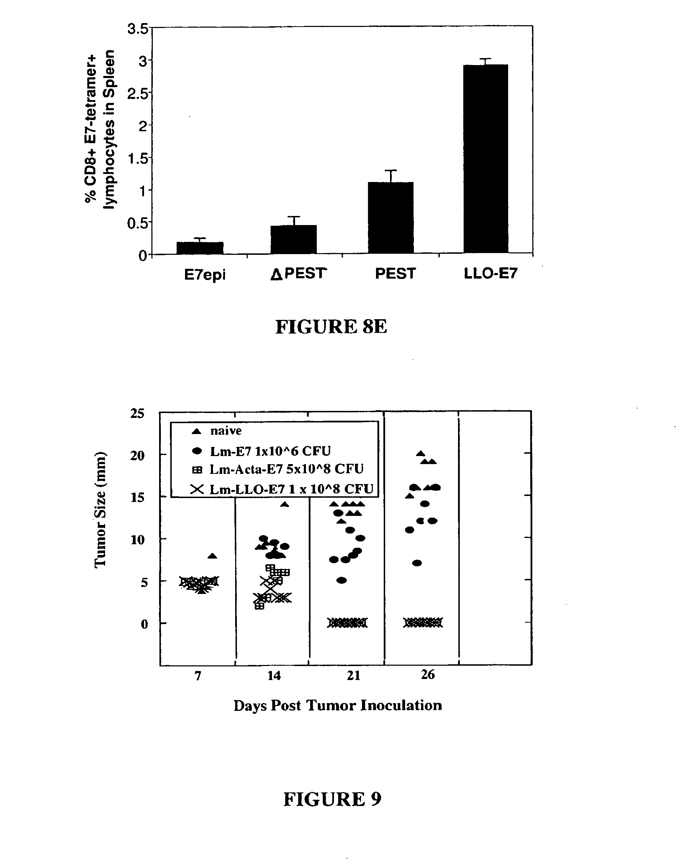

Figure 8. A. schematic representation of the plasmid inserts used to create 4 LM vaccines. Lm-LLO-E7 insert contains all of the *Listeria* genes used. It contains the *hly* promoter, the first 1.3 kb of the *hly* gene (which encodes the protein LLO), and the HPV-16 E7 gene. The first 1.3 kb of *hly* includes the signal sequence (ss) and the PEST region. Lm-PEST-E7 includes the *hly* promoter, the signal sequence, and PEST and E7 sequences, but excludes the remainder of the truncated LLO gene. Lm-ΔPEST-E7 excludes the PEST region, but contains the *hly* promoter, the signal sequence, E7, and the remainder of the truncated LLO. Lm-E7epi has only the *hly* promoter, the signal sequence, and E7. B. Schematic representation of the pActA-E7 expression system used to express and secrete E7 from recombinant *Listeria* bacteria. The *hly* promoter (pHLY) drives expression, the prfA gene is used to select retention of the plasmid by recombinant *Listeria in vivo.* C. Top panel: *Listeria* constructs containing PEST regions induce tumor regression. Filled triangles: naive mice; circles: Lm-LLO-E7; squares: Lm-E7epi; + signs: Lm-ΔPEST-E7; open triangles: Lm-PEST-E7. D. Average tumor sizes at day 28 post-tumor challenge in two separate experiments. E. *Listeria* constructs containing PEST regions induce a higher percentage of E7-specific lymphocytes in the spleen. Average and SE of data from three experiments are depicted.
Figure 9. Tumor size in mice administered Lm-ActA-E7 (open squares), Lm-E7 (filled circles), Lm-LLO-E7 (X), and naive mice (non-vaccinated; filled triangles).

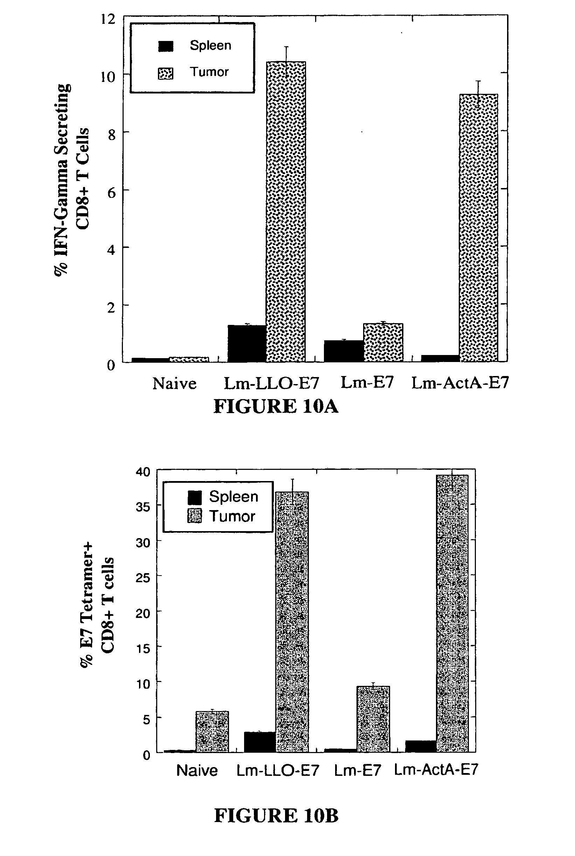

Figure 10. A. Induction of E7-specific IFN-gamma-secreting CD8⁺ T cells in the spleens and the numbers penetrating the tumors, in mice administered TC-1 tumor cells and subsequently administered Lm-E7, Lm-LLO-E7, Lm-ActA-E7, or no vaccine (naive). B. Induction and penetration of E7-specific CD8⁺ cells in the spleens and tumors of the mice described for (A).

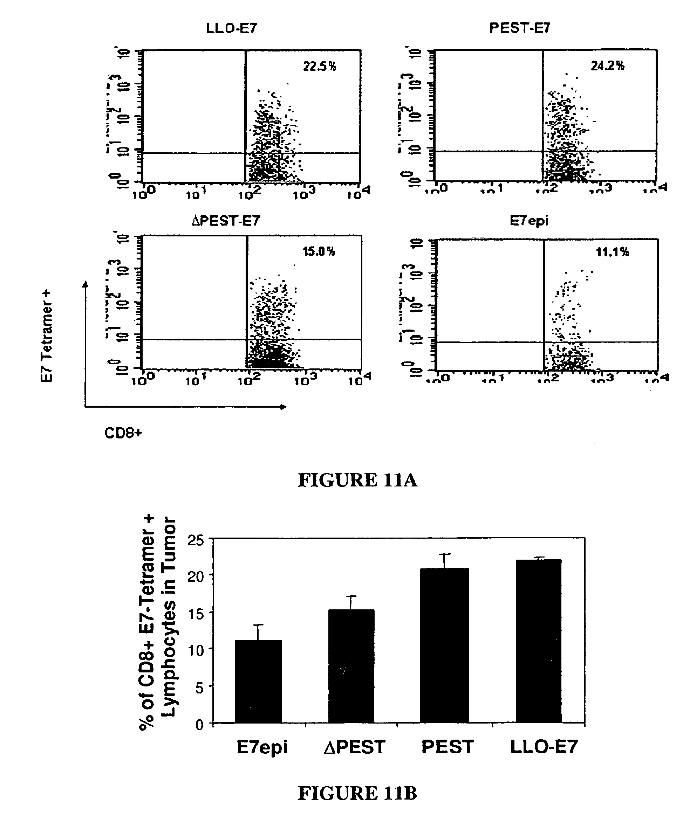

Figure 11. *Listeria* constructs containing PEST regions induce a higher percentage of E7-specific lymphocytes within the tumor. A. representative data from one experiment. B. average and SE of data from all three experiments.

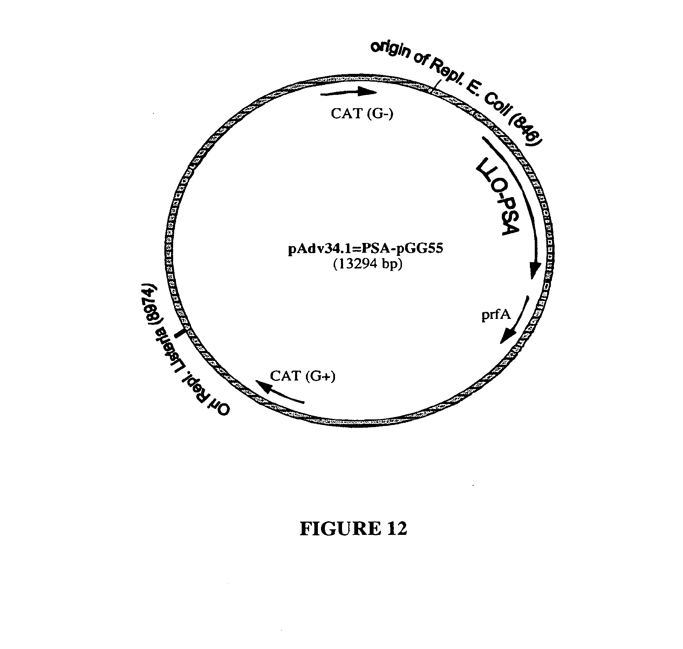

Figure 12. **Schematic map of pAdv34 in pGG55.** CAT(G-), chloramphenicol acetyltransferase for *E. coli;* CAT(G+), chloramphenicol acetyltransferase for *Lm;* Ori, replication region; *prfA, Lm* pathogenicity-regulating factor A; LLO-PSA, fusion between the gene encoding a C-terminally truncated listeriolysin O, including its promoter, and the gene encoding human PSA.

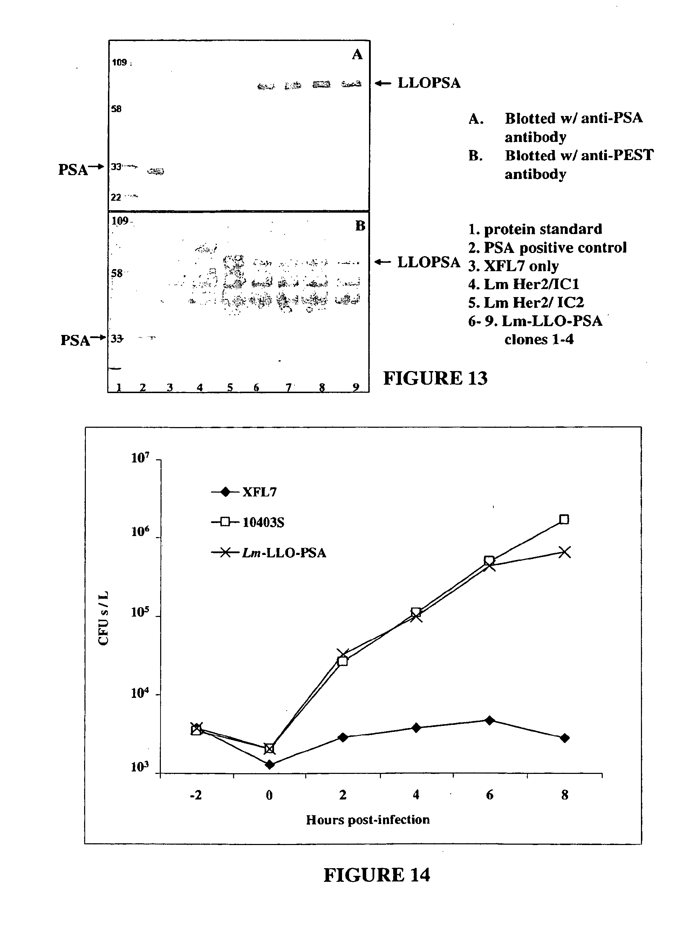

Figure 13. **Expression and secretion of LLO-PSA fusion protein by *Lm*-LLO-PSA.** Bacteria were grown overnight and cell supernatants were precipitated in the presence of 10% TCA at 4°C. Proteins were separated by SDS-PAGE and blotted with anti-PSA (A) and anti-LLO (B) antibodies. PSA positive control was a BscI cell lysate infected with PSA/vaccinia. The parent bacterial strain XFL7 and two irrelevant *Lm* strains expressing fragments of human Her2/neu antigen were used as negative controls.
Figure 14. **Uptake and growth of *Lm*-LLO-PSA in macrophages.** Murine macrophages like cells (J774A.1) were infected *in vitro* with MOI of 1:1 with *Lm*-LLO-PSA, the parent *Lm* XFL7 or the wild type *Lm* 10403s strains. The intracellular growth was monitored for 8 hours by taking duplicate samples every other hour from the cells followed by titration of the bacteria on BHI plates. CFUs: Colony Forming Units.

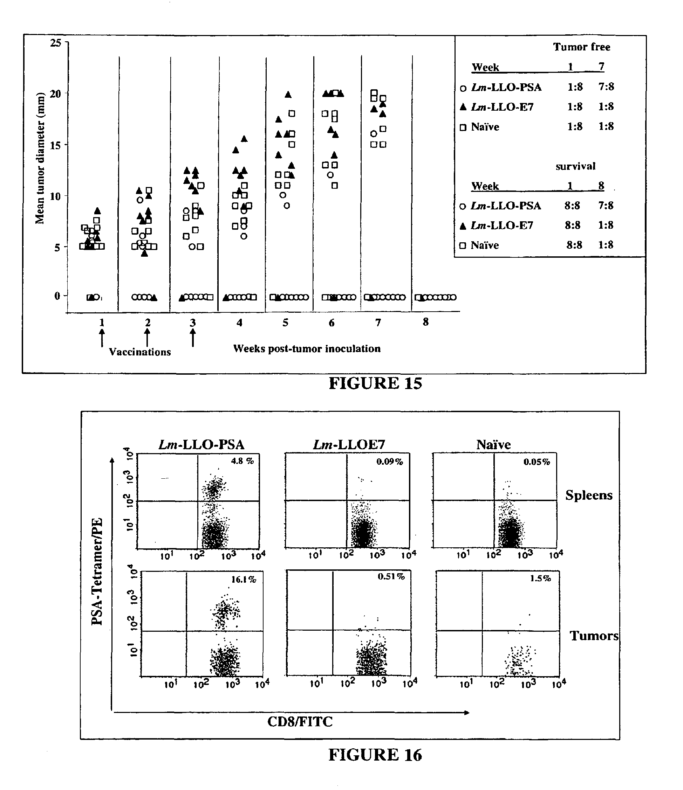

Figure 15. **Tumor regression upon *Lm*-LLO-PSA immunization.** Groups of 8 mice were inoculated s.c. with 5 x 10⁶ T-PSA23 cells on day 0 and immunized three times with one week intervals, i.p. with *Lm*-LLO-PSA, Lm-LLO-E7 or received no immunization (naive). Tumors were monitored weekly using an electronic caliper and are expressed as the mean of two perpendicular diameters. Mice were sacrificed when the tumors reached a size of 15-18 mm in diameter. Results are depicted as tumor size from each individual mouse. The table shows the number of mice in each group which were either tumor free (upper panel) or survived (lower panel) relative to the total number of mice per group 1 week or 7-8 weeks post-tumor inoculation. This experiment was repeated three time showing similar results.
Figure 16. **PSA specific CD8+ T cells in spleens and tumors.** T-PSA23 cells were implanted s.c. as a mixture with matrigel. Mice were immunized twice with *Lm*-LLO-PSA or *Lm*-LLO-E7 or were left naïve. Spleens and tumors were extracted 6 days after the second immunization and tested for the presence of CD8⁺/PSA tetramer positive T cells by FACS analysis. *Lm*-LLO-PSA immunizations resulted in the generation of PSA specific CD8⁺ T cells in both spleens (upper panel) and tumors (lower panel). Numbers in each corner indicated the % of PSA positive cells over total number of CD8⁺ T cells in each tissue.

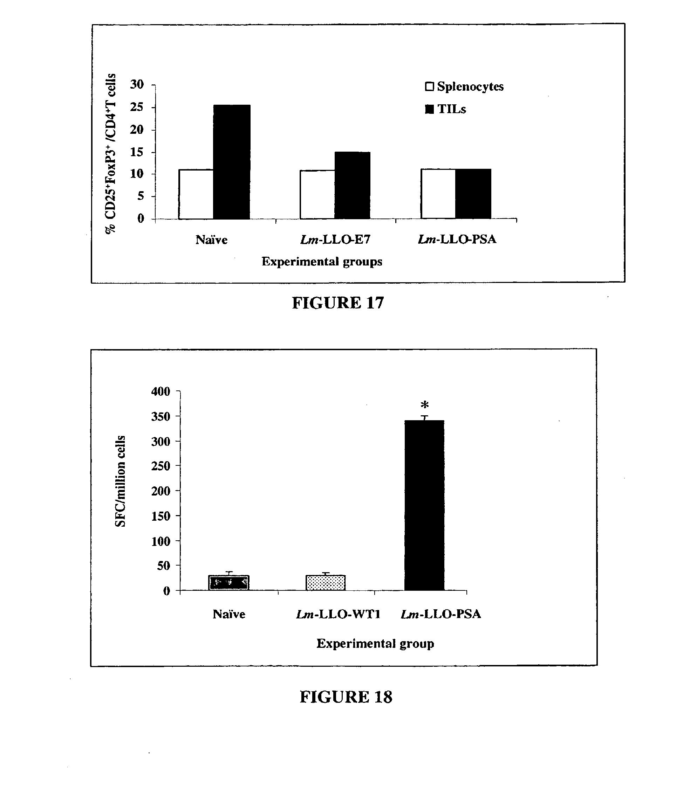

Figure 17. **Effect of vaccination with *Lm* vaccines on the presence of Tregs in spleens and tumors.** Isolated splenocytes and TILs extracted from 3 tumor bearing mice in the previous experiment were pooled and stained for CD4, CD25 and FoxP3 to elucidate the effect of immunization with *Lm*-LLO-PSA on the presence of Tregs in these tissues. Each column presents the % of CD25⁺/FoxP3⁺ T cell population in relation to the total CD4⁺ T cells in each pool.
Figure 18. **IFN-γ secretion stimulated by *Lm*-LLO-PSA vaccination detected by ELISpot assay.** Mice were immunized three times with 0.1 LD₅₀ of *Lm-*LLO-PSA. Isolated splenocytes were prepared and incubated overnight in the presence of a 1 µM PSA specific H2D^{b} peptide, HCIRNKSVIL. IFN-γ secretion by isolated cells was detected using the ELISpot kit from Mabtech and expressed as spot forming cells (SFC)/million cells. Splenocytes from *Lm*-LLO-WT1 immunized or naive mice were used as negative controls. (* indicates statistical significance with the naive groups with p<0.0001).

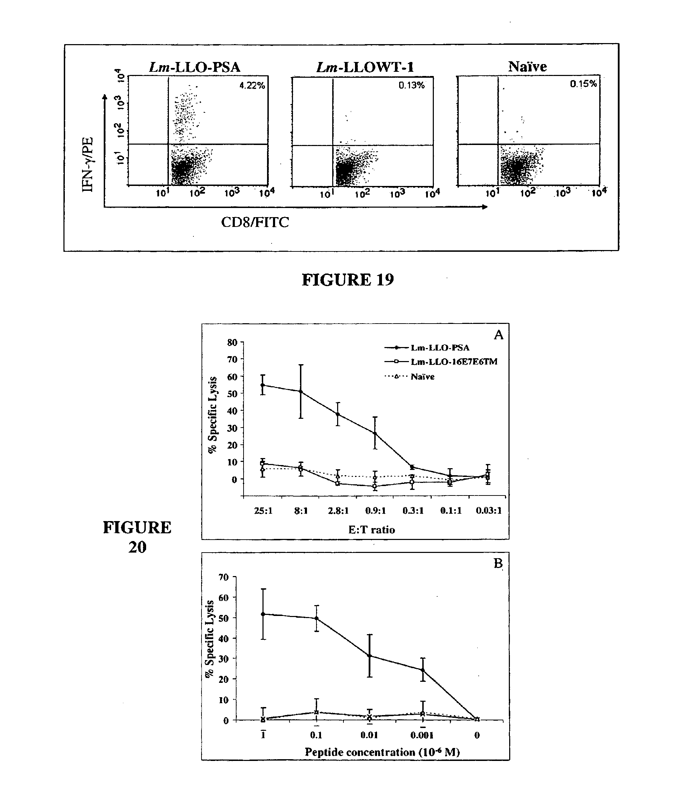

Figure 19. **IFN-γ production stimulated by *Lm*-LLO-PSA vaccination detected by intracellular staining.** Mice were immunized three times with 0.1 LD₅₀ of *Lm*-LLO-PSA. Isolated splenocytes were prepared and incubated for 5 hours in the presence of a PSA specific H2D^{b} peptide. IFN-γ production by activated CD8⁺ T cells was determined as described in methods. Splenocytes from *Lm-*LLO-WT1 immunized or naive mice were used as negative controls.
Figure 20. **PSA-specific cytotoxic responses in mice.** Animals were immunized three times with 0.1 LD₅₀ of Lm-LLO-PSA, *Lm*-LLO-HPV16E7E6 (as *Lm* negative control) or were left naïve. Isolated splenocytes were prepared and incubated with PSA expressing stimulator cells for 5 days. CTL assay was performed for 4 hours by incubating effector cells (E) with target EL4 cells pulsed with PSA H2D^{b} peptide: A) at different E:T ratio/fixed peptide concentration (1µM); or B) at a fixed E:T ratio of 1:25 but different peptide concentrations.

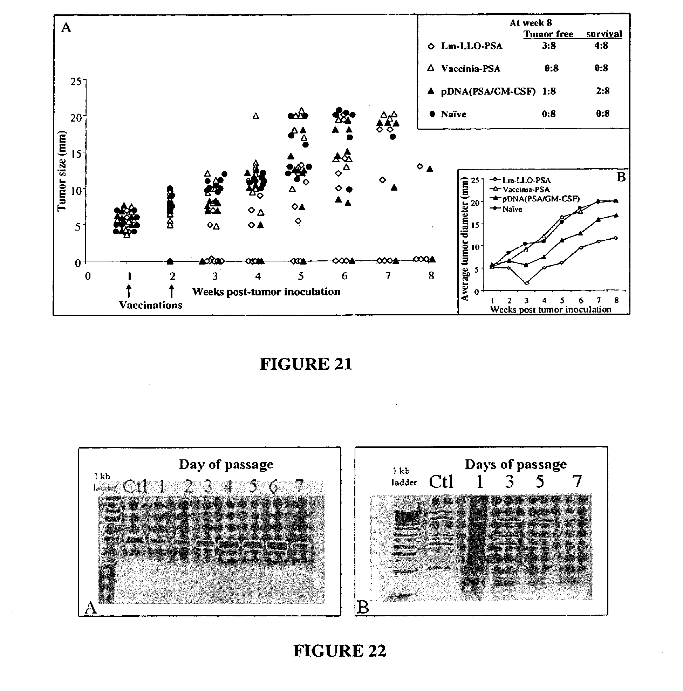

Figure 21. **Comparison of anti-tumor effects of Lm-LLO-PSA with other PSA based vaccines.** Groups of 8 mice were inoculated s.c. with 5 x 10⁶ T-PSA23 cells on day 0 and immunized two times with one week intervals, with *Lm*-LLO-PSA, vaccinia-PSA or pDNA (PSA+GM-CSF) or received no immunization (naïve). Tumors were monitored weekly using an electronic caliper and are expressed as the mean of two perpendicular diameters. Mice were sacrificed when the tumors reached a size of 20 mm in diameter. Results are depicted as tumor size from each individual mouse (A). The table shows the number of mice in each group which were either tumor free or survived for 8 weeks post-tumor inoculation. B) Average of tumor sizes in each group. When tumors reached a size of 20 mm, mice were sacrificed. They were given a value of 20 mm for making the average curve. This experiment was repeated two times showing similar results.
Figure 22. Stability of Lm-PSA. Lm-PSA was grown and passaged for 7 consecutive days *in vitro.* Plasmid DNA was purified from bacterial samples taken every day during the *in vitro* growth and tested by amplification of PSA gene by PCR (A) or EcoRI/HindIII restriction mapping of the plasmid (B).

### DETAILED DESCRIPTION OF THE INVENTION

The present invention provides KLK3-LLO fusion peptides, recombinant polypeptides comprising same, recombinant nucleotide molecules encoding same, recombinant *Listeria* strains comprising same, and immunogenic and therapeutic methods utilizing same.

Specifically, the present invention provides a recombinant Listeria strain expressing a fusion peptide of a kallikrein-related peptidase 3 (KLK3) peptide which is operatively linked to an N-terminal non-hemolytic listeriolysin (LLO) peptide, said fusion peptide comprising the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence. In an embodiment, the recombinant *Listeria* strain may have been passaged through an animal host.

The recombinant *Listeria* strain may be an auxotrophic *Listeria* or a recombinant *Listeria monocytogenes* strain. The auxotrophic *Listeria* strain may be a dal/dat mutant or further comprise a deletion in the endogenous ActA gene. The auxotrophic *Listeria strain* may comprise an episomal expression vector comprising a metabolic enzyme that complements the auxotrophy of the auxotrophic *Listeria* strain, and the metabolic enzyme may be an alanine racemase enzyme or a D-amino acid transferase enzyme.

The present invention also provides a recombinant polypeptide comprising a fusion peptide of a KLK3 peptide which is operatively linked to an N-terminal LLO peptide, wherein said recombinant polypeptide comprises the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence. The invention also provides a nucleotide molecule encoding the recombinant polypeptide, and a recombinant vaccine vector encoding the recombinant polypeptide or comprising the nucleotide molecule encoding the recombinant polypeptide.

The present invention also provides a vaccine comprising the recombinant *Listeria* strain, the recombinant polypeptide or the nucleotide molecule encoding the recombinant polypeptide, and an adjuvant.

The invention provides the recombinant *Listeria* strain, or an immunogenic composition comprising the recombinant polypeptide, or the recombinant nucleotide, for use as a medicament.

The invention further provides the recombinant *Listeria* strain, or an immunogenic composition comprising the recombinant polypeptide or the recombinant nucleotide, for use in inducing an anti-KLK3 immune response in a subject.

The invention also provides the recombinant *Listeria* strain, the recombinant polypeptide, or the recombinant nucleotide, for use in treating, inhibiting or suppressing a kallikrein-related peptidase 3 (KLK3) protein-expressing prostate cancer in a subject, whereby the subject mounts an immune response against the KLK3 protein-expressing prostate cancer.

The recombinant polypeptide may be made by a process comprising the step of chemically conjugating a polypeptide comprising the KLK3 peptide to a polypeptide comprising the N-terminal LLO peptide.

In an embodiment, the present invention provides a recombinant *Listeria* strain expressing a kallikrein-related peptidase 3 (KLK3)-LLO fusion peptide, said fusion peptide comprising the sequence of SEQ ID NO: 54 or a sequence at least 99% homologous thereto. In another embodiment, the sequence of the KLK3 peptide in the fusion protein is selected from SEQ ID NOs: 25, 32, 34, and 39. In another embodiment, the sequence of the KLK3 peptide comprises a sequence selected from SEQ ID No: 25, 32, 34, and 39. In another embodiment, the KLK3 peptide is an immunogenic fragment of a larger KLK3 peptide, wherein the sequence of the larger KLK3 peptide is a sequence selected from SEQ ID No: 25, 32, 34, and 39. In another embodiment, the sequence of the KLK3 peptide comprises an immunogenic fragment of a sequence selected from SEQ ID No: 25, 32, 34, and 39.

The "KLK3 peptide" is lacking the KLK3 signal peptide. In another embodiment, the term refers to a KLK3 protein that contains the entire KLK3 sequence except the KLK3 signal peptide. "KLK3 signal sequence" refers, in another embodiment, to any signal sequence found in nature on a KLK3 protein. In another embodiment, a KLK3 protein of methods and compositions of the present invention does not contain any signal sequence.

In one embodiment, "variant" refers to an amino acid or nucleic acid sequence (or in other embodiments, an organism or tissue) that is different from the majority of the population but is still sufficiently similar to the common mode to be considered to be one of them, for example splice variants.

In one embodiment, "isoform" refers to a version of a molecule, for example, a protein, with only slight differences compared to another isoform, or version, of the same protein. In one embodiment, isoforms may be produced from different but related genes, or in another embodiment, may arise from the same gene by alternative splicing. In another embodiment, isoforms are caused by single nucleotide polymorphisms.

In one embodiment, "fragment" refers to a protein or polypeptide that is shorter or comprises fewer amino acids than the full length protein or polypeptide. In another embodiment, fragment refers to a nucleic acid that is shorter or comprises fewer nucleotides than the full length nucleic acid. In another embodiment, the fragment is an N-terminal fragment. In another embodiment, the fragment is a C-terminal fragment. In one embodiment, the fragment is an intrasequential section of the protein, peptide, or nucleic acid. In one embodiment, the fragment is a functional fragment. In another embodiment, the fragment is an immunogenic fragment. In one embodiment, a fragment has 10-250 nucleic or amino acids.

In one embodiment, a "homologue" refers to a nucleic acid or amino acid sequence which shares a certain percentage of sequence identity with a particular nucleic acid or amino acid sequence. In another embodiment, a sequence useful in the composition and methods as provided herein may be a homologue of any sequence described herein. In an embodiment, a homologue shares at least 99% identity with a particular sequence. In one embodiment, it is to be understood that a homologue of any of the sequences as provided herein and/or as described herein is considered to be a part of the invention.

In one embodiment, "heterologous" as used herein describes a nucleic acid, amino acid, peptide, polypeptide, protein, etc derived from a different species than the reference species. Thus, for example, a *Listeria* strain expressing a heterologous polypeptide, in one embodiment, would express a polypeptide that is not native or endogenous to the *Listeria* strain, or in another embodiment, a polylpeptide that is not normally expressed by the *Listeria* strain, or in another embodiment, a polypeptide from a source other than the *Listeria* strain.

In one embodiment, "endogenous" as used herein describes a nucleic acid, amino acid, peptide, polypeptide, protein, etc that has developed or originated within the reference organism or arisen from causes within the reference organism. In another embodiment, endogenous refers to native.

In another embodiment, the kallikrein-related peptidase 3 (KLK3 protein) that is the source of a KLK3 peptide of methods and compositions of the present invention is a PSA protein.

In another embodiment, the KLK3 protein is a mature KLK3 protein. In another embodiment, the KLK3 protein is a pro-KLK3 protein. In another embodiment, the leader sequence has been removed from a mature KLK3 protein of methods and compositions of the present invention. An example of a mature KLK3 protein is encoded by aa378-1088 of SEQ ID No: 40.

In another embodiment, the KLK3 protein that is the source of a KLK3 peptide of methods and compositions of the present invention is a human KLK3 protein.

In another embodiment, the KLK3 protein has the sequence of SEQ ID No: 25 (GenBank Accession No. X14810). In another embodiment, the KLK3 protein is a fragment of SEQ ID No: 25.

In another embodiment, the KLK3 protein is encoded by a nucleotide molecule having the sequence of SEQ ID No: 26 (GenBank Accession No. X14810). In another embodiment, the KLK3 protein is encoded by residues 401-446, 1688-1847, 3477-3763, 3907-4043, 5413-5568, or a combination thereof, of SEQ ID No: 26. In another embodiment, the KLK3 protein is encoded by a homologue of SEQ ID No: 26. In another embodiment, the KLK3 protein is encoded by a variant of SEQ ID No: 26. In another embodiment, the KLK3 protein is encoded by an isoform of SEQ ID No: 26. In another embodiment, the KLK3 protein is encoded by a fragment of SEQ ID No: 26.

In another embodiment, the KLK3 protein has the sequence of SEQ ID No: 32 (GenBank Accession No. NM_001648). In another embodiment, the KLK3 protein is a fragment of SEQ ID No: 32.

In another embodiment, the KLK3 protein is encoded by a nucleotide molecule having the sequence of SEQ ID No: 33 (GenBank Accession No. NM_001648). In another embodiment, the KLK3 protein is encoded by residues 42-827 of SEQ ID No: 33. In another embodiment, the KLK3 protein is encoded by a homologue of SEQ ID No: 33. In another embodiment, the KLK3 protein is encoded by a variant of SEQ ID No: 33. In another embodiment, the KLK3 protein is encoded by an isoform of SEQ ID No: 33. In another embodiment, the KLK3 protein is encoded by a fragment of SEQ ID No: 33.

In another embodiment, the KLK3 protein has the sequence of SEQ ID No: 34 (GenBank Accession No. BC056665). In another embodiment, the KLK3 protein is a fragment of SEQ ID No: 34.

In another embodiment, the KLK3 protein is encoded by a nucleotide molecule having the sequence of SEQ ID No: 35 (GenBank Accession No. BC056665). In another embodiment, the KLK3 protein is encoded by residues 47-832 of SEQ ID No: 35. In another embodiment, the KLK3 protein is encoded by a homologue of SEQ ID No: 35. In another embodiment, the KLK3 protein is encoded by a variant of SEQ ID No: 35. In another embodiment, the KLK3 protein is encoded by an isoform of SEQ ID No: 35. In another embodiment, the KLK3 protein is encoded by a fragment of SEQ ID No: 35.

In another embodiment, the KLK3 protein has the sequence of SEQ ID No: 39 (GenBank Accession No. AJ459783). In another embodiment, the KLK3 protein is a fragment of SEQ ID No: 39.

In another embodiment, the KLK3 protein is encoded by a nucleotide molecule having the sequence of SEQ ID No: 40 (GenBank Accession No. X07730). In another embodiment, the KLK3 protein is encoded by residues 67-1088 of SEQ ID No: 40. In another embodiment, the KLK3 protein is encoded by a homologue of SEQ ID No: 40. In another embodiment, the KLK3 protein is encoded by a variant of SEQ ID No: 40. In another embodiment, the KLK3 protein is encoded by an isoform of SEQ ID No: 40. In another embodiment, the KLK3 protein is encoded by a fragment of SEQ ID No: 40.

In another embodiment, the KLK3 protein has a sequence set forth in one of the following GenBank Accession Numbers: BC005307, AJ310938, AJ310937, AF335478, AF335477, M27274, and M26663. In another embodiment, the KLK3 protein is encoded by a sequence set forth in one of the above GenBank Accession Numbers.

In another embodiment, the KLK3 protein is encoded by a sequence set forth in one of the following GenBank Accession Numbers: NM_001030050, NM_001030049, NM_001030048, AJ459782, AJ512346, or AJ459784.

In another embodiment, the KLK3 protein has the sequence that comprises a sequence set forth in one of the following GenBank Accession Numbers: X13943, X13942, X13940, X13941, and X13944.

In another embodiment, the present invention provides a vaccine comprising a recombinant *Listeria* strain of the present invention and an adjuvant.

In another embodiment, the present invention provides an immunogenic composition comprising a recombinant *Listeria* strain of the present invention.

In another embodiment, the recombinant *Listeria* strain expresses a recombinant polypeptide that comprises a KLK3:LLO fusion peptide. In another embodiment, the recombinant *Listeria* strain comprises a recombinant polypeptide, wherein the recombinant peptide comprises a KLK3:LLO fusion peptide. In another embodiment, the recombinant *Listeria* strain comprises a recombinant nucleotide encoding the recombinant polypeptide.

The KLK3:LLO fusion peptide expressed by the recombinant *Listeria* enhances the immunogenicity of the KLK3 peptide.

The non-KLK3/ non-FOLH1 peptide in the fusion peptide of the present invention is an LLO peptide, which in another embodiment, may be a non-hemolytic LLO peptide

In another embodiment, the present invention provides a recombinant *Listeria* strain comprising a recombinant polypeptide of the present invention. In another embodiment, the present invention provides a recombinant *Listeria* strain comprising a recombinant nucleotide encoding a recombinant polypeptide of the present invention. In another embodiment, the *Listeria* vaccine strain is a strain of the species *Listeria monocytogenes* (LM). In another embodiment, the present invention provides a composition comprising the *Listeria* strain. In another embodiment, the present invention provides an immunogenic composition comprising the *Listeria* strain..

In another embodiment, the *Listeria* strain is a recombinant *Listeria seeligeri* strain. In another embodiment, the *Listeria* strain is a recombinant *Listeria grayi* strain. In another embodiment, the *Listeria* strain is a recombinant *Listeria ivanovii* strain. In another embodiment, the *Listeria* strain is a recombinant *Listeria murrayi* strain. In another embodiment, the *Listeria* strain is a recombinant *Listeria welshimeri* strain. In another embodiment, the *Listeria* strain is a recombinant strain of any other *Listeria* species known in the art.

In another embodiment, a recombinant *Listeria* strain of the present invention has been passaged through an animal host. The passaging may maximize efficacy of the strain as a vaccine vector, stabilize the immunogenicity of the *Listeria* strain, stabilize the virulence of the *Listeria* strain, increase the immunogenicity of the *Listeria* strain, increase the virulence of the *Listeria* strain, remove unstable sub-strains of the *Listeria* strain, and/or reduce the prevalence of unstable sub-strains of the *Listeria* strain. In another embodiment, the *Listeria* strain contains a genomic insertion of the gene encoding the KLK3 peptide. In another embodiment, the *Listeria* strain carries a plasmid comprising the gene encoding the KLK3:LLO recombinant fusion peptide. Methods for passaging a recombinant *Listeria* strain through an animal host are well known in the art, and are described, for example, in United States Patent Application Publication No. 2006/0233835. In another embodiment, the passaging is performed by any other method known in the art.

In another embodiment, the recombinant *Listeria* strain utilized in methods of the present invention has been stored in a frozen cell bank or in a lyophilized cell bank. The cell bank may be a master cell bank, a working cell bank, and/or a Good Manufacturing Practice (GMP) cell bank. The cell bank may be intended for production of clinical-grade material, and/or conforms to regulatory practices for human use.

"Good Manufacturing Practices" are defined, in another embodiment, by (21 CFR 210-211) of the United States Code of Federal Regulations. In another embodiment, "Good Manufacturing Practices" are defined by other standards for production of clinical-grade material or for human consumption; e.g. standards of a country other than the United States.

In another embodiment, a recombinant *Listeria* strain utilized in the present invention is from a batch of vaccine doses, or it is from a frozen stock or a lyophilized stock produced by a method disclosed herein.

In another embodiment, a cell bank, frozen stock, or batch of vaccine doses of the present invention exhibits viability upon thawing of greater than 90%. In another embodiment, the thawing follows storage for cryopreservation or frozen storage for 24 hours. In another embodiment, the storage is for 2 days, 3 days, 4 days, 1 week, 2 weeks, 3 weeks, 1 month, 2 months, 3 months, 5 months, 6 months, for 9 months, or the storage is for 1 year.

In another embodiment, a cell bank, frozen stock, or batch of vaccine doses of the present invention is cryopreserved by a method that comprises growing a culture of the *Listeria* strain in a nutrient media, freezing the culture in a solution comprising glycerol, and storing the *Listeria* strain at below -20 degrees Celsius. In another embodiment, the temperature is about -70 degrees Celsius. In another embodiment, the temperature is about -70 - ⁻80 degrees Celsius.

In another embodiment, a cell bank, frozen stock, or batch of vaccine doses of the present invention is cryopreserved by a method that comprises growing a culture of the *Listeria* strain in a defined media (as described below), freezing the culture in a solution comprising glycerol, and storing the *Listeria* strain at below -20 degrees Celsius. In another embodiment, the temperature is about -70 degrees Celsius. In another embodiment, the temperature is about -70 - ⁻80 degrees Celsius. In another embodiment, any defined microbiological media may be used in this method.

In another embodiment, the culture (e.g. the culture of a *Listeria* vaccine strain that is used to produce a batch of *Listeria* vaccine doses) is inoculated from a cell bank. In another embodiment, the culture is inoculated from a frozen stock. In another embodiment, the culture is inoculated from a starter culture. In another embodiment, the culture is inoculated from a colony. In another embodiment, the culture is inoculated at mid-log growth phase. In another embodiment, the culture is inoculated at approximately mid-log growth phase. In another embodiment, the culture is inoculated at another growth phase.

The solution used for freezing may contain another colligative additive or additive with anti-freeze properties, in place of or in addition to glycerol. In another embodiment, the additive is mannitol, DMSO, sucrose, or any other colligative additive or additive with anti-freeze properties that is known in the art.

The nutrient medium utilized for growing a culture of a *Listeria* strain may be LB, TB, or a modified, animal product-free Terrific Broth. In another embodiment, the nutrient medium is a defined medium. In another embodiment, the nutrient medium is a defined medium of the present invention. In another embodiment, the nutrient medium is any other type of nutrient medium known in the art.

The step of growing may be performed in a shaker flask, such as a baffled shaker flask, in a batch fermenter, in a stirred tank or flask, in an airflit fermenter, in a fed batch reactor, in a continuous cell reactor, or in an immobilized cell reactor.

In another embodiment, a constant pH is maintained during growth of the culture (e.g. in a batch fermenter). In another embodiment, the pH is maintained at about 7.0, about 6, about 6.5, about 7.5, or the pH is about 8. In another embodiment, the pH is 6.5-7.5. In another embodiment, the pH is 6-8. In another embodiment, the pH is 6-7. In another embodiment, the pH is 7-8.

In another embodiment, a constant temperature is maintained during growth of the culture. In another embodiment, the temperature is maintained at about 37°C. In another embodiment, the temperature is 37°C, 25°C, 27°C, 30°C,34°C, 36°C, 38°C, or In another embodiment, the temperature is 39°C.

In another embodiment, a constant dissolved oxygen concentration is maintained during growth of the culture. In another embodiment, the dissolved oxygen concentration is maintained at 20% of saturation. In another embodiment, the concentration is 15% of saturation, or 16% of saturation, or 18%, or 22%, or 25%, or 30%, or 35%, or 40%, or 45%, or 50%, or 55%, 60%, or 65%, or 70%, or 75%, or 80%, or 85%, or 90%, or 95%, or 100% of saturation, or near 100% of saturation.

In another embodiment, the *Listeria* culture is flash-frozen in liquid nitrogen, followed by storage at the final freezing temperature. In another embodiment, the culture is frozen in a more gradual manner; e.g. by placing in a vial of the culture in the final storage temperature.

In another embodiment, the storage temperature of the culture is between ⁻20 and ⁻80 degrees Celsius (°C). In another embodiment, the temperature is significantly below ⁻20°C. In another embodiment, the temperature is not warmer than ⁻70°C, or is ⁻70°C or is about ⁻70°C. In another embodiment, the temperature is ⁻20°Cor about ⁻20°C, ⁻30°C, ⁻40°C, ⁻50°C, ⁻60°C, ⁻80°C, -30 - ⁻70°C, -40 - ⁻70°C, -50 - - 70°C, -60 - ⁻70°C, -30 - ⁻80°C, -40 - ⁻80°C, -50 - ⁻80°C, ⁻60 - ⁻80°C, -70 - ⁻80°C, or the temperature is colder than ⁻70°C or colder than ⁻80°C.

Methods for lyophilization and cryopreservation of recombinant *Listeria* strains are well known to those skilled in the art.

The *Listeria*-containing composition is, in another embodiment, an immunogenic composition. In another embodiment, the composition is inherently immunogenic by virtue of its comprising a *Listeria* strain of the present invention. In another embodiment, the composition further comprises an adjuvant.

In some embodiments, the term "comprising" refers to the inclusion of other recombinant polypeptides, amino acid sequences, or nucleic acid sequences, as well as inclusion of other polypeptides, amino acid sequences, or nucleic acid sequences, that may be known in the art, which in one embodiment may comprise antigens or *Listeria* polypeptides, amino acid sequences, or nucleic acid sequences. In some embodiments, the term "consisting essentially of" refers to a composition for use in the methods as provided herein, which has the specific recombinant polypeptide, amino acid sequence, or nucleic acid sequence, or fragment thereof. However, other polypeptides, amino acid sequences, or nucleic acid sequences may be included that are not involved directly in the utility of the recombinant polypeptide(s). In some embodiments, the term "consisting of" refers to a composition for use in the methods as provided herein having a particular recombinant polypeptide, amino acid sequence, or nucleic acid sequence, or fragment or combination of recombinant polypeptides, amino acid sequences, or nucleic acid sequences or fragments as described herein.

In another embodiment, the present invention provides a recombinant polypeptide, comprising a KLK3 peptide operatively linked to an LLO peptidewhich enhances the immunogenicity of the KLK3 peptide.

As provided herein, a recombinant *Listeria* strain expressing an LLO-KLK3 fusion protects mice from tumors and elicits formation of antigen-specific CTL. Thus, *Listeria* strains expressing prostate-specific antigens (e.g. prostate-specific antigen/KLK3) are antigenic and efficacious in vaccination methods. Further, fusions of LLO and fragments thereof to prostate-specific antigens (e.g. prostate-specific antigen/KLK3) are antigenic and efficacious in vaccination methods.

Further, as provided herein, Lm-LLO-E7 induces regression of established subcutaneous HPV-16 immortalized tumors from C57B1/6 mice (Example 1). Further, as provided herein, Lm-LLO-NP protects mice from RENCA-NP, a renal cell carcinoma (Example 3). Further, as disclosed herein, fusion of antigens to ActA and PEST-like sequences produces similar results. Thus, non-hemolytic LLO, ActA, and PEST-like sequences are all efficacious at enhancing the immunogenicity of KLK3, PSCA and FOLH1 peptides.

In another embodiment, the present invention provides a vaccine comprising a recombinant fusion polypeptide of the present invention and an adjuvant.

In another embodiment, the present invention provides an immunogenic composition comprising a recombinant fusion polypeptide of the present invention.

In another embodiment, the present invention provides a recombinant vaccine vector encoding a recombinant fusion polypeptide of the present invention.

In another embodiment, the present invention provides a nucleotide molecule encoding a recombinant fusion polypeptide of the present invention.

In another embodiment, the present invention provides a vaccine comprising a nucleotide molecule of the present invention and an adjuvant.

In another embodiment, the present invention provides an immunogenic composition comprising a nucleotide molecule of the present invention.

In another embodiment, the present invention provides a recombinant vaccine vector comprising a nucleotide molecule of the present invention.

In other embodiments, the adjuvant of methods and compositions of the present invention is Montanide ISA 51. Montanide ISA 51 contains a natural metabolizable oil and a refined emulsifier. In another embodiment, the adjuvant is GM-CSF. In another embodiment, the adjuvant is KLH. Recombinant GM-CSF is a human protein grown, in another embodiment, in a yeast *(S. cerevisiae)* vector. GM-CSF promotes clonal expansion and differentiation of hematopoietic progenitor cells, APC, and dendritic cells and T cells.

The adjuvant may be a cytokine a growth factor, a cell population, QS21, Freund's incomplete adjuvant, aluminum phosphate, aluminum hydroxide, BCG, alum, an interleukin, an unmethylated CpG oligonucleotide, quill glycosides, monophosphoryl lipid A, liposomes, a bacterial mitogen, a bacterial toxin, or a chemokine. In another embodiment, the adjuvant is any other type of adjuvant known in the art. In another embodiment, the vaccine of the present invention comprises two of the above adjuvants. In another embodiment, the vaccine comprises more than two of the above adjuvants.

In another embodiment, the present invention provides a composition comprising a recombinant *Listeria* strain of the present invention for use in inducing an anti-KLK3 immune response in a subject.

In another embodiment, the present invention provides a composition comprising a recombinant *Listeria* strain of the present invention, for use in treating a KLK3-expressing tumor in a subject. In another embodiment, the KLK3 expressing tumor is a KLK3-expressing prostate cancer. In another embodiment, the KLK3-expressing tumor is a KLK3-expressing prostate carcinoma.

In one embodiment, "treating" refers to both therapeutic treatment and prophylactic or preventive measures, wherein the object is to prevent or lessen the targeted pathologic condition or disorder as described herein. Thus, in one embodiment, treating may include directly affecting or curing, suppressing, inhibiting, preventing, reducing the severity of, delaying the onset of, reducing symptoms associated with the disease, disorder or condition, or a combination thereof. Thus, in one embodiment, "treating" refers, *inter alia,* to delaying progression, expediting remission, inducing remission, augmenting remission, speeding recovery, increasing efficacy of or decreasing resistance to alternative therapeutics, or a combination thereof. In one embodiment, "preventing" or "impeding" refers, *inter alia,* to delaying the onset of symptoms, preventing relapse to a disease, decreasing the number or frequency of relapse episodes, increasing latency between symptomatic episodes, or a combination thereof. In one embodiment, "suppressing" or "inhibiting" or "protecting against" refer, *inter alia,* to reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

In another embodiment, the present invention provides a composition comprising a recombinant *Listeria* strain of the present invention for use in protecting a human subject against a KLK3-expressing tumor. In another embodiment, the KLK3 expressing tumor is a KLK3-expressing prostate cancer. In another embodiment, the KLK3-expressing tumor is a KLK3-expressing prostate carcinoma.

Methods for assessing efficacy of prostate cancer vaccines are well known in the art, and are described, for example, in Dzojic H et al. (Adenovirus-mediated CD40 ligand therapy induces tumor cell apoptosis and systemic immunity in the TRAMP-C2 mouse prostate cancer model. Prostate. 2006 Jun 1;66(8):831-8), Naruishi K et al. (Adenoviral vector-mediated RTVP-1 gene-modified tumor cell-based vaccine suppresses the development of experimental prostate cancer. Cancer Gene Ther. 2006 Jul;13(7):658-63), Sehgal I et al. (Cancer Cell Int. 2006 Aug 23;6:21), and Heinrich JE et al. (Vaccination against prostate cancer using a live tissue factor deficient cell line in Lobund-Wistar rats. Cancer Immunol Immunother 2007;56(5):725-30).

In another embodiment, the prostate cancer model used to test methods and compositions of the present invention is the PSA expressing TRAMP-C1 mouse tumor model (TPSA23). In another embodiment, the prostate cancer model is a 178-2 BMA cell model. In another embodiment, the prostate cancer model is a PAIII adenocarcinoma cells model. In another embodiment, the prostate cancer model is a PC-3M model. In another embodiment, the prostate cancer model is any other prostate cancer model known in the art.

In another embodiment, the vaccine is tested in human subjects, and efficacy is monitored using methods well known in the art, e.g., directly measuring CD4⁺ and CD8⁺ T cell responses, or measuring disease progression, e.g., by determining the number or size of tumor metastases, or monitoring disease symptoms (cough, chest pain, weight loss, etc.). Methods for assessing the efficacy of a prostate cancer vaccine in human subjects are well known in the art, and are described, for example, in Uenaka A et al. (T cell immunomonitoring and tumor responses in patients immunized with a complex of cholesterol-bearing hydrophobized pullulan (CHP) and NY-ESO-1 protein. Cancer Immun. 2007 Apr 19;7:9) and Thomas-Kaskel AK et al. (Vaccination of advanced prostate cancer patients with PSCA and PSA peptide-loaded dendritic cells induces DTH responses that correlate with superior overall survival. Int J Cancer. 2006 Nov 15;119(10):2428-34).

In another embodiment, the present invention provides a an immunogenic composition comprising a recombinant fusion polypeptide of the present invention for use in inducing an anti-KLK3 immune response in a subject.

In another embodiment, the present invention provides an immunogenic composition comprising a recombinant fusion polypeptide of the present invention for use in treating a KLK3 expressing tumor in a subject. In anembodiment, the KLK3 expressing tumor is a KLK3-expressing prostate cancer. In another embodiment, the KLK3-expressing tumor is a KLK3-expressing prostate carcinoma.

In another embodiment, the present invention provides an immunogenic composition comprising a recombinant fusion polypeptide of the present inventionfor use in protecting a human subject against a KLK3 expressing tumor. In another embodiment, the KLK3 expressing tumor is a KLK3-expressing prostate cancer. In another embodiment, the KLK3-expressing tumor is a KLK3-expressing prostate carcinoma.

In another embodiment, the present invention provides a nucleotide molecule of the present invention for use in inducing an anti-KLK3 immune response in a subject.

In another embodiment, the present invention provides an immunogenic composition comprising a nucleotide molecule of the present invention for use in treating a KLK3 expressing tumor in a subject. In another embodiment, the KLK3 expressing tumor is a KLK3-expressing prostate cancer. In another embodiment, the KLK3-expressing tumor is a KLK3-expressing prostate carcinoma.

In another embodiment, the present invention provides an immunogenic composition comprising a nucleotide molecule of the present invention for use in protecting a human subject against a KLK3 expressing tumor. In another embodiment, the KLK3 expressing tumor is a KLK3-expressing prostate cancer. In another embodiment, the KLK3-expressing tumor is a KLK3-expressing prostate carcinoma.

In another embodiment, the present invention provides a composition comprising a recombinant *Listeria* strain, wherein the strain comprises a recombinant fusion polypeptide of the present invention, for use in inducing an anti-KLK3 immune response in a subject.

In another embodiment, the present invention provides a composition comprising a recombinant *Listeria* strain, wherein the strain comprises a recombinant fusion polypeptide of the present invention, for use in treating a KLK3-expressing tumor in a subject. In another embodiment, the KLK3-expressing tumor is a KLK3-expressing prostate cancer. In another embodiment, the KLK3-expressing tumor is a KLK3-expressing prostate carcinoma.

In another embodiment, the present invention provides a composition comprising a recombinant *Listeria* strain, wherein the strain comprises a recombinant fusion polypeptide of the present invention for use in protecting a human subject against a KLK3-expressing tumor. In another embodiment, the KLK3-expressing tumor is a KLK3-expressing prostate cancer. In another embodiment, the KLK3-expressing tumor is a KLK3-expressing prostate carcinoma.

In another embodiment, the present invention provides composition comprising a recombinant *Listeria* strain of the present invention for use in impeding a growth of a KLK3-expressing prostate cancer tumor in a subject.

In another embodiment, the present invention provides a composition comprising a recombinant *Listeria* strain of the present invention for use in overcoming an immune tolerance of a subject to a KLK3-expressing prostate cancer tumor. In another embodiment, the present invention provides a combination of the treatments, which in one embodiment comprises priming with *Listeria* and boosting with polypeptide.

In another embodiment, the present invention provides an immunogenic composition comprising a recombinant fusion polypeptide of the present invention for use in impeding growth of a KLK3-expressing prostate cancer tumor in a subject.

In another embodiment, the present invention provides an immunogenic composition comprising a recombinant fusion polypeptide of the present inventionfor use in overcoming an immune tolerance of a subject to a KLK3-expressing prostate cancer tumor.

In another embodiment, the present invention provides an immunogenic composition comprising a nucleotide molecule of the present invention for use in impeding growth of a KLK3-expressing prostate cancer tumor in a subject.

In another embodiment, the present invention provides an immunogenic composition comprising a nucleotide molecule of the present invention for use in overcoming an immune tolerance of a subject to a KLK3-expressing prostate cancer tumor.

"Tolerance" refers, in another embodiment, to a lack of responsiveness of the host to an antigen. In another embodiment, the term refers to a lack of detectable responsiveness of the host to an antigen. In another embodiment, the term refers to a lack of immunogenicity of an antigen in a host. In another embodiment, tolerance is measured by lack of responsiveness in an *in vitro* CTL assay. In another embodiment, tolerance is measured by lack of responsiveness in a delayed-type hypersensitivity assay. In another embodiment, tolerance is measured by lack of responsiveness in any other suitable assay known in the art. In another embodiment, tolerance is determined or measured as depicted in the Examples herein.

"Overcome" refers, in another embodiment, to a reversible of tolerance by a vaccine. In another embodiment, the term refers to conferment of detectable immune response by a vaccine. In another embodiment, overcoming of immune tolerance is determined or measured as depicted in the Examples herein.

In another embodiment, the present invention provides a KLK3-expressing *Listeria* strain of the present invention for use in treating, or impeding the progression of, benign prostate hyperplasia (BPH) in a subject.

In another embodiment, the present invention provides a KLK3-expressing *Listeria* strain of the present invention for use in treating or impeding the progression of prostatic intraepithelial neoplasia (PIN) in a subject.

In another embodiment, the present invention provides a KLK3-containing fusion peptide of the present invention for use in treating or impeding the progression of BPH in a subject.

In another embodiment, the present invention provides a KLK3-containing fusion peptide of the present invention for use in treating or impeding the progression of PIN in a subject

In another embodiment, the present invention provides a a KLK3 fusion protein-encoding nucleotide molecule of the present invention for use in treating or impeding the progression of BPH in a subject.

In another embodiment, the present invention provides a KLK3 fusion protein-encoding nucleotide molecule of the present invention for use in treating or impeding the progression of prostatic intraepithelial neoplasia in a subject.

In another embodiment, fusion proteins of the present invention need not be expressed by LM, but rather can be expressed and isolated from other vectors and cell systems used for protein expression and isolation.

As provided herein, LLO-E7 fusions exhibit significant therapeutic efficacy. In these experiments, a vaccinia vector that expresses E7 as a fusion protein with a non-hemolytic truncated form of LLO was constructed. Expression of the LLO-E7 fusion product by plaque purified vaccinia was verified by Western blot using an antibody directed against the LLO protein sequence. Vac-LLO-E7 was demonstrated to produce CD8⁺ T cells specific to LLO and E7 as determined using the LLO (91-99) and E7 (49-57) epitopes of Balb/c and C57/BL6 mice, respectively. Results were confirmed by a CTL assay (Example 4).

Thus, expression of an antigen, e.g. KLK3, PSCA or FOLH1, as a fusion protein with a non-hemolytic truncated form of LLO, ActA, or a PEST-like sequence in host cell systems in *Listeria* and host cell systems other than *Listeria* results in enhanced immunogenicity of the antigen. While comparative experiments were performed with vaccinia, a multitude of other plasmids and expression systems which can be used to express these fusion proteins are known. For example, bacterial vectors useful in the present invention include, but are not limited to *Salmonella sp., Shigella sp.,* BCG, *L. monocytogenes* and *S*. *gordonii.* In addition the fusion proteins can be delivered by recombinant bacterial vectors modified to escape phagolysosomal fusion and live in the cytoplasm of the cell. Viral vectors useful in the present invention include, but are not limited to, vaccinia, avipox, adenovirus, AAV, vaccinia virus NYVAC, modified vaccinia strain Ankara (MVA), Semliki Forest virus, Venezuelan equine encephalitis virus, herpes viruses, and retroviruses. Naked DNA vectors can also be used.

In another embodiment, a KLK3 protein expressed by the target tumor cell shares complete homology with the KLK3 peptide (throughout the length of the peptide) expressed by the *Listerial* vector. In another embodiment, the KLK3 protein is highly homologous (throughout the length of the peptide) to the KLK3 peptide expressed by the *Listerial* vector. "Highly homologous" refers, in another embodiment, to a homology of greater than 90%. In another embodiment, the term refers to a homology of greater than 92%. In another embodiment, the term refers to a homology of greater than 93%. In another embodiment, the term refers to a homology of greater than 94%. In another embodiment, the term refers to a homology of greater than 95%. In another embodiment, the term refers to a homology of greater than 96%. In another embodiment, the term refers to a homology of greater than 97%. In another embodiment, the term refers to a homology of greater than 98%. In another embodiment, the term refers to a homology of greater than 99%. In another embodiment, the term refers to a homology of 100%.

The KLK3 peptide of the fusion protein of the present invention is, in another embodiment, 240-261 amino acids (AA) in length. In another embodiment, the length is 250-261 AA.. In another embodiment, the length is about 240 AA. In another embodiment, the length is about 260 AA.

In another embodiment, the KLK3 fragment consists of about 90% of the KLK3 protein. In another embodiment, the fragment consists of about 95% thereof. In another embodiment, the fragment consists of about 100% thereof.

In another embodiment, a KLK3 fusion peptide of the present invention is an immunogenic peptide. "Immunogenic" refers, in another embodiment, to an ability to induce an immune response when administered to a subject. In another embodiment, the subject is a human subject. In another embodiment, the immune response elicited is a T-cell response. In another embodiment, the immune response elicited is a cytotoxic T lymphocyte (CTL) response. In another embodiment, the immune response elicited is detectable. In another embodiment, the immune response elicited is detectable by an *in vitro* assay. In another embodiment, the assay is a cytokine release assay (e.g. fluorescence-activated cell sorting; or FACS). In another embodiment, the assay is a chromium-release assay or other *in vitro* cytotoxicity assay.

In another embodiment, the present invention provides a vaccine, immunogenic composition, or vector comprising a recombinant *Listeria* strain of the present invention for use in reducing a size of a KLK3-expressing tumor. In another embodiment, a cell of the tumor expresses KLK3.

In another embodiment, the present invention provides an effective amount of a vaccine comprising either: (a) a recombinant *Listeria* strain comprising an N-terminal fragment of an LLO protein fused to a KLK3 peptide; or (b) a recombinant nucleotide encoding the recombinant polypeptide for use in suppressing a formation of a KLK3-expressing tumor.

In another embodiment, the present invention provides a vaccine, immunogenic composition, or vector comprising a recombinant fusion polypeptide of the present inventionfor use in reducing a size of a KLK3-expressing tumor. In another embodiment, a cell of the tumor expresses KLK3.

In another embodiment, the present invention provides an effective amount of a vaccine comprising either: (a) a recombinant polypeptide comprising an N-terminal fragment of an LLO protein fused to a KLK3 peptide; or (b) a recombinant nucleotide encoding the recombinant polypeptide for use in suppressing a formation of a KLK3-expressing tumor.

In another embodiment, the present invention provides a vaccine, immunogenic composition, or vector comprising a recombinant nucleotide molecule of the present invention for use in reducing a size of a KLK3-expressing tumor. In another embodiment, a cell of the tumor expresses KLK3.

In another embodiment, the present invention provides an effective amount of a vaccine comprising either: (a) a recombinant nucleotide molecule comprising an N-terminal fragment of an LLO protein fused to a KLK3 peptide; or (b) a recombinant nucleotide encoding the recombinant polypeptide for use in suppressing a formation of a KLK3-expressing tumor.

In this invention, the recombinant fusion peptide of the medical uses and compositions of the present invention is an LLO-KLK3 fusion peptide comprising the sequence set forth in SEQ ID No: 54. In another embodiment, the fusion peptide has the sequence set forth in SEQ ID No: 54. In another embodiment, the fusion peptide is homologous to the sequence set forth in SEQ ID No: 54, and has a percentage identity to SEQ ID No: 54 of greater than 99%.

The sequence of the LLO protein utilized to construct vaccines of the present invention is, in another embodiment, a fragment of SEQ ID NO: 17 (GenBank Accession No. P13128;; nucleic acid sequence is set forth in GenBank Accession No. X15127). The first 25 amino acids of the proprotein corresponding to this sequence are the signal sequence and are cleaved from LLO when it is secreted by the bacterium. Thus, in this embodiment, the full length active LLO protein is 504 residues long.

In another embodiment, "LLO peptide" and "LLO fragment" refer to an N-terminal fragment of an LLO protein. In another embodiment, the terms refer to a full-length but non-hemolytic LLO protein. In another embodiment, the terms refer to a non-hemolytic protein containing a point mutation in cysteine 484 of sequence ID No: 17 or a corresponding residue thereof in a homologous LLO protein.

In another embodiment, the N-terminal fragment of an LLO protein utilized in compositions and methods of the present invention has the sequence of SEQ ID NO: 18.

As disclosed herein, recombinant *Listeria* strains expressing PEST-like sequence-antigen fusions induce anti-tumor immunity (Example 5) and generate antigen-specific, tumor-infiltrating T cells (Example 6).

In another embodiment of the present invention, "nucleic acids" or "nucleotide" refers to a string of at least two base-sugar-phosphate combinations. The term includes, in one embodiment, DNA and RNA. "Nucleotides" refers, in one embodiment, to the monomeric units of nucleic acid polymers. RNA may be, in one embodiment, in the form of a tRNA (transfer RNA), snRNA (small nuclear RNA), rRNA (ribosomal RNA), mRNA (messenger RNA), anti-sense RNA, small inhibitory RNA (siRNA), micro RNA (miRNA) and ribozymes. The use of siRNA and miRNA has been described (Caudy AA et al., Genes & Devel 16: 2491-96 and references cited therein). DNA may be in form of plasmid DNA, viral DNA, linear DNA, or chromosomal DNA or derivatives of these groups. In addition, these forms of DNA and RNA may be single, double, triple, or quadruple stranded. The term also includes, in another embodiment, artificial nucleic acids that may contain other types of backbones but the same bases. In one embodiment, the artificial nucleic acid is a PNA (peptide nucleic acid). PNA contain peptide backbones and nucleotide bases and are able to bind, in one embodiment, to both DNA and RNA molecules. In another embodiment, the nucleotide is oxetane modified. In another embodiment, the nucleotide is modified by replacement of one or more phosphodiester bonds with a phosphorothioate bond. In another embodiment, the artificial nucleic acid contains any other variant of the phosphate backbone of native nucleic acids known in the art. The use of phosphothiorate nucleic acids and PNA are known to those skilled in the art, and are described in, for example, Neilsen PE, Curr Opin Struct Biol 9:353-57; and Raz NK et al. Biochem Biophys Res Commun. 297:1075-84. The production and use of nucleic acids is known to those skilled in art and is described, for example, in Molecular Cloning, (2001), Sambrook and Russell, eds. and Methods in Enzymology: Methods for molecular cloning in eukaryotic cells (2003) Purchio and G. C. Fareed.

Also described herein is a kit comprising a reagent utilized in performing the present invention. In another embodiment, the present invention provides a kit comprising a composition, tool, or instrument of the present invention.

In another embodiment, the LLO fragment is attached to the KLK3 peptide by chemical conjugation. In another embodiment, paraformaldehyde is used for the conjugation. In another embodiment, maleimide is used for the conjugation. In another embodiment, the conjugation is performed using any suitable method known in the art.

In another embodiment, the KLK3 expressing tumor targeted by compositions of the present invention is a KLK3-expressing prostate cancer. In another embodiment, the KLK3-expressing tumor is a KLK3-expressing prostate carcinoma.

As disclosed herein, enhanced cell mediated immunity was demonstrated for fusion proteins comprising an antigen and truncated LLO containing the PEST-like amino acid sequence, SEQ ID NO: 1. The ΔLLO used in some of the Examples was 416 amino acids long (following cleavage of the signal peptide), as 88 residues from the carboxy terminus which is inclusive of the activation domain containing cysteine 484 were truncated. However, it is apparent from the present disclosure that other ΔLLO without the activation domain, and in particular cysteine 484, are efficacious in methods of the present invention.

As provided herein, fusion of an antigen to a non-hemolytic truncated form of listeriolysin O (LLO) enhanced immunogenicity. An LM vector that expresses and secretes a fusion product of human papilloma virus (HPV) strain 16 E7 and LLO was a more potent cancer immunotherapeutic for HPV-immortalized tumors than LM secreting the E7 protein alone. Further, a recombinant vaccinia virus that carries the gene for the fusion protein LLO-E7 is a more potent cancer immunotherapeutic for HPV-immortalized tumors than an isogenic strain of vaccinia that carries the gene for E7 protein alone. In comparison, a short fusion protein Lm-AZ/-E7 comprising the E7 antigen fused to the promoter, signal sequence and the first 7 AA residues of LLO was an ineffective anti-tumor immunotherapeutic. This short fusion protein terminates directly before the PEST-like sequence and does not contain it.

"Fusion protein" refers, in another embodiment, to a protein comprising 2 or more proteins linked together by peptide bonds or other chemical bonds. In another embodiment, the proteins are linked together directly by a peptide or other chemical bond. In another embodiment, the proteins are linked together with one or more amino acids (e.g. a "spacer") between the two or more proteins.

Fusion proteins comprising a KLK3 peptide are, in another embodiment, prepared by any suitable method. In another embodiment, a fusion protein is prepared by cloning and restriction of appropriate sequences or direct chemical synthesis by methods discussed below. In another embodiment, subsequences are cloned and the appropriate subsequences cleaved using appropriate restriction enzymes. The fragments are then ligated, in another embodiment, to produce the desired DNA sequence. In another embodiment, DNA encoding the KLK3 peptide is produced using DNA amplification methods, for example polymerase chain reaction (PCR). First, the segments of the native DNA on either side of the new terminus are amplified separately. The 5' end of the one amplified sequence encodes the peptide linker, while the 3' end of the other amplified sequence also encodes the peptide linker. Since the 5' end of the first fragment is complementary to the 3' end of the second fragment, the two fragments (after partial purification, e.g. on LMP agarose) can be used as an overlapping template in a third PCR reaction. The amplified sequence will contain codons, the segment on the carboxy side of the opening site (now forming the amino sequence), the linker, and the sequence on the amino side of the opening site (now forming the carboxyl sequence). The KLK3 peptide-encoding gene is then ligated into a plasmid.

In another embodiment, the KLK3 peptide is conjugated to the truncated LLO protein by any of a number of means well known to those of skill in the art. In another embodiment, the KLK3 peptide is conjugated, either directly or through a linker (spacer), to the protein. In another embodiment, wherein both the KLK3 peptide and the LLO protein are polypeptides, the chimeric molecule is recombinantly expressed as a single-chain fusion protein.

In another embodiment, the peptides and proteins of the present invention are readily prepared by standard, well-established solid-phase peptide synthesis (SPPS) as described by Stewart et al. in Solid Phase Peptide Synthesis, 2nd Edition, 1984, Pierce Chemical Company, Rockford, Ill.; and as described by Bodanszky and Bodanszky (The Practice of Peptide Synthesis, 1984, Springer-Verlag, New York). At the outset, a suitably protected amino acid residue is attached through its carboxyl group to a derivatized, insoluble polymeric support, such as cross-linked polystyrene or polyamide resin. "Suitably protected" refers to the presence of protecting groups on both the alpha-amino group of the amino acid, and on any side chain functional groups. Side chain protecting groups are generally stable to the solvents, reagents and reaction conditions used throughout the synthesis, and are removable under conditions which will not affect the final peptide product. Stepwise synthesis of the oligopeptide is carried out by the removal of the N-protecting group from the initial amino acid, and couple thereto of the carboxyl end of the next amino acid in the sequence of the desired peptide. This amino acid is also suitably protected. The carboxyl of the incoming amino acid can be activated to react with the N-terminus of the support-bound amino acid by formation into a reactive group such as formation into a carbodiimide, a symmetric acid anhydride or an "active ester" group such as hydroxybenzotriazole or pentafluorophenly esters.

Examples of solid phase peptide synthesis methods include the BOC method which utilized tert-butyloxcarbonyl as the alpha-amino protecting group, and the FMOC method which utilizes 9-fluorenylmethyloxcarbonyl to protect the alpha-amino of the amino acid residues, both methods of which are well-known by those of skill in the art.

Incorporation of N- and/or C-blocking groups can also be achieved using protocols conventional to solid phase peptide synthesis methods. For incorporation of C-terminal blocking groups, for example, synthesis of the desired peptide is typically performed using, as solid phase, a supporting resin that has been chemically modified so that cleavage from the resin results in a peptide having the desired C-terminal blocking group. To provide peptides in which the C-terminus bears a primary amino blocking group, for instance, synthesis is performed using a p-methylbenzhydrylamine (MBHA) resin so that, when peptide synthesis is completed, treatment with hydrofluoric acid releases the desired C-terminally amidated peptide. Similarly, incorporation of an N-methylamine blocking group at the C-terminus is achieved using N-methylaminoethyl-derivatized DVB, resin, which upon HF treatment releases a peptide bearing an N-methylamidated C-terminus. Blockage of the C-terminus by esterification can also be achieved using conventional procedures. This entails use of resin/blocking group combination that permits release of side-chain peptide from the resin, to allow for subsequent reaction with the desired alcohol, to form the ester function. FMOC protecting group, in combination with DVB resin derivatized with methoxyalkoxybenzyl alcohol or equivalent linker, can be used for this purpose, with cleavage from the support being effected by TFA in dicholoromethane. Esterification of the suitably activated carboxyl function e.g. with DCC, can then proceed by addition of the desired alcohol, followed by deprotection and isolation of the esterified peptide product.

Incorporation of N-terminal blocking groups can be achieved while the synthesized peptide is still attached to the resin, for instance by treatment with a suitable anhydride and nitrile. To incorporate an acetyl blocking group at the N-terminus, for instance, the resin coupled peptide can be treated with 20% acetic anhydride in acetonitrile. The N-blocked peptide product can then be cleaved from the resin, deprotected and subsequently isolated.

In another embodiment, to ensure that the peptide obtained from either chemical or biological synthetic techniques is the desired peptide, analysis of the peptide composition is conducted. In another embodiment, amino acid composition analysis is conducted using high resolution mass spectrometry to determine the molecular weight of the peptide. Alternatively, or additionally, the amino acid content of the peptide can be confirmed by hydrolyzing the peptide in aqueous acid, and separating, identifying and quantifying the components of the mixture using HPLC, or an amino acid analyzer. Protein sequencers, which sequentially degrade the peptide and identify the amino acids in order, may also be used to determine definitely the sequence of the peptide.

In another embodiment, prior to its use, the peptide is purified to remove contaminants. In this regard, it will be appreciated that the peptide will be purified so as to meet the standards set out by the appropriate regulatory agencies and guidelines. Any one of a number of a conventional purification procedures may be used to attain the required level of purity including, for example, reversed-phase high-pressure liquid chromatography (HPLC) using an alkylated silica column such as C₄-,C₈- or C₁₈-silica. A gradient mobile phase of increasing organic content is generally used to achieve purification, for example, acetonitrile in an aqueous buffer, usually containing a small amount of trifluoroacetic acid. Ion-exchange chromatography can be also used to separate peptides based on their charge.

Solid phase synthesis in which the C-terminal AA of the sequence is attached to an insoluble support followed by sequential addition of the remaining amino acids in the sequence is used, in another embodiment, for the chemical synthesis of the polypeptides of this invention. Techniques for solid phase synthesis are described by Barany and Merrifield in Solid-Phase Peptide Synthesis; pp. 3-284 in The Peptides: Analysis, Synthesis, Biology. Vol. 2: Special Methods in Peptide Synthesis, Part A., Merrifield et al. J. Am. Chem. Soc., 85: 2149-2156 (1963), and Stewart et al., Solid Phase Peptide Synthesis, 2nd ed. Pierce Chem. Co., Rockford, Ill. (1984).

In another embodiment, peptides of the present invention can incorporate AA residues which are modified without affecting activity. For example, the termini may be derivatized to include blocking groups, i.e. chemical substituents suitable to protect and/or stabilize the N- and C-termini from "undesirable degradation", a term meant to encompass any type of enzymatic, chemical or biochemical breakdown of the compound at its termini which is likely to affect the function of the compound, i.e. sequential degradation of the compound at a terminal end thereof.

In another embodiment, blocking groups include protecting groups conventionally used in the art of peptide chemistry which will not adversely affect the *in vivo* activities of the peptide. For example, suitable N-terminal blocking groups can be introduced by alkylation or acylation of the N-terminus. Examples of suitable N-terminal blocking groups include C₁-C₅ branched or unbranched alkyl groups, acyl groups such as formyl and acetyl groups, as well as substituted forms thereof, such as the acetamidomethyl (Acm) group. Desamino analogs of amino acids are also useful N-terminal blocking groups, and can either be coupled to the N-terminus of the peptide or used in place of the N-terminal reside. Suitable C-terminal blocking groups, in which the carboxyl group of the C-terminus is either incorporated or not, include esters, ketones or amides. Ester or ketone-forming alkyl groups, particularly lower alkyl groups such as methyl, ethyl and propyl, and amide-forming amino groups such as primary amines (--NH₂), and mono- and di-alkyl amino groups such as methyl amino, ethylamino, dimethylamino, diethylamino, methylethylamino and the like are examples of C-terminal blocking groups. Descarboxylated amino acid analogues such as agmatine are also useful C-terminal blocking groups and can be either coupled to the peptide's C-terminal residue or used in place of it. Further, it will be appreciated that the free amino and carboxyl groups at the termini can be removed altogether from the peptide to yield desamino and descarboxylated forms thereof without affect on peptide activity.

In another embodiment, other modifications are incorporated without adversely affecting the activity. In another embodiment, such modifications include, but are not limited to, substitution of one or more of the amino acids in the natural L-isomeric form with amino acids in the D-isomeric form. Thus, the peptide may include one or more D-amino acid resides, or may comprise amino acids which are all in the D-form. Retro-inverso forms of peptides in accordance with the present invention are also contemplated, for example, inverted peptides in which all amino acids are substituted with D-amino acid forms.

In another embodiment, acid addition salts peptides of the present invention are utilized as functional equivalents thereof. In another embodiment, a peptide in accordance with the present invention treated with an inorganic acid such as hydrochloric, hydrobromic, sulfuric, nitric, phosphoric, and the like, or an organic acid such as an acetic, propionic, glycolic, pyruvic, oxalic, malic, malonic, succinic, maleic, fumaric, tataric, citric, benzoic, cinnamie, mandelic, methanesulfonic, ethanesulfonic, p-toluenesulfonic, salicyclic and the like, to provide a water soluble salt of the peptide is suitable for use in the invention.

In another embodiment, modifications (which do not normally alter primary sequence) include *in vivo,* or *in vitro* chemical derivatization of polypeptides, e.g., acetylation, or carboxylation. Also included are modifications of glycosylation, e.g., those made by modifying the glycosylation patterns of a polypeptide during its synthesis and processing or in further processing steps; e.g., by exposing the polypeptide to enzymes which affect glycosylation, e.g., mammalian glycosylating or deglycosylating enzymes. Also embraced are sequences which have phosphorylated amino acid residues, e.g., phosphotyrosine, phosphoserine, or phosphothreonine.

In another embodiment polypeptides are modified using ordinary molecular biological techniques so as to improve their resistance to proteolytic degradation or to optimize solubility properties or to render them more suitable as a therapeutic agent. Analogs of such polypeptides include those containing residues other than naturally occurring L-amino acids, e.g., D-amino acids or non-naturally occurring synthetic amino acids. The peptides of the invention are not limited to products of any of the specific exemplary processes listed herein.

In another embodiment, the chimeric fusion proteins of the present invention are synthesized using recombinant DNA methodology. Generally this involves creating a DNA sequence that encodes the fusion protein, placing the DNA in an expression cassette, such as the plasmid of the present invention, under the control of a particular promoter/regulatory element, and expressing the protein. DNA encoding a fusion protein of the present invention are prepared, in another embodiment, by any suitable method, including, for example, cloning and restriction of appropriate sequences or direct chemical synthesis by methods such as the phosphotriester method of Narang et al. (1979, Meth. Enzymol. 68: 90-99); the phosphodiester method of Brown et al. (1979, Meth. Enzymol 68: 109-151); the diethylphosphoramidite method of Beaucage et al. (1981, Tetra. Lett., 22: 1859-1862); and the solid support method of U.S. Pat. No. 4,458,066.

Chemical synthesis produces a single stranded oligonucleotide. This is converted, in another embodiment, into double stranded DNA by hybridization with a complementary sequence, or by polymerization with a DNA polymerase using the single strand as a template. One of skill in the art would recognize that while chemical synthesis of DNA is limited to sequences of about 100 bases, longer sequences may be obtained by the ligation of shorter sequences.

In another embodiment, "isolated nucleic acid" includes an RNA or a DNA sequence encoding a fusion protein of the invention, and any modified forms thereof, including chemical modifications of the DNA or RNA which render the nucleotide sequence more stable when it is cell free or when it is associated with a cell. Chemical modifications of nucleotides may also be used to enhance the efficiency with which a nucleotide sequence is taken up by a cell or the efficiency with which it is expressed in a cell. Such modifications are detailed elsewhere herein. Any and all combinations of modifications of the nucleotide sequences are contemplated in the present invention.

In another embodiment, the present invention provides an isolated nucleic acid encoding a KLK3 peptide operably linked to a non-hemolytic LLO sequence, wherein the isolated nucleic acid further comprises a promoter/regulatory sequence, such that the nucleic acid is preferably capable of directing expression of the protein encoded by the nucleic acid. Thus, the invention encompasses expression vectors and methods for the introduction of exogenous DNA into cells with concomitant expression of the exogenous DNA in the cells such as those described, for example, in Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York).

In another embodiment, a nucleotide of the present invention is operably linked to a promoter/regulatory sequence that drives expression of the encoded peptide in the *Listeria* strain. Promoter/regulatory sequences useful for driving constitutive expression of a gene are well known in the art and include, but are not limited to, for example, the P_{hlyA}, P_{ActA}, and p60 promoters of *Listeria,* the *Streptococcus* bac promoter, the *Streptomyces griseus* sgiA promoter, and the *B. thuringiensis* phaZ promoter. Thus, it will be appreciated that the invention includes the use of any promoter/regulatory sequence that is capable of driving expression of the desired protein operably linked thereto.

Expressing a KLK3 peptide operably linked to a non-hemolytic LLO sequence using a vector allows the isolation of large amounts of recombinantly produced protein. It is well within the skill of the artisan to choose particular promoter/regulatory sequences and operably link those promoter/regulatory sequences to a DNA sequence encoding a desired polypeptide. Such technology is well known in the art and is described, for example, in Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York).

In another embodiment, the present invention provides a vector comprising an isolated nucleic acid encoding a KLK3 peptide operably linked to a non-hemolytic LLO sequence. The incorporation of a desired nucleic acid into a vector and the choice of vectors is well-known in the art as described in, for example, Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York).

In another embodiment, the present invention provides cells, viruses, proviruses, and the like, containing such vectors. Methods for producing cells comprising vectors and/or exogenous nucleic acids are well-known in the art. See, for example, Sambrook et al. (1989, Molecular Cloning: A Laboratory Manual, Cold Spring Harbor Laboratory, New York), and in Ausubel et al. (1997, Current Protocols in Molecular Biology, John Wiley & Sons, New York).

In another embodiment, the nucleic acids encoding a KLK3 peptide operably linked to a non-hemolytic LLO sequence are cloned into a plasmid vector. In another embodiment, a recombinant *Listeria* strain is transformed with the plasmid vector.

Once armed with the present invention, it is readily apparent to one skilled in the art that other nucleic acids encoding a KLK3 peptide operably linked to a non-hemolytic LLO sequence can be obtained by following the procedures described herein in the experimental details section for the generation of other fusion proteins as disclosed herein (e.g., site-directed mutagenesis, frame shift mutations, and the like), and procedures in the art.

Methods for the generation of derivative or variant forms of fusion proteins are well known in the art, and include, inter alia, using recombinant DNA methodology well known in the art such as, for example, that described in Sambrook et al. (1989, Molecular Cloning: A Laboratory Manual, Cold Spring Harbor Laboratory Press, New York) and Ausubel et al. (1997, Current Protocols in Molecular Biology, Green & Wiley, New York), and elsewhere herein.

In another embodiment, the present invention provides a nucleic acid encoding a KLK3 peptide operably linked to a non-hemolytic LLO sequence, wherein a nucleic acid encoding a tag polypeptide is covalently linked thereto. That is, the invention encompasses a chimeric nucleic acid wherein the nucleic acid sequence encoding a tag polypeptide is covalently linked to the nucleic acid encoding a KLK3 peptide-containing protein. Such tag polypeptides are well known in the art and include, for instance, green fluorescent protein (GFP), myc, myc-pyruvate kinase (myc-PK), His₆, maltose biding protein (MBP), an influenza virus hemagglutinin tag polypeptide, a flag tag polypeptide (FLAG), and a glutathione-S-transferase (GST) tag polypeptide. However, the invention should in no way be construed to be limited to the nucleic acids encoding the above-listed tag polypeptides. Rather, any nucleic acid sequence encoding a polypeptide which may function in a manner substantially similar to these tag polypeptides should be construed to be included in the present invention.

In another embodiment, the present invention provides a KLK3 fusion peptide with enhanced immunogenicity. That is, as the data disclosed herein demonstrate, a KLK3 peptide fused to a truncated non-hemolytic LLO protein, when administered to an animal, results in a clearance of existing tumors and the induction of antigen-specific cytotoxic lymphocytes capable of infiltrating tumor or infected cells. When armed with the present disclosure, and the medical uses and compositions disclosed herein, the skilled artisan will readily realize that the present invention in amenable to treatment and/or prevention of a multitude of diseases.

In another embodiment, a commercially available plasmid is used in the present invention. Such plasmids are available from a variety of sources, for example, Invitrogen (Carlsbad, Calif.), Stratagene (La Jolla, Calif.), Clontech (Palo Alto, Calif.), or can be constructed using methods well known in the art. A commercially available plasmid such as pET (Gibbstown, NJ), which is a prokaryotic expression vector with a prokaryotic origin of replication and promoter/regulatory elements to facilitate expression in a prokaryotic organism.

The present invention further comprises transforming such a *Listeria* strain with a plasmid comprising (a) a KLK3 peptide; and (b) an isolated nucleic acid encoding a truncated truncated LLO protein. In another embodiment, if an LM vaccine strain comprises a deletion in the prfA gene or the actA gene, the plasmid comprises a prfA or actA gene in order to complement the mutation, thereby restoring function to the *L. monocytogenes* vaccine strain. As described elsewhere herein, methods for transforming bacteria are well known in the art, and include calcium-chloride competent cell-based methods, electroporation methods, bacteriophage-mediated transduction, chemical, and physical transformation techniques (de Boer et al., 1989, Cell 56:641-649; Miller et al., 1995, FASEB J., 9:190-199; Sambrook et al. 1989, Molecular Cloning: A Laboratory Manual, Cold Spring Harbor Laboratory, New York; Ausubel et al., 1997, Current Protocols in Molecular Biology, John Wiley & Sons, New York; Gerhardt et al., eds., 1994, Methods for General and Molecular Bacteriology, American Society for Microbiology, Washington, D.C.; Miller, 1992, A Short Course in Bacterial Genetics, Cold Spring Harbor Laboratory Press, Cold Spring Harbor, N.Y.).

The plasmid of the present invention comprises, in another embodiment, a promoter/regulatory sequence operably linked to a gene encoding a fusion protein.

Plasmids and other expression vectors useful in the present invention are described elsewhere herein, and can include such features as a promoter/regulatory sequence, an origin of replication for gram negative and/or gram positive bacteria, and an isolated nucleic acid encoding a fusion protein. Further, the isolated nucleic acid encoding a fusion protein will have its own promoter suitable for driving expression of such an isolated nucleic acid. Promoters useful for driving expression in a bacterial system are well known in the art, and include bacteriophage lambda, the bla promoter of the beta-lactamase gene of pBR322, and the CAT promoter of the chloramphenicol acetyl transferase gene of pBR325. Further examples of prokaryotic promoters include the major right and left promoters of bacteriophage lambda (P_{L} and P_{R}), the trp, recA, lacZ, lacd, and gal promoters of E. coli, the alpha-amylase (Ulmanen et al, 1985. J. Bacteriol. 162:176-182) and the S28-specific promoters of *B. subtilis* (Gilman et al, 1984 Gene 32:11-20), the promoters of the bacteriophages of *Bacillus* (Gryczan, 1982, In: The Molecular Biology of the Bacilli, Academic Press, Inc., New York), and *Streptomyces* promoters (Ward et al, 1986, Mol. Gen. Genet. 203:468-478). Additional prokaryotic promoters contemplated in the present invention are reviewed in, for example, Glick (1987, J. Ind. Microbiol. 1:277-282); Cenatiempo, (1986, Biochimie, 68:505-516); and Gottesman, (1984, Ann. Rev. Genet. 18:415-442). Further examples of promoter/regulatory elements contemplated in the present invention include, but are not limited to the *Listerial* prfA promoter (GenBank Acc. No. Y07639), the *Listerial* hly promoter (GenBank Acc. No. X15127), and the *Listerial* p60 promoter (GenBank Acc. No. AY126342), or fragments thereof.

In another embodiment, a *Listeria* strain of the present invention contains an integrated gene encoding a peptide that comprises a KLK3 fusion peptide.

In another embodiment, a *Listeria* strain of the present invention is created using a site-specific integration vector. In another embodiment, a *Listeria* strain containing an integrated gene is created using homologous recombination. In another embodiment, a *Listeria* strain containing an integrated gene is created using any other method known in the art of integrating a gene into the *Listeria* chromosome.

In another embodiment, the integration vector comprises a PSA attPP' site. In another embodiment, the integration vector comprises a gene encoding a PSA integrase. In another embodiment, the integration vector comprises a U153 attPP' site. In another embodiment, the integration vector comprises a gene encoding a U153 integrase. In another embodiment, the integration vector comprises an A118 attPP' site. In another embodiment, the integration vector comprises a gene encoding an A118 integrase. In another embodiment, the integration vector comprises any other attPP' site known in the art. In another embodiment, the integration vector comprises any other phage integrase known in the art.

In another embodiment, a *Listeria* strain of methods and compositions of the present invention contains a mutation or auxotrophy in a metabolic gene. In another embodiment, a *Listeria* strain of methods and compositions of the present invention further comprises a mutation in the endogenous ActA gene. In another embodiment, a plasmid carrying a KLK3 fusion peptide comprises a metabolic gene that complements the mutation or auxotrophy. In another embodiment, a KLK3 fusion peptide-encoding integration vector or construct used for integration into the *Listeria* chromosome contains a gene that complements the mutation or auxotrophy. In another embodiment, the metabolic gene is used for selection instead of an antibiotic resistance gene. In another embodiment, the metabolic gene is used for selection in addition to an antibiotic resistance gene.

In another embodiment, the metabolic gene is a gene encoding an amino acid metabolism enzyme. In another embodiment, the metabolic enzyme is an alanine racemase (dal) enzyme. In another embodiment, the metabolic enzyme is D-amino acid transferase enzyme (dat).

In another embodiment, the metabolic enzyme metabolizes an amino acid (AA) that is used for a bacterial growth process. In another embodiment, the product AA is used for a replication process. In another embodiment, the product AA is used for cell wall synthesis. In another embodiment, the metabolic enzyme catalyzes the formation of an amino acid used for cell wall synthesis. In another embodiment, the metabolic enzyme is an amino acid metabolism enzyme. In another embodiment, the product AA is used for protein synthesis. In another embodiment, the product AA is used for metabolism of a fatty acid. In another embodiment, the product AA is used for any other growth or replication process known in the art.

In another embodiment, the metabolic enzyme catalyzes the formation of an AA used in cell wall synthesis. In another embodiment, the metabolic enzyme catalyzes synthesis of an AA used in cell wall synthesis. In another embodiment, the metabolic enzyme is involved in synthesis of an AA used in cell wall synthesis. In another embodiment, the AA is used in cell wall biogenesis.

In another embodiment, the metabolic enzyme is a synthetic enzyme for D-glutamic acid, a cell wall component.

In another embodiment, the metabolic enzyme is encoded by an alanine racemase gene (dal) gene. D-glutamic acid synthesis is controlled in part by the dal gene, which is involved in the conversion of D-glu + pyr to alpha-ketoglutarate + D-ala, and the reverse reaction.

In another embodiment, the dal protein of the methods and compositions of the present invention has the sequence of SEQ ID No: 56 (GenBank Accession No: AF038438). In another embodiment, the dal protein is homologous to SEQ ID No: 56. In another embodiment, the dal protein is a variant of SEQ ID No: 56. In another embodiment, the dal protein is an isoform of SEQ ID No: 56. In another embodiment, the dal protein is a fragment of SEQ ID No: 56. In another embodiment, the dal protein is a fragment of a homologue of SEQ ID No: 56. In another embodiment, the dal protein is a fragment of a variant of SEQ ID No: 56. In another embodiment, the dal protein is a fragment of an isoform of SEQ ID No: 56.

In another embodiment, the dal protein is any other *Listeria* dal protein known in the art. In another embodiment, the dal protein is any Bacillus subtilis dal protein known in the art. In another embodiment, the dal protein is any other gram-positive dal protein known in the art. In another embodiment, the dal protein is any other dal protein known in the art.

The dat protein of methods and compositions of the present invention is encoded, in another embodiment, by the sequence of SEQ ID No: 57 (GenBank Accession No: AF038439). In another embodiment, the dat protein is homologous to SEQ ID No: 57. In another embodiment, the dat protein is a variant of SEQ ID No: 57. In another embodiment, the dat protein is an isoform of SEQ ID No: 57. In another embodiment, the dat protein is a fragment of SEQ ID No: 57. In another embodiment, the dat protein is a fragment of a homologue of SEQ ID No: 57. In another embodiment, the dat protein is a fragment of a variant of SEQ ID No: 57. In another embodiment, the dat protein is a fragment of an isoform of SEQ ID No: 57.

In another embodiment, the dat protein is any other *Listeria* dat protein known in the art. In another embodiment, the dat protein is any other gram-positive dat protein known in the art. In another embodiment, the dat protein is any other dat protein known in the art.

In another embodiment, the metabolic enzyme is a D-glutamic acid synthesis gene. In another embodiment, the metabolic enzyme is encoded by dga. In another embodiment, the metabolic enzyme is encoded by an alr (alanine racemase) gene. In another embodiment, the metabolic enzyme is any other enzyme known in the art that is involved in alanine synthesis.

In another embodiment, the metabolic enzyme is encoded by serC, a phosphoserine aminotransferase. In another embodiment, the metabolic enzyme is encoded by asd (aspartate beta-semialdehyde dehydrogenase), involved in synthesis of the cell wall constituent diaminopimelic acid. In another embodiment, the metabolic enzyme is encoded by gsaB- glutamate-1-semialdehyde aminotransferase, which catalyzes the formation of 5-aminolevulinate from (S)-4-amino-5-oxopentanoate. In another embodiment, the metabolic enzyme is encoded by HemL, which catalyzes the formation of 5-aminolevulinate from (S)-4-amino-5-oxopentanoate. In another embodiment, the metabolic enzyme is encoded by aspB, an aspartate aminotransferase that catalyzes the formation of oxalozcetate and L-glutamate from L-aspartate and 2-oxoglutarate. In another embodiment, the metabolic enzyme is encoded by argF-1, involved in arginine biosynthesis. In another embodiment, the metabolic enzyme is encoded by aroE, involved in amino acid biosynthesis. In another embodiment, the metabolic enzyme is encoded by aroB, involved in 3-dehydroquinate biosynthesis. In another embodiment, the metabolic enzyme is encoded by aroD, involved in amino acid biosynthesis. In another embodiment, the metabolic enzyme is encoded by aroC, involved in amino acid biosynthesis. In another embodiment, the metabolic enzyme is encoded by hisB, involved in histidine biosynthesis. In another embodiment, the metabolic enzyme is encoded by hisD, involved in histidine biosynthesis. In another embodiment, the metabolic enzyme is encoded by hisG, involved in histidine biosynthesis. In another embodiment, the metabolic enzyme is encoded by metX, involved in methionine biosynthesis. In another embodiment, the metabolic enzyme is encoded by proB, involved in proline biosynthesis. In another embodiment, the metabolic enzyme is encoded by argR, involved in arginine biosynthesis. In another embodiment, the metabolic enzyme is encoded by argJ, involved in arginine biosynthesis. In another embodiment, the metabolic enzyme is encoded by thiI, involved in thiamine biosynthesis. In another embodiment, the metabolic enzyme is encoded by LMOf2365_1652, involved in tryptophan biosynthesis. In another embodiment, the metabolic enzyme is encoded by aroA, involved in tryptophan biosynthesis. In another embodiment, the metabolic enzyme is encoded by ilvD, involved in valine and isoleucine biosynthesis. In another embodiment, the metabolic enzyme is encoded by ilvC, involved in valine and isoleucine biosynthesis. In another embodiment, the metabolic enzyme is encoded by leuA, involved in leucine biosynthesis. In another embodiment, the metabolic enzyme is encoded by dapF, involved in lysine biosynthesis. In another embodiment, the metabolic enzyme is encoded by thrB, involved in threonine biosynthesis (all GenBank Accession No. NC_002973).

In another embodiment, the metabolic enzyme is a tRNA synthetase. In another embodiment, the metabolic enzyme is encoded by the trpS gene, encoding tryptophanyltRNA synthetase. In another embodiment, the metabolic enzyme is any other tRNA synthetase known in the art.

In another embodiment, the host strain bacteria is Δ(trpS aroA), and both markers are contained in the integration vector.

In another embodiment, the metabolic enzyme is encoded by murE, involved in synthesis of diaminopimelic acid (GenBank Accession No: NC_003485).

In another embodiment, the metabolic enzyme is encoded by LMOf2365_2494, involved in teichoic acid biosynthesis.

In another embodiment, the metabolic enzyme is encoded by WecE (lipopolysaccharide biosynthesis protein rffA; GenBank Accession No: AE014075.1). In another embodiment, the metabolic enzyme is encoded by amiA, an N-acetylmuramoyl-L-alanine amidase. In another embodiment, the metabolic enzyme is aspartate aminotransferase. In another embodiment, the metabolic enzyme is histidinol-phosphate aminotransferase (GenBank Accession No. NP_466347). In another embodiment, the metabolic enzyme is the cell wall teichoic acid glycosylation protein GtcA.

In another embodiment, the metabolic enzyme is a synthetic enzyme for a peptidoglycan component or precursor. In another embodiment, the component is UDP-N-acetylmuramyl-pentapeptide. In another embodiment, the component is UDP-N-acetylglucosamine. In another embodiment, the component is MurNAc-(pentapeptide)-pyrophosphoryl-undecaprenol. In another embodiment, the component is GlcNAc-Δ-(1,4)-MurNAc-(pentapeptide)-pyrophosphoryl-undecaprenol. In another embodiment, the component is any other peptidoglycan component or precursor known in the art.

In another embodiment, the metabolic enzyme is encoded by murG. In another embodiment, the metabolic enzyme is encoded by murD. In another embodiment, the metabolic enzyme is encoded by murA-1. In another embodiment, the metabolic enzyme is encoded by murA-2 (all set forth in GenBank Accession No. NC_002973). In another embodiment, the metabolic enzyme is any other synthetic enzyme for a peptidoglycan component or precursor.

In another embodiment, the metabolic enzyme is a trans-glycosylase. In another embodiment, the metabolic enzyme is trans-peptidase. In another embodiment, the metabolic enzyme is a carboxypeptidase. In another embodiment, the metabolic enzyme is any other class of metabolic enzyme known in the art.

In another embodiment, the metabolic enzyme is any other *Listeria monocytogenes* metabolic enzyme known in the art.

In another embodiment, the metabolic enzyme is any other *Listeria* metabolic enzyme known in the art.

In another embodiment, the metabolic enzyme is any other gram-positive bacteria metabolic enzyme known in the art.

In another embodiment, the metabolic enzyme is any other metabolic enzyme known in the art.

In another embodiment, the integration vector is any other site-specific integration vector known in the art that is capable of infecting *Listeria.*

In another embodiment, the present invention provides compositions for enhancing the immunogenicity of a KLK3 antigen via fusion of the antigen to a non-hemolytic truncated form of LLO ("ΔLLO"), such as SEQ ID NO: 1 of LLO. The present invention further provides methods and compositions for enhancing the immunogenicity of a KLK3 antigen by fusing the antigen to a truncated LLO protein. As demonstrated by the data disclosed herein, an antigen fused to an ActA protein elicits an immune response that clears existing tumors and results in the induction of antigen-specific cytotoxic lymphocytes.

In another embodiment, fusion proteins of the present invention are produced recombinantly via transcription and translation, in a bacterium, of a plasmid or nucleotide molecule that encodes both a KLK3 peptide and an LLO peptide. In another embodiment, the plasmid or nucleotide is transcribed and/or translated *in vitro.* In another embodiment, the antigen is chemically conjugated to the truncated form of LLO comprising the PEST-like AA sequence of L. monocytogenes or a PEST-like AA sequence derived from another prokaryotic organism. "Antigen" refers, in another embodiment, to the native KLK3 gene product or truncated versions of these that include identified T cell epitopes. In another embodiment, these fusion proteins are then incorporated into vaccines for administration to a subject, to invoke an enhanced immune response against the antigen of the fusion protein. In other embodiments, the fusion proteins of the present invention are delivered as DNA vaccines, RNA vaccines or replicating RNA vaccines. As will be apparent to those of skill in the art upon this disclosure, vaccines comprising the fusion proteins of the present invention are particularly useful in the prevention and treatment of infectious and neoplastic diseases.

The present invention further comprises a composition comprising a vaccine of the present invention suitable for administering to an animal or human. The composition comprises, among other things, a pharmaceutically acceptable carrier. In another embodiment, the composition includes a *Listeria* vaccine strain comprising a truncated LLO protein, or fragment thereof, fused to a KLK3 peptide, and a pharmaceutically acceptable carrier.

To the extent that the following Examples do not relate to the fusion of a KLK3 peptide and an LLO protein, they are comparative examples.

### EXPERIMENTAL DETAILS SECTION

### EXAMPLE 1: LLO-ANTIGEN FUSIONS INDUCE ANTI-TUMOR IMMUNITY

### MATERIALS AND EXPERIMENTAL METHODS (EXAMPLES 1-2)

### Cell lines

The C57BL/6 syngeneic TC-1 tumor was immortalized with HPV-16 E6 and E7 and transformed with the c-Ha-ras oncogene. TC-1 expresses low levels of E6 and E7 and is highly tumorigenic. TC-1 was grown in RPMI 1640, 10% FCS, 2 mM L-glutamine, 100 U/ml penicillin, 100 µg/ml streptomycin, 100 µM nonessential amino acids, 1 mM sodium pyruvate, 50 micromolar (mcM) 2-ME, 400 microgram (mcg)/ml G418, and 10% National Collection Type Culture-109 medium at 37° with 10% CO₂. C3 is a mouse embryo cell from C57BL/6 mice immortalized with the complete genome of HPV 16 and transformed with pEJ-ras. EL-4/E7 is the thymoma EL-4 retrovirally transduced with E7.

### L. monocytogenes strains and propagation

*Listeria* strains used were Lm-LLO-E7 (hly-E7 fusion gene in an episomal expression system; Figure 1A), Lm-E7 (single-copy E7 gene cassette integrated into *Listeria* genome), Lm-LLO-NP ("DP-L2028"; hly-NP fusion gene in an episomal expression system), and Lm-Gag ("ZY-18"; single-copy HIV-1 Gag gene cassette integrated into the chromosome).

To generate pGG-55, the LLO-E7 plasmid, E7 was amplified by PCR using the primers 5'-GGCTCGAGCATGGAGATACACC-3' (SEQ ID No: 8; XhoI site is underlined) and 5'-GGGGACTAGTTTATGGTTTCTGAGAACA-3' (SEQ ID No: 9; SpeI site is underlined) and ligated into pCR2.1 (Invitrogen, San Diego, CA). E7 was excised from pCR2.1 by XhoI/ SpeI digestion and ligated into pDP-2028 (Ikonomidis G et al. Delivery of a viral antigen to the class I processing and presentation pathway by Listeria monocytogenes. J Exp Med. 1994 Dec 1;180(6):2209-18). The hly-E7 fusion gene and the pluripotential transcription factor prfA were amplified and subcloned into pAM401, a multicopy shuttle plasmid (Wirth R et al., J Bacteriol, 165: 831, 1986), generating pGG-55. The hly promoter and gene fragment were amplified using primers 5'-GGGGGCTAGCCCTCCTTTGATTAGTATATTC-3' (SEQ ID No: 10; NheI site is underlined) and 5'-CTCCCTCGAGATCATAATTTACTTCATC-3' (SEQ ID No: 11; XhoI site is underlined). The prfA gene was PCR amplified using primers 5'-GACTACAAGGACGATGACCGACAAGTGATAACCCGGGATCTAAATAAATCCGTTT-3' (SEQ ID No: 12; XbaI site is underlined) and 5'-CCCGTCGACCAGCTCTTCTTGGTGAAG-3' (SEQ ID No: 13; SalI site is underlined).

In the resulting plasmid, pGG-55, the hly promoter drives the expression of the first 441 AA of the hly gene product, including the subsequently cleaved signal sequence, which is joined by the XhoI site to the E7 gene, yielding a hly-E7 fusion gene that is transcribed and secreted as LLO-E7. This LLO fragment lacks the hemolytic C-terminus and has the sequence set forth in SEQ ID No: 18. It is referred to below as "ΔLLO," and is merely an exemplary ΔLLO of many that could be used with methods and compositions of the present invention. Transformation of a prfA-negative strain of Listeria, XFL-7 (provided by Dr. Hao Shen, University of Pennsylvania), with pGG-55 selected for the retention of the plasmid *in vivo* (Figures 1A-B).

Lm-E7 was generated by introducing an expression cassette containing the hly promoter and signal sequence driving the expression and secretion of E7 into the orfZ domain of the LM genome. E7 was amplified by PCR using the primers 5'-GCGGATCCCATGGAGATACACCTAC-3' (SEQ ID No: 22; BamHI site is underlined) and 5'-GCTCTAGATTATGGTTTCTGAG-3' (SEQ ID No: 23; XbaI site is underlined). E7 was then ligated into the pZY-21 shuttle vector. LM strain 10403S was transformed with the resulting plasmid, pZY-21-E7, which includes an expression cassette inserted in the middle of a 1.6-kb sequence that corresponds to the orfX, Y, Z domain of the LM genome. The homology domain allows for insertion of the E7 gene cassette into the orfZ domain by homologous recombination. Clones were screened for integration of the E7 gene cassette into the orfZ domain. Bacteria were grown in brain heart infusion medium with (Lm-LLO-E7 and Lm-LLO-NP) or without (Lm-E7 and ZY-18) chloramphenicol (20 µg/ml). Bacteria were frozen in aliquots at -80°C. Expression was verified by western blotting (Figure 2)

### Western blotting

*Listeria* strains were grown in Luria-Bertoni medium at 37°C and were harvested at the same optical density measured at 600 nm. The supernatants were TCA-precipitated and resuspended in 1x sample buffer supplemented with 0.1 N NaOH. Identical amounts of each cell pellet or each TCA-precipitated supernatant were loaded on 4-20% Tris-glycine SDS-PAGE gels (NOVEX, San Diego, CA). The gels were transferred to polyvinylidene difluoride and probed with an anti-E7 monoclonal antibody (mAb) (Zymed Laboratories, South San Francisco, CA), then incubated with HRP-conjugated anti-mouse secondary Ab (Amersham Pharmacia Biotech, Little Chalfont, U.K.), developed with Amersham ECL detection reagents, and exposed to Hyperfilm (Amersham Pharmacia Biotech).

### Measurement of tumor growth

Tumors were measured every other day with calipers spanning the shortest and longest surface diameters. The mean of these two measurements was plotted as the mean tumor diameter in millimeters against various time points. Mice were sacrificed when the tumor diameter reached 20 mm. Tumor measurements for each time point are shown only for surviving mice.

### Effects of Listeria recombinants on established tumor growth

Six- to 8-wk-old C57BL/6 mice (Charles River) received 2 x 10⁵ TC-1 cells s.c. on the left flank. One week following tumor inoculation, the tumors had reached a palpable size of 4-5 mm in diameter. Groups of 8 mice were then treated with 0.1 LD₅₀ i.p. Lm-LLO-E7 (10⁷ CFU), Lm- E7 (10⁶ CFU), Lm-LLO-NP (10⁷ CFU), or Lm-Gag (5 x 10⁵ CFU) on days 7 and 14.

### ⁵¹Cr release assay

C57BL/6 mice, 6-8 wk old, were immunized i.p. with 0.1LD₅₀ Lm-LLO-E7, Lm-E7, Lm-LLO-NP, or Lm-Gag. Ten days post-immunization, spleens were harvested. Splenocytes were established in culture with irradiated TC-1 cells (100:1, splenocytes:TC-1) as feeder cells; stimulated *in vitro* for 5 days, then used in a standard ⁵¹Cr release assay, using the following targets: EL-4, EL-4/E7, or EL-4 pulsed with E7 H-2b peptide (RAHYNIVTF). E:T cell ratios, performed in triplicate, were 80:1, 40:1, 20:1, 10:1, 5:1, and 2.5:1. Following a 4-h incubation at 37°C, cells were pelleted, and 50 µl supernatant was removed from each well. Samples were assayed with a Wallac 1450 scintillation counter (Gaithersburg, MD). The percent specific lysis was determined as [(experimental counts per minute - spontaneous counts per minute)/(total counts per minute - spontaneous counts per minute)] x 100.

### TC-1-specific proliferation

C57BL/6 mice were immunized with 0.1 LD₅₀ and boosted by i.p. injection 20 days later with 1 LD₅₀ Lm-LLO-E7, Lm-E7, Lm-LLO-NP, or Lm-Gag. Six days after boosting, spleens were harvested from immunized and naive mice. Splenocytes were established in culture at 5 x 10⁵/well in flat-bottom 96-well plates with 2.5 x 10⁴, 1.25 x 10⁴, 6 x 10³, or 3 x 10³ irradiated TC-1 cells/well as a source of E7 Ag, or without TC-1 cells or with 10 µg/ml Con A. Cells were pulsed 45 h later with 0.5 µCi [³H]thymidine/well. Plates were harvested 18 h later using a Tomtec harvester 96 (Orange, CT), and proliferation was assessed with a Wallac 1450 scintillation counter. The change in counts per minute was calculated as experimental counts per minute - no Ag counts per minute.

### Flow cytometric analysis

C57BL/6 mice were immunized intravenously (i.v.) with 0.1 LD₅₀ Lm-LLO-E7 or Lm-E7 and boosted 30 days later. Three-color flow cytometry for CD8 (53-6.7, PE conjugated), CD62 ligand (CD62L; MEL-14, APC conjugated), and E7 H-2Db tetramer was performed using a FACSCalibur® flow cytometer with CellQuest® software (Becton Dickinson, Mountain View, CA). Splenocytes harvested 5 days after the boost were stained at room temperature (rt) with H-2Db tetramers loaded with the E7 peptide (RAHYNIVTF) or a control (HIV-Gag) peptide. Tetramers were used at a 1/200 dilution and were provided by Dr. Larry R. Pease (Mayo Clinic, Rochester, MN) and by the National Institute of Allergy and Infectious Diseases Tetramer Core Facility and the National Institutes of Health AIDS Research and Reference Reagent Program. Tetramer⁺, CD8⁺, CD62L^{low} cells were analyzed.

### Depletion of specific immune components

CD8⁺ cells, CD4⁺ cells and IFN were depleted in TC-1-bearing mice by injecting the mice with 0.5 mg per mouse of mAb: 2.43, GK1.5, or xmg1.2, respectively, on days 6, 7, 8, 10, 12, and 14 post-tumor challenge. CD4⁺ and CD8⁺ cell populations were reduced by 99% (flow cytometric analysis). CD25⁺ cells were depleted by i.p. injection of 0.5 mg/mouse anti-CD25 mAb (PC61, provided by Andrew J. Caton) on days 4 and 6. TGF was depleted by i.p. injection of the anti-TGF- mAb (2G7, provided by H. I. Levitsky), into TC-1-bearing mice on days 6, 7, 8, 10, 12, 14, 16, 18, and 20. Mice were treated with 10⁷ Lm-LLO-E7 or Lm-E7 on day 7 following tumor challenge.

### Adoptive transfer

Donor C57BL/6 mice were immunized and boosted 7 days later with 0.1 LD₅₀ Lm-E7 or Lm-Gag. The donor splenocytes were harvested and passed over nylon wool columns to enrich for T cells. CD8⁺ T cells were depleted *in vitro* by incubating with 0.1 µg 2.43 anti-CD8 mAb for 30 min at rt. The labeled cells were then treated with rabbit complement. The donor splenocytes were >60% CD4⁺ T cells (flow cytometric analysis). TC-1 tumor-bearing recipient mice were immunized with 0.1 LD₅₀ 7 days post-tumor challenge. CD4⁺-enriched donor splenocytes (10⁷) were transferred 9 days after tumor challenge to recipient mice by i.v. injection.

### B16F0-Ova experiment

24 C57BL/6 mice were inoculated with 5 x 10⁵ B16F0-Ova cells. On days 3, 10 and 17, groups of 8 mice were immunized with 0.1 LD₅₀ Lm-OVA (10⁶ cfu), Lm-LLO-OVA (10⁸ cfu) and eight animals were left untreated.

### Statistics

For comparisons of tumor diameters, mean and SD of tumor size for each group were determined, and statistical significance was determined by Student's t test. p ≤ 0.05 was considered significant.

### RESULTS

Lm-E7 and Lm-LLO-E7 were compared for their abilities to impact on TC-1 growth. Subcutaneous tumors were established on the left flank of C57BL/6 mice. Seven days later tumors had reached a palpable size (4-5 mm). Mice were vaccinated on days 7 and 14 with 0.1 LD₅₀ Lm-E7, Lm-LLO-E7, or, as controls, Lm-Gag and Lm-LLO-NP. Lm-LLO-E7 induced complete regression of 75% of established TC-1 tumors, while the other two mice in the group controlled their tumor growth (Figure 3A). By contrast, immunization Lm-E7 and Lm-Gag did not induce tumor regression. This experiment was repeated multiple times, always with very similar results. In addition, similar results were achieved for Lm-LLO-E7 under different immunization protocols. In another experiment, a single immunization was able to cure mice of established 5 mm TC-1 tumors.

In other experiments, similar results were obtained with two other E7-expressing tumor cell lines: C3 and EL-4/E7. To confirm the efficacy of vaccination with Lm-LLO-E7, animals that had eliminated their tumors were re-challenged with TC-1 or EL-4/E7 tumor cells on day 60 or day 40, respectively. Animals immunized with Lm-LLO-E7remained tumor free until termination of the experiment (day 124 in the case of TC-1 and day 54 for EL-4/E7).

A similar experiment was performed with the chicken ovalbumin antigen (OVA). Mice were immunized with either Lm-OVA or Lm-LLO-OVA, then challenged with either an EL-4 thymoma engineered to express OVA or the very aggressive murine melanoma cell line B16F0-Ova, which has very low MHC class I expression. In both cases, Lm-LLO-OVA, but not Lm-OVA, induced the regression of established tumors. For example, at the end of the B16F0 experiment (day 25), all the mice in the naive group and the Lm-OVA group had died. All the Lm-LLO-OVA mice were alive, and 50% of them were tumor free. (Figure 3B).

Thus, expression of an antigen gene as a fusion protein with ΔLLO enhances the immunogenicity of the antigen.

### EXAMPLE 2: LM-LLO-E7 TREATMENT ELICITS TC-1 SPECIFIC SPLENOCYTE PROLIFERATION

To measure induction of T cells by Lm-E7 with Lm-LLO-E7, TC-1-specific proliferative responses of splenocytes from rLm-immunized mice, a measure of antigen-specific immunocompetence, were assessed. Splenocytes from Lm-LLO-E7-immunized mice proliferated when exposed to irradiated TC-1 cells as a source of E7, at splenocyte: TC-1 ratios of 20:1, 40:1, 80:1, and 160:1 (Figure 4). Conversely, splenocytes from Lm-E7 and rLm control immunized mice exhibited only background levels of proliferation.

### EXAMPLE 3: FUSION OF NP TO LLO ENHANCES ITS IMMUNOGENICITY

### MATERIALS AND EXPERIMENTAL METHODS

Lm-LLO-NP was prepared as depicted in Figure 1, except that influenza nucleoprotein (NP) replaced E7 as the antigen. 32 BALB/c mice were inoculated with 5 x 10⁵ RENCA-NP tumor cells. RENCA-NP is a renal cell carcinoma retrovirally transduced with influenza nucleoprotein NP (described in U.S. Pat. No. 5,830,702, which is incorporated herein by reference). After palpable macroscopic tumors had grown on day 10, 8 animals in each group were immunized i.p. with 0.1 LD₅₀ of the respective *Listeria* vector. The animals received a second immunization one week later.

### RESULTS

In order to confirm the generality of the finding that fusing LLO to an antigen confers enhanced immunity, Lm-LLO-NP and Lm-NP (isogenic with the Lm-E7 vectors, but expressing influenza antigen) were constructed, and the vectors were compared for ability to induce tumor regression, with Lm-Gag (isogenic with Lm-NP except for the antigen expressed) as a negative control. As depicted in Figure 5, 6/8 of the mice that received Lm-LLO-NP were tumor-free. By contrast, only 1/8 and 2/8 mice in the Lm-Gag and Lm-NP groups, respectively, were tumor-free. All the mice in the naive group had large tumors or had died by day 40. Thus, LLO strains expressing NP and LLO-NP fusions are immunogenic. Similar results were achieved for Lm-LLO-E7 under different immunization protocols. Further, just a single immunization was demonstrated to cure mice of established TC-1 of 5 mm diameter.

### EXAMPLE 4: ENHANCEMENT OF IMMUNOGENICITY BY FUSION OF AN ANTIGEN TO LLO DOES NOT REQUIRE A LISTERIA VECTOR

### MATERIALS AND EXPERIMENTAL METHODS

### Construction of Vac-SigE7Lamp

The WR strain of vaccinia was used as the recipient and the fusion gene was excised from the *Listerial* plasmid and inserted into pSC11 under the control of the p75 promoter. This vector was chosen because it is the transfer vector used for the vaccinia constructs Vac-SigE7Lamp and Vac-E7 and would therefore allow direct comparison with Vac-LLO-E7. In this way all three vaccinia recombinants would be expressed under control of the same early/late compound promoter p7.5. In addition, SC11 allows the selection of recombinant viral plaques to TK selection and beta-galactosidase screening. Figure 6 depicts the various vaccinia constructs used in these experiments. Vac-SigE7Lamp is a recombinant vaccinia virus that expressed the E7 protein fused between lysosomal associated membrane protein (LAMP-1) signal sequence and sequence from the cytoplasmic tail of LAMP-1. It was designed to facilitate the targeting of the antigen to the MHC class II pathway.

The following modifications were made to allow expression of the gene product by vaccinia: (a) the T5XT sequence that prevents early transcription by vaccinia was removed from the 5' portion of the LLO-E7 sequence by PCR; and (b) an additional XmaI restriction site was introduced by PCR to allow the final insertion of LLO-E7 into SC11. Successful introduction of these changes (without loss of the original sequence that encodes for LLO-E7) was verified by sequencing. The resultant pSC1 1-E7 construct was used to transfect the TK-ve cell line CV1 that had been infected with the wild-type vaccinia strain, WR. Cell lysates obtained from this co-infection/transfection step contain vaccinia recombinants that were plaque-purified 3 times. Expression of the LLO-E7 fusion product by plaque purified vaccinia was verified by Western blot using an antibody directed against the LLO protein sequence. In addition, the ability of Vac-LLO-E7 to produce CD8⁺ T cells specific to LLO and E7 was determined using the LLO (91-99) and E7 (49-57) epitopes of Balb/c and C57/BL6 mice, respectively. Results were confirmed in a chromium release assay.

### RESULTS

To determine whether enhancement of immunogenicity by fusion of an antigen to LLO requires a *Listeria* vector, a vaccinia vector expressing E7 as a fusion protein with a non-hemolytic truncated form of LLO (ΔLLO) was constructed. Tumor rejection studies were performed with TC-1 following the protocol described for Example 1. Two experiments were performed with differing delays before treatment was started. In one experiment, treatments were initiated when the tumors were about 3 mm in diameter (Figure 7). As of day 76, 50% of the Vac-LLO-E7 treated mice were tumor free, while only 25% of the Vac-SigE7Lamp mice were tumor free. In other experiments, ΔLLO-antigen fusions were more immunogenic than E7 peptide mixed with SBAS2 or unmethylated CpG oligonucleotides in a side-by-side comparison.

These results show that (a) fusion of ΔLLO-antigen fusions are immunogenic not only in the context of *Listeria,* but also in other contexts; and (b) the immunogenicity of ΔLLO-antigen fusions compares favorably with other accepted vaccine approaches.

### EXAMPLE 5: ActA-ANTIGEN AND PEST-ANTIGEN FUSIONS CONFER ANTI-TUMOR IMMUNITY

### MATERIALS AND EXPERIMENTAL METHODS

### Constriction of Lm-PEST-E7, Lm-ΔPEST-E7, and Lm-E7epi (Figure 8A)

Lm-PEST-E7 is identical to Lm-LLO-E7, except that it contains only the promoter and PEST sequence of the hly gene, specifically the first 50 AA of LLO. To construct Lm-PEST-E7, the hly promoter and PEST regions were fused to the full-length E7 gene using the SOE (gene splicing by overlap extension) PCR technique. The E7 gene and the hly-PEST gene fragment were amplified from the plasmid pGG-55, which contains the first 441 AA of LLO, and spliced together by conventional PCR techniques. To create a final plasmid, pVS16.5, the hly-PEST-E7 fragment and the prfA gene were subcloned into the plasmid pAM401, which includes a chloramphenicol resistance gene for selection *in vitro,* and the resultant plasmid was used to transform XFL-7.

Lm-ΔPEST-E7 is a recombinant *Listeria* strain that is identical to Lm-LLO-E7 except that it lacks the PEST sequence. It was made essentially as described for Lm-PEST-E7, except that the episomal expression system was constructed using primers designed to remove the PEST-containing region (bp 333-387) from the hly-E7 fusion gene. Lm-E7epi is a recombinant strain that secretes E7 without the PEST region or LLO. The plasmid used to transform this strain contains a gene fragment of the hly promoter and signal sequence fused to the E7 gene. This construct differs from the original Lm-E7, which expressed a single copy of the E7 gene integrated into the chromosome. Lm-E7epi is completely isogenic to Lm- LLO-E7, Lm-PEST-E7, and Lm-ΔPEST-E7 except for the form of the E7 antigen expressed.

### Construction of Lm-actA-E7

Lm-actA-E7 is a recombinant strain of LM, comprising a plasmid that expresses the E7 protein fused to a truncated version of the actA protein. Lm-actA-E7 was generated by introducing a plasmid vector pDD-1 constructed by modifying pDP-2028 into LM. pDD-1 comprises an expression cassette expressing a copy of the 310 bp hly promoter and the hly signal sequence (ss), which drives the expression and secretion of actA-E7; 1170 bp of the actA gene that comprises 4 PEST sequences (SEQ ID No: 16) (the truncated ActA polypeptide consists of the first 390 AA of the molecule, SEQ ID No: 15); the 300 bp HPV E7* gene; the 1019 bp prfA* gene (controls expression of the virulence genes); and the CAT gene (chloramphenicol resistance gene) for selection of transformed bacteria clones. (Figure 8B).

The hly promoter (pHly) and gene fragment were PCR amplified from pGG-55 (Example 1) using the primers 5'-GGGGTCTAGACCTCCTTTGATTAGTATATTC-3' (Xba I site is underlined; SEQ ID NO: 46) and 5'-ATCTTCGCTATCTGTCGCCGCGGCGCGTGCTTCAGTTTGTTGCGC-'3 (Not I site is underlined; the first 18 nucleotides are the ActA gene overlap; SEQ ID NO: 47). The actA gene was PCR amplified from the LM 10403s wild-type genome using primer 5'-GCGCAACAAACTGAAGCAGCGGCCGCGGCGACAGATAGCGAAGAT-3' (NotI site is underlined; SEQ ID NO: 48) and primer 5'-TGTAGGTGTATCTCCATGCTCGAGAGCTAGGCGATCAATTTC-3' (XhoI site is underlined; SEQ ID NO: 49). The E7 gene was PCR amplified from pGG55 (pLLO-E7) using primer 5'-GGAATTGATCGCCTAGCTCTCGAGCATGGAGATACACCTACA-3' (XhoI site is underlined; SEQ ID NO: 50) and primer 5'-AAACGGATTTATTTAGATCCCGGGTTATGGTTTCTGAGAACA-3' (XmaI site is underlined; SEQ ID NO: 51). The prfA gene was PCR amplified from the LM 10403s wild-type genome using primer 5'-TGTTCTCAGAAACCATAACCCGGGATCTAAATAAATCCGTTT-3' (XmaI site is underlined; SEQ ID NO: 52) and primer 5'-GGGGGTCGACCAGCTCTTCTTGGTGAAG-3' (SalI site is underlined; SEQ ID NO: 53). The hly promoter was fused to the actA gene (pHly-actA) was PCR generated and amplified from purified pHly DNA and purified actA DNA using the upstream pHly primer (SEQ ID NO: 46) and downstream actA primer (SEQ ID NO: 49).

The E7 gene fused to the prfA gene (E7-prfA) was PCR generated and amplified from purified E7 DNA and purified prfA DNA using the upstream E7 primer (SEQ ID NO: 50) and downstream prfA gene primer (SEQ ID NO: 53).

The pHly-actA fusion product fused to the E7-prfA fusion product was PCR generated and amplified from purified fused pHly-actA DNA product and purified fused E7-prfA DNA product using the upstream pHly primer (SEQ ID NO: 46) and downstream prfA gene primer (SEQ ID NO: 53) and ligated into pCRII (Invitrogen, La Jolla, Calif.). Competent *E. coli* (TOP10'F, Invitrogen, La Jolla, Calif.) were transformed with pCRII-ActAE7. After lysis and isolation, the plasmid was screened by restriction analysis using BamHI (expected fragment sizes 770 bp and 6400 bp (or when the insert was reversed into the vector: 2500 bp and 4100 bp)) and BstXI (expected fragment sizes 2800 bp and 3900 bp) and also screened with PCR analysis using the upstream pHly primer (SEQ ID NO: 46) and the downstream prfA gene primer (SEQ ID NO: 53).

The pHly-ActA-E7-PrfA DNA insert was excised from pCRII by double digestion with Xba I and Sal I and ligated into pDP-2028 also digested with Xba I and Sal I. After transforming TOP10'F competent *E*. *coli* (Invitrogen, La Jolla, Calif.) with expression system pActAE7, chloramphenicol resistant clones were screened by PCR analysis using the upstream pHly primer (SEQ ID NO: 46) and the downstream PrfA gene primer (SEQ ID NO: 53). A clone carrying pHly-ActA-E7 was grown in brain heart infusion medium with 20 mcg (microgram)/ml(milliliter) chloramphenicol (Difco, Detroit, MI), and pActAE7 was isolated from the bacteria cell using a midiprep DNA purification system kit (Promega, Madison, Wis.). Penicillin-treated *Listeria* strain XFL-7 was transformed with pActAE7, and clones were selected for the retention of the *plasmid in vivo.* Clones were grown in brain heart infusion with chloramphenicol (20 mcg/ml) at 37 °C. Bacteria were frozen in aliquots at -80 °C.

### RESULTS

To compare the anti-tumor immunity induced by Lm-ActA-E7 versus Lm-LLO-E7, 2 x 10⁵ TC-1 tumor cells were implanted subcutaneously in mice and allowed to grow to a palpable size (approximately 5 millimeters [mm]). Mice were immunized i.p. with one LD₅₀ of either Lm-ActA-E7 (5 x10⁸ CFU), (crosses) Lm-LLO-E7 (10⁸ CFU) (squares) or Lm-E7 (10⁶ CFU) (circles) on days 7 and 14. By day 26, all of the animals in the Lm-LLO-E7 and Lm-ActA-E7 were tumor-free and remained so, whereas all of the naive animals (triangles) and the animals immunized with Lm-E7 grew large tumors (Figure 9). Thus, vaccination with ActA-E7 fusions causes tumor regression.

In addition, Lm-LLO-E7, Lm-PEST-E7, Lm-ΔPEST-E7, and Lm-E7epi were compared for their ability to cause regression of E7-expressing tumors. S.c. TC-1 tumors were established on the left flank of 40 C57BL/6 mice. After tumors had reached 4-5 mm, mice were divided into 5 groups of 8 mice. Each groups was treated with 1 of 4 recombinant LM vaccines, and 1 group was left untreated. Lm-LLO-E7 and Lm-PEST-E7 induced regression of established tumors in 5/8 and 3/8 cases, respectively. There was no statistical difference between the average tumor size of mice treated with Lm-PEST-E7 or Lm-LLO-E7 at any time point. However, the vaccines that expressed E7 without the PEST sequences, Lm-ΔPEST-E7 and Lm-E7epi, failed to cause tumor regression in all mice except one (Figure 8C). This was representative of two experiments, wherein a statistically significant difference in mean tumor sizes at day 28 was observed between tumors treated with Lm-LLO-E7 or Lm-PEST-E7 and those treated with Lm-E7epi or Lm-ΔPEST-E7; P < 0.001, Student's t test; Figure 8D). In addition, increased percentages of tetramer-positive splenocytes were seen reproducibly over three experiments in the spleens of mice vaccinated with PEST-containing vaccines (Figure 8E). Thus, vaccination with PEST-E7 fusions causes tumor regression.

### EXAMPLE 6: FUSION OF E7 TO LLO, ActA, OR A PEST-LIKE SEQUENCE ENHANCES ANTIGEN-SPECIFIC IMMUNITY AND GENERATES TUMOR-INFILTRATING E7-SPECIFIC CD8⁺ CELLS

### MATERIALS AND EXPERIMENTAL METHODS

500 mcl (microliter) of MATRIGEL®, comprising 100 mcl of 2 x 10⁵ TC-1 tumor cells in phosphate buffered saline (PBS) plus 400 mcl of MATRIGEL® (BD Biosciences, Franklin Lakes, N.J.) were implanted subcutaneously on the left flank of 12 C57BL/6 mice (n=3). Mice were immunized intraperitoneally on day 7, 14 and 21, and spleens and tumors were harvested on day 28. Tumor MATRIGELs were removed from the mice and incubated at 4 °C overnight in tubes containing 2 milliliters (ml) of RP 10 medium on ice. Tumors were minced with forceps, cut into 2 mm blocks, and incubated at 37 °C for 1 hour with 3 ml of enzyme mixture (0.2 mg/ml collagenase-P, 1 mg/ml DNAse-1 in PBS). The tissue suspension was filtered through nylon mesh and washed with 5% fetal bovine serum + 0.05% of NaN₃ in PBS for tetramer and IFN-gamma staining.

Splenocytes and tumor cells were incubated with 1 micromole (mcm) E7 peptide for 5 hours in the presence of brefeldin A at 10⁷ cells/ml. Cells were washed twice and incubated in 50 mcl of anti-mouse Fc receptor supernatant (2.4 G2) for 1 hour or overnight at 4 °C. Cells were stained for surface molecules CD8 and CD62L, permeabilized, fixed using the permeabilization kit Golgi-stop® or Golgi-Plug® (Pharmingen, San Diego, Calif.), and stained for IFN-gamma. 500,000 events were acquired using two-laser flow cytometer FACSCalibur and analyzed using Cellquest Software (Becton Dickinson, Franklin Lakes, NJ). Percentages of IFN-gamma secreting cells within the activated (CD62L^{low}) CD8⁺ T cells were calculated.

For tetramer staining, H-2D^{b} tetramer was loaded with phycoerythrin (PE)-conjugated E7 peptide (RAHYNIVTF, SEQ ID NO: 24), stained at rt for 1 hour, and stained with anti-allophycocyanin (APC) conjugated MEL-14 (CD62L) and FITC-conjugated CD8 at 4 °C for 30 min. Cells were analyzed comparing tetramer⁺CD8⁺ CD62L^{low} cells in the spleen and in the tumor.

### RESULTS

To analyze the ability of Lm-ActA-E7 to enhance antigen specific immunity, mice were implanted with TC-1 tumor cells and immunized with either Lm-LLO-E7 (1 x 10⁷ CFU), Lm-E7 (1 x 10⁶ CFU), or Lm-ActA-E7 (2 x 10⁸ CFU), or were untreated (naïve). Tumors of mice from the Lm-LLO-E7 and Lm-ActA-E7 groups contained a higher percentage of IFN-gamma-secreting CD8⁺ T cells (Figure 10A) and tetramer-specific CD8⁺ cells (Figure 10B) than in Lm-E7 or naive mice.

In another experiment, tumor-bearing mice were administered Lm-LLO-E7, Lm-PEST-E7, Lm-ΔPEST-E7, or Lm-E7epi, and levels of E7-specific lymphocytes within the tumor were measured. Mice were treated on days 7 and 14 with 0.1 LD₅₀ of the four vaccines. Tumors were harvested on day 21 and stained with antibodies to CD62L, CD8, and with the E7/Db tetramer. An increased percentage of tetramer-positive lymphocytes within the tumor were seen in mice vaccinated with Lm-LLO-E7 and Lm-PEST-E7 (Figure 11A). This result was reproducible over three experiments (Figure 11B).

Thus, Lm-LLO-E7, Lm-ActA-E7, and Lm-PEST-E7 are each efficacious at induction of tumor-infiltrating CD8⁺ T cells and tumor regression.

### EXAMPLE 7: CREATION AND VERIFICATION OF LISTERIA-LLO-PSA CONSTRUCTS

### MATERIALS AND EXPERIMENTAL METHODS

Oligos and pCDNA3.1 were purchased from Invitrogen (Carlsbad, CA) and DNA sequencing was performed by Genewiz Inc (Plainfield, NJ). Peptides were synthesized by EZbiolabs (Westfield, IN). Flow cytometry reagents were purchased from Becton Dickinson (BD) Biosciences (San Diego, CA). Cell culture media and supplements were from Gibco/Invitrogen. ELISpot antibodies were purchased from Mabtech AB (Cincinnati, OH). Other reagents, unless indicated, were from Sigma (St. Louis, MO). PSA/vaccinia was kindly provided by Dr. Schlom at National Cancer institute, Bethesda, MD.

### Mice, cell lines and media

C57BL/6 mice were purchased from Jackson Laboratories (Bar Harbor, ME). Mice were maintained at Cook Campus animal facility at Rutgers University, New Brunswick, NJ. Experiments on mice were performed in accordance with regulations by the Institutional Animal Care and Use Committee of Rutgers University. All cell lines were purchased from ATCC (Manassas, VA). TRAMPC-1 (Transgenic Adenocarcinoma of the Mouse Prostate) cell line, derived from a C57BL/6 mouse, has been previously described. Cells were grown and maintained in Dulbecco's modified Eagle's medium with 4 mM L-glutamine adjusted to contain 1.5 g/L sodium bicarbonate and 4.5 g/L glucose supplemented with 5 µg/ml bovine insulin, 10 nM dehydroisoandrosterone, 5% fetal bovine serum and 5%Nu-Serum IV. MC57G fibrosarcoma cells (from C57BL/6 background) were maintained in Eagle's minimum essential medium (EMEM) with 2 mM L-glutamine and Earle's BSS adjusted to contain 1.5 g/L sodium bicarbonate, 0.1 mM non-essential amino acids, and 1.0 mM sodium pyruvate and 10 % fetal bovine serum. EL4 lymphoma cells were maintained in Dulbecco's modified Eagle's medium with 4 mM L-glutamine adjusted to contain 1.5 g/L sodium bicarbonate and 4.5 g/L glucose, 10 % fetal bovine serum. J774A.1, a murine macrophage cell line was maintained in RPMI1640 medium with 4 mM L-glutamine adjusted to contain 1.5 g/L sodium bicarbonate and 4.5 g/L glucose and 10% fetal bovine serum. Complete RPMI (C-RPMI) medium contained RPMI 1640 complemented with 2 mM glutamine, 0.1 mM non-essential amino acids, and 1.0 mM sodium pyruvate and 10% fetal bovine serum.

### Construction of PSA/pCDNA3.1 vaccine

The entire open reading frame of PSA including its signal sequence was cloned into pCDNA3.1⁽⁻⁾ vector backbone (Invitrogen) at XbaI and XhoI sites. In this plasmid, expression of PSA was under the control of CMV promoter and was confirmed by transient transfection into 293 cell line (ATCC). Secretion of PSA by transfected cells was confirmed in the supernatant of cultured cells using an anti-PSA ELISA kit (American Qualex, San Clemente, CA). Plasmid GM-CSF/pCDNA was a kind gift from Dr. Vonderheide, University of Pennsylvania, Philadelphia, PA.

### Construction of Lm-LLO-PSA vaccine

The gene encoding for human PSA (excluding its secretion signal sequence) was amplified using *pfx* polymerase, forward oligo: gtgCTCGAGattgtgggaggctgggagtg (SEQ ID NO: 58), and the reverse oligo: gatACTAGTttaggggttggccacgatgg (SEQ ID NO: 59). The two restriction sites, XhoI and SpeI that were used for in frame cloning of PSA as a fusion to LLO in pGG55 are indicated in capital letters. The stop codon to terminate the translation of LLO-PSA fusion protein is underlined in the reverse oligo. Amplified PSA was cloned into the *Lm* plasmid pGG55, as a fusion protein to the first 441 amino acids at the N-terminal of LLO (pAdv34, Figure 12), between the restriction sites XhoI and SpeI. The resulting plasmid was sequenced to assure the absence of mutations. The plasmid pAdv34 was electroporated into *Lm* strain XFL7 (which lacks the genomic *prfA* gene) and was originally derived from the streptomycin resistant strain *Lm* 10403s. *Listeriae* containing the plasmid (Lm-LLO-PSA) were selected using chloramphenicol (34 µg/ml) and 250µg/ml of Streptomycin on Brain Heart Infusion (BHI/Chl/S) plates. Colonies were screened by PCR to confirm the presence of the PSA gene.

### Subcloning of LLO-PSA

A truncated PSA open reading frame (GenBank Accession Number NM_001648), lacking its secretory signal sequence, the first 24 AA, was amplified using the primers: Adv60-PSA(XhoI-no ATG)F: gtgCTCGAGattgtgggaggctgggagtg (SEQ ID No: 58) and Adv61-PSA(SpeI-Stop)R: gatACTAGTttaggggttggccacgatgg (SEQ ID No: 59) and was subcloned in-frame with the first 441 amino acids of LLO (Figure 12). The plasmid backbone, pGG55 (Example 1) also has a copy of the *Listeria* virulence gene prfA, and two chloramphenicol acetyl-transferase genes that render chloramphenicol resistance in both Gram-positive and Gram-negative bacterial strains. The AA sequence of LLO-PSA is as follows:

There is one AA difference between this PSA and the sequence in NM_001648, at position N 221 Y). pGG55-LLO-PSA was electroporated into *L. monocytogenes* XFL-7 (Example 1).

### Growth and storage of bacterial vaccine strains

Recombinant *Listeria*-PSA was grown in an animal product-free medium (Modified Terrific Broth), in the presence of 34 µg/ml chloramphenicol and 250 µg/ml streptomycin at 37°C in a shaker incubator. After reaching an optical density (OD₆₀₀) of 0.5, which indicated a logarithmic growth phase, bacteria were collected by centrifugation, and the pellet was washed two times in phosphate-buffered saline (PBS) and resuspended in PBS containing 2% glycerol, then aliquoted and stored at -80°C. One aliquot was thawed 1 day later and titrated to determine bacterial titer (Colony Forming Units/ml). *Listeria* vaccines stored in this manner are stable for up to 1 year. These aliquots were then thawed, diluted at 1 x 10⁷ CFU/dose and used for the immunogenicity studies as follows.

### Expression of LLO-PSA by Lm-LLO-PSA

*Lm*-LLO-PSA colonies were grown in BHI/Chl/S broth at 37°C and 225 rpm for 8 hours. Bacteria were separated from the culture medium by centrifugation at 10,000g for 5 min and the supernatants recovered. Proteins in the culture supernatant were precipitated for at least 1 hour in the presence of 10% v/v of tri-chloroacetic acid at 4°C and separated on a 4-20% gel by SDS-PAGE. After transferring the proteins onto PVDF membranes, LLO-PSA fusion protein was detected by immunoblotting with either rabbit polyclonal anti-LLO (made in house) or anti-PSA antibodies (Sigma). Signals were developed using the Western-Breeze chromogenic Kit (Invitrogen).

### In vivo passaging of Lm-LLO-PSA.

*Lm*-LLO-PSA was passaged *in vivo* two times. Briefly, a dose of 1 x 10⁶ CFUs of *Lm*-LLO-PSA was injected intraperitoneally (i.p.) to 6-8 week old mice. Spleens were harvested 3 days after the injection; single cell suspensions were prepared from the spleens and plated on BHI/Chl/S plates to select for *Lm*-LLO-PSA growth. This procedure was repeated one more time using a LLO-PSA positive colony from the first passage. *Lm*-LLO-PSA colonies recovered after two *in vivo* passages were tested for LLO-PSA secretion. A single clone was chosen to be used for plasmid DNA sequencing, tumor studies and other functional assays.

### In vivo virulence study

A virulence study was performed to determine a safe dose of *Lm*-LLO-PSA in mice. Two groups of 4 male, C57BL/6 mice (7 weeks old) were injected i.p. with two different doses of Lm-PSA: 1 x 10⁸ and 5 x 10⁷ CFUs/dose. Mice were monitored for signs of sickness for up to 5 days post inoculation.

### Plasmid sequencing

*Lm*-LLO-PSA was grown overnight in 200 ml of BHI/Chl/S at 37°C and 225 rpm. Bacteria were separated from culture broth by centrifugation at 10,000g *at* 4°C for 10 min. Bacterial pellet was treated with lysozyme (2 mg/ml) at 37°C for 30 min to break the cell wall. The plasmid pAdv34 was purified using PureLink plasmid midiprep kit (Invitrogen) according to manufacturer's instructions and entirely sequenced by the dideoxi chain terminator method.

### RESULTS

A *Listeria* strain was created that expresses a non-hemolytic LLO fused to a truncated PSA (kallikrein-related peptidase 3). The resulting recombinant *Listeria* strain secretes a protein of the predicted size for LLO-PSA (75 Kd), which is detected by both anti-LLO and anti-PSA antibodies, showing that LLO-PSA protein was expressed and secreted (Figure 13).

**Generation of recombinant *L. monocytogenes* expressing PSA.** The gene encoding for human PSA was cloned as a fusion protein with a truncated, non-hemolytic segment of LLO, which contains a conserved PEST sequence at its N terminal (Figure 12). The plasmid backbone pGG55 as previously described, allows for the expression and secretion of LLO fusion proteins from an episomal origin by the attenuated *Lm* strain XFL7, which lacks the genomic virulence factor *prfA.* The *prfA* gene is required for the *in vivo* survival of *Lm* and thus, is complemented by a copy carried on the plasmid. The PSA signal sequence was deleted to avoid any intervention of this sequence with the proper secretion of the LLO-PSA fusion protein, which is mediated by the LLO secretion signal sequence in Lm-LLO-PSA.

***Lm*-LLO-PSA can express and secrete LLO-PSA fusion protein.** Expression and secretion of the LLO-PSA fusion protein from *Lm* was tested in the cell culture supernatants after *in vitro* growth of the bacteria. We tested four colonies of *Lm*-LLO-PSA and all were able to secrete the fusion protein LLO-PSA as detected with both anti-LLO and anti-PSA antibodies (Figure 13). This secretion was maintained in bacteria isolated after two *in vivo* passages in mice (data not shown), suggesting that the plasmid is stable and retained *in vivo* for at least three days. In order to confirm stability, pAdv34 was extracted from the passaged *Lm*-LLO-PSA and was entirely sequenced. There were no mutations detected in the plasmid backbone or the PSA gene after two *in vivo* passages, as compared to our reference plasmid pGG55 and the human PSA gene sequence (NCBI: NM_001648). To determine a safe dose of *Lm*-LLO-PSA to be used for the animal studies, two doses of this construct were tested in mice by i.p. injection (1 x 10⁸ and 5 x 10⁷ CFUs/dose). The dose used in the animal experiments (1x10⁷ CFU/mouse) was defined as one-tenth of the lowest dose observed to have deleterious effect on the immunized mice.

To test the *in vitro* stability of Lm-PSA, the strain was grown and passaged for 7 consecutive days in modified terrific broth. After this time, the bacteria retained the plasmid, the plasmid contained the PSA gene and there were no deletions or re-arrangements in the plasmid, indicating plasmid stability (Figure 22).

To test the *in vivo* stability of Lm-PSA, the strain was passaged twice through mice. The plasmid was then sequenced by Genewiz™ and found to have the following sequence:

### EXAMPLE 8: LISTERIA-LLO-PSA CONSTRUCTS ELICIT ANTIGEN-SPECIFIC CYTOTOXIC T LYMPHOCYTES AND PROVIDE TUMOR PROTECTION-I

### MATERIALS AND EXPERIMENTAL METHODS

### CTL assays

Male C57BL/6 mice were immunized i.p. with either 0.1 LD50 of Lm-PSA or 0.1 LD50 of Lm-HPV16E7E6TM and boosted 1 time after 2 weeks. Spleens were harvested 6 days after the boost. Isolated splenocytes were prepared and stimulated for 5 days with mitomycin-treated, PSA-vaccinia infected, MC57G cells as feeders. In the first experiment, a CTL assay was performed using PSA H2Db peptide (1 µM, HCIRNKSVIL; SEQ ID No: 60)-pulsed EL4 cells as targets labeled with 100 µM of europium (Sigma), using the following E:T ratios: 25:1, 8:1, 2.8:1, 0.9:1, 0.3:1, 0.1:1 and 0.03:1. After a 4-hour incubation of mixed targets and effectors, cells were separated from the culture supernatant by centrifugation. Released europium from lysed cells in the supernatant was determined as follows: 10 µl of the supernatant was added to 100 µl Europium enhancement solution (Delfia). Absorbance was read at 590 nm using Victor II spectrophotometer (Perkin Elmer). Maximum release of Europium was determined from the supernatant of labeled target cells with 1% triton X-100 and the spontaneous release was determined from the target cells incubated in the absence of effector cells. In the second experiment, E:T ratio was kept constant at 25:1, and the peptide concentrations was varied as indicated. Percent specific lysis was determined as [(experimental release - spontaneous release)/(maximum release - spontaneous release)] x 100.

### Cytokine secretion assays

Male C57BL/6 mice were immunized with either Lm-PSA or *Listeria* expressing different fragments of Wilm's tumor antigen (negative control) or left un-immunized. Mice were boosted once after two weeks and the spleens were harvested 6 days after the boost. Isolated splenocytes were prepared and stimulated *in vitro* overnight in the presence of 1 µM PSA H2Db peptide. IFN-γ secretion by isolated splenocytes was determined by ELISpot assay.

### Cell Culture, Materials, and Reagents

TRAMP-C1 mouse prostate adenocarcinoma cells derived from a C57BL/6 mouse prostate tumor was purchased from ATCC. This cell line is negative for PSA expression. Cells were maintained in Dulbecco's modified Eagle's medium with 4 mM L-glutamine adjusted to contain 1.5 g/L sodium bicarbonate and 4.5 g/L glucose supplemented with 0.005 mg/ml bovine insulin and 10 nM dehydroisoandrosterone, 90%; fetal bovine serum, 5%; Nu-Serum IV, 5%. The gene encoding the full-length human PSA protein, including its signal sequence, was subcloned into a pUV6/v5 plasmid (Invitrogen). After confirmation of the correct sequence, the plasmid was linearized and transfected into TRAMP-C1 cells using Lipofectamine 2000™ (Invitrogen). Positive clones were selected in the presence of 10 µg/ml blasticidin. Several stably expressing PSA clones were isolated and tested for the secretion of human PSA into the cell culture medium.

### Subcutaneous tumor inoculation

Two different clones of PSA-expressing TRAMP-C1 cells were resuspended at 5 x 10⁶ cells per 200 mcl dose. Male C57BL/6 mice (8 per group, 6-8 weeks old) were inoculated s.c. in the left flank.

### Tumor regression studies

Seven days after tumor inoculation, mice are immunized with either 0.1 LD₅₀ of Lm-PSA (10⁷ CFU), 0.1 LD₅₀ of Lm-HPV16E7, or PBS. Two boosts are administered on days 15 and 25 post-tumor inoculation. Tumors are monitored for 90 days. Tumor size is defined as the mean of two perpendicular diameters.

### Orthotopic Injection of Prostate Tumor Cells

Six-week-old male C57BL/6 mice are anesthetized with 2% isoflurane. In a sterile field, a lower midline incision is made to access the prostate. The left lobe of the dorsal prostate is injected with 1 × 10⁵ TRAMPC-1/PSA tumor cells from a single-cell suspension in PBS, using a 27-gauge needle fitted on a 50-µl Hamilton syringe. Mice are sutured, and sutures are removed 10 days after surgery. Seven days later, mice are immunized i.v. with Lm-PSA, LmHPV16E7 or PBS. Mice are sacrificed at different time points, prostates are removed surgically and weighed for determination of the tumor growth.

### Tumor protection studies

C57BL/6 mice are immunized and boosted with Lm-PSA, LmHPV16E7, or PBS, as described in the previous Example. Seven days after the boost, mice are injected s.c. with 5 x 10⁶ TRAMPC-1/PSA tumor cells. Growth of the tumors is monitored by measuring with a caliper for 90 days.

### Inhibition of prostate cancer metastases

For orthotopic tumor inoculation, 8-10 week old C57BL/6 male mice (Jackson labs) are anesthetized with isoflurane. A low abdominal skin incision cranial to the prepucial glands is made, and the seminal vesicles are carefully exteriorized to expose the dorso-lateral prostate. Using a 29 gauge insulin syringe, 5 × 10⁵ TRAMPC-1/PSA cells suspended in PBS are injected into the dorso-lateral prostate in a 20 L volume. The seminal vesicles and prostate are held for one minute to allow the injected cells to settle into the gland and then gently replaced into the abdominal cavity. Body wall and skin wounds closed are closed with 5-0 PDS and 5-0 nylon, respectively.

Tumors are allowed to develop for 50 days. The primary tumor is removed during necropsy and fixed in formalin, and then paraffin embedded, sectioned and stained with H&E. Enlarged lymph nodes from the paralumbar region are visualized under surgical microscopy and then dissected out, fixed, embedded, and histologically analyzed for prostate cancer cells.

### Tissue immunostaining

Formalin-fixed prostate tumor tissues are paraffin embedded, sectioned, applied to Plus slides™ (VWR Corp), and then stained using a Dako autostainer system. Slides are pre-treated with 3.0% hydrogen peroxide for 10 minutes, then rinsed and treated with a 10 µg/mL solution of proteinase K solution for 3 minutes to enhance antigen retrieval. Non-specific binding sites are blocked by addition of normal goat serum for 30 minutes, and then a 10 µg/mL solution of rabbit anti-human PSA antibody (Sigma) or rabbit anti-human Proliferating Cell Nuclear Antigen (AB15497, AbCam antibodies) is applied to the tissue for 30 minutes. Primary antibody is removed by washing, and appropriate horseradish peroxidase-labeled secondary antibody is applied for a 30-minute period and detected using NovaRed™ substrate (Vector Labs, Burlingame, CA) in an 8-minute incubation. Slides are counter-stained with hematoxylin before drying.

Cells from slides of primary and lymph node sections are scored as either positive or negative for human PSA. Four regions of each slide were randomly selected, and 20 cells from each region are scored. PSA staining in tumors is compared to lymph node metastases from the same mouse.

### Listeria strains

*Listeria* vaccines are prepared and stored as described in the previous Example.

### RESULTS

To test the immunogenicity of LLO-PSA, 6-8 weeks old C57BL/6 mice (Jackson Laboratories) were immunized i.p. with either Lm-PSA (0.1 LD₅₀, 1 x 10⁷ CFU/dose) or Lm-HPV16E7E6TM (negative control, 0.1 LD₅₀, 1 x 10⁶ CFU/dose) or left un-immunized. Splenocytes from vaccinated mice were tested for ability to recognize and lyse PSA peptide presenting cells *in vitro* in a CTL assay. Splenocytes from the immunized mice were able to recognize and lyse PSA-peptide pulsed tumor cells with high efficiency (Figure 20A). Further, the response was dose-dependent with regard to the amount of antigen presented by the target cells (Figure 20B).

In additional assays, mice were immunized with Lm-PSA or strains expressing fragments of Wilm's tumor antigen (negative control), and cytokine secretion was determined, in response to incubation with the PSA peptide. Splenocytes from the vaccinated mice exhibited high levels of IFN-γ secretion (Figure 18).

Thus, PSA-expressing LM strains and LLO-PSA fusions are efficacious in the induction of antigen-specific CTL that are capable of target cell lysis and IFN-γ secretion. Accordingly, PSA-expressing LM strains and LLO-PSA fusions are efficacious in therapeutic and prophylactic vaccination against PSA-expressing prostate cancer.

*Listeria* vaccines described in the previous Example are used in tumor protection experiments in an orthotopic prostate carcinoma animal model. Mice are immunized with either Lm-PSA, LmHPV16E7, or PBS, then injected with TRAMPC-1 Lm-PSA protects mice from tumor formation.

In additional experiments, mice are first injected with TRAMPC-1/PSAprostate cancer cells, vaccinated with Lm-PSA, LmHPV16E7, or PBS 4 days later, and boosted with the same vaccine. Lm-PSA impedes growth of prostate metastases.

Thus, PSA-producing LM strains and LLO-PSA fusions induce tumor protection.

### EXAMPLE 9: LISTERIA-LLO-PSA CONSTRUCTS ELICIT ANTIGEN-SPECIFIC CYTOTOXIC T LYMPHOCYTES AND PROVIDE TUMOR PROTECTION-II

### MATERIALS AND EXPERIMENTAL METHODS

***In** vitro* **macrophage invasion study.** Murine macrophage-like J774A.1 cells were plated at 1 x 10⁶ cells per well of 24 well plates. The day after, cells were infected at MOI of 1:1 with different *Lm* strains for 1 hour in the absence of antibiotics. Extracellular bacteria were eliminated by washing and incubating the cells in medium containing 50µg/ml of gentamicin for 30 min at 37°C and 5% CO₂. The intracellular bacterial growth was monitored by taking samples at different time points for up to 8 hours followed by titration of the bacteria on BHI plates after lysing the cells with dH₂O.

**Anti-tumor efficacy.** TRAMPC-1 is a murine adenocarcinoma prostate tumor cell line on the C57BL/6 mouse background and was used to construct a PSA secreting prostate tumor model for testing the efficacy of *Lm*-LLO-PSA as a vaccine. The complete human PSA open reading frame, including its secretory signal sequence at the N-terminal of the molecule was cloned under the control of the UbC promoter in the plasmid pUB6/V5 (Invitrogen). The resulting plasmid (pAdv63) was sequenced for the confirmation of the correct PSA gene sequence and then transfected into the TRAMPC-1 cell line using lipofectamine 2000 (Invitrogen). Transfected cells were selected by culturing the cells in medium containing 10 µg/ml of blasticidin (Invitrogen). Single clones were isolated by the limiting dilution method and were screened for the secretion of PSA into the extracellular culture medium using an anti-human PSA ELISA kit. Several positive clones were obtained, expanded and stored in liquid nitrogen. One clone (T-PSA23) was selected for further studies.

**Tumor regression study.** Three groups of 8 male C57BL/6 mice (6-8 week old) were inoculated s.c. in the right flank with 5 x 10⁶ T-PSA23 cells. Seven days later, when tumors reached an average diameter of 4-5 mm, they were immunized i.p. with *Lm*-LLO-PSA (1 x 10⁷ CFUs/dose), *Lm*-LLO-E7 (1 x 10⁸ CFUs/dose) or were not treated. *Lm*-LLO-E7 previously described by Gunn et al., was constructed in the same manner as *Lm*-LLO-PSA, but instead of PSA, it expressed HPV16E7 and was used in this experiment as an irrelevant *Lm* control. Immunizations were repeated twice with 1 week intervals. Tumors were monitored weekly for seven weeks. Blood samples from all experimental animals were collected on day 40 post-tumor inoculation from the tail vein and the presence of PSA protein in the sera was tested using the PSA ELISA kit. For comparison studies, tumor cells were injected as described above. Mice were immunized twice with *Lm*-LLO-PSA (1 x 10⁷ CFUs/dose in 200 µl PBS), a recombinant PSA-vaccinia (1 x 10⁷ PFUs/dose in 200 µl PBS, i.p.) or a pDNA vaccine comprising of a mixture of PSA/pCDNA3.1 (100 µg) with GM-CSF/pCDNA3.1 (100 µg) in 50 µl volume, by intramuscular injection in the left quadriceps. Tumors were monitored once a week using an electronic caliper and tumor sizes were expressed as the mean of two perpendicular diameters.

### Immunogenicity Studies in mice

**ELISpot assay:** Groups of 4-5 male C57BL/6 mice (6-8-weeks old) were immunized three times with either *Lm*-LLO-PSA or a similar construct containing an irrelevant antigen as negative control *(Lm-*LLO-WT1), or were not immunized (naive). Six days after the boost, spleens were harvested and processed to a single cell suspension. Red blood cells were lysed by 2 min incubation with ACK lysing solution followed by two washes with C-RPMI medium. Isolated splenocytes were plated at 2 x 10⁵ cells/ well in the presence of 1 µM PSA₆₅₋₇₄ peptide (H2D^{b}-restricted PSA epitope HCIRNKSVIL) on ELISpot plates previously coated with mouse IFN-γ antibody (mAb AN18). Plates were incubated overnight at 37°C. Spot forming cells (SFC) were detected according to manufacture's instructions (Mabtech AB). Spots were counted using a dissecting microscope.

**Intracellular staining for IFN-γ.** Splenocytes isolated from the immunized, control or naïve mice were stimulated at 2x10⁶ cells/well for 5 h in round bottom 96-well plates with 1µM of either PSA₆₅₋₇₄ peptide or control peptides in the presence of IL-2 (50U/ml) and monensin A (StopGolgi, BD Biosciences). Cells incubated in the medium without peptide were used as negative control. After incubation, cells were stained with anti-mouse CD8-FITC, CD3-PerCP-Cy5.5 and CD62L-APC antibodies. Subsequently, cells were permeabilized and stained with anti-mouse IFNγ-PE antibody and analyzed in a FACS Calibur (BD Biosciences) cytometer. The data were analyzed using CellQuest Pro software. Cells were gated as CD3⁺CD8⁺CD62L^{low} and the number of IFN-γ-positive cells expressed as a percentage of total gated cells.

**Cytotoxicity assay.** The cytotoxic activity of T cells in response to *Lm*-LLO-PSA vaccination was measured by a PSA specific CTL assay. Groups of 4 male C57BL/6 mice were injected i.p. with three doses of *Lm*-LLO-PSA (1 x 10⁷ CFUs) or a *Listeria* expressing an irrelevant antigen (HPV16E7E6), with one week intervals. Naive group did not receive any immunization. Spleens of the immunized and naïve mice were harvested 6 days after the last boost. Splenocytes were incubated for 5 days in C-RPMI medium containing 20U/ml of mouse IL-2 (Sigma), in the presence of MC57G cells infected with PSA/vaccinia (MOI 5 for 4 hours) and treated with mitomycin C at an effector: stimulator ratio of 1: 20. The cytotoxicity assay was performed for 4 hours by incubating the effector cells with EL4 target cells, pulsed for 1 hour with the H2D^{b}, PSA₆₅₋₇₄ peptide and labeled with europium. Europium released from the lysed cells was measured by mixing a sample of the cell culture supernatant with europium enhancement solution (Delfia/Perkin Elmer, Waltham, MA) and an absorbance reading at 590 nm (Perkin Elmer/Wallac, VictorII). Two sets of assays were performed: a) using different effector: target (E:T) ratios at a fixed peptide concentration of 1 µM; and b) using different concentrations of the PSA peptide at a fixed E:T ratio of 25:1. SC₅₀ values were calculated as the peptide concentration required for 50% of the value of maximum peptide concentration used (1 µM) minus the background level at 0 µM.

**Analysis of tumor infiltrating lymphocytes (TILs):** T-PSA23 cells embedded in matrigel were inoculated s.c. in the right flank of male C57BL/6 mice, which were immunized on days 7 and 14 with *Lm-*LLO-PSA, a control *Lm* vaccine or left untreated. On day 20, the tumors were surgically excised, washed in ice-cold PBS and minced with a scalpel. The tumor was incubated in matrigel cell recovery solution (BD Biosciences) for 2 hours on ice. TILs were further released by mechanical disruption of the tissue in a cell strainer using a syringe plunge. Isolated cells were stained with a PSA₆₅₋₇₄ H-2D^{b} tetramer-PE and anti-mouse CD8-FITC, CD3-PerCP-Cy5.5 and CD62L-APC antibodies to characterize CTLs specific for the PSA epitope. To analyze regulatory T cells in the tumor, TILs were also stained with CD4-FITC, CD3-PerCP-Cy5.5 and CD25-APC. Cells were subsequently permeabilized and stained with anti-FoxP3-PE antibody (Milteny Biotec). Cells were analyzed in a FACS Calibur (BD Biosciences) cytometer. The data were analyzed using CellQuest Pro software. Regulatory T cells were defined as CD3⁺CD4⁺CD25⁺Fop3⁺ cells.

Statistical analysis: Non-parametric Mann-Whitney and Kruskal Wallis tests were applied to compare tumor sizes among different treatment groups. Tumor sizes were compared on day 40 for statistical analysis, because this was the latest time-point with the highest number of mice in each group. The student's *t*-test was used to compare the means in the ELISpot experiments. A p-value of less than 0.05 was considered statistically significant in these analyzes.

### RESULTS

***Lm*-LLO-PSA is able to infect macrophages** *in **vitro.*** Uptake by antigen presenting cells such as dendritic cells and macrophages is an essential requirement for *Lm* based vaccines to successfully deliver and present antigens into the immune system. This property can be tested *in vitro* by infecting the murine macrophage-like cell line J774A.1 and quantifying the intracellular bacterial growth, which is an indication that the *Lm* vaccine strain has been able to properly enter the cells and multiply. In this assay, *Lm*-LLO-PSA was shown to be able to invade macrophages and grow in a similar manner to the wild type *Lm* strain 10403s. In contrast, the *Lm* strain XFL7, which lacks *prfA* and does not escape the phagolysosome, is not able to proliferate in J774A.1 cells and its titer remains constant several hours post-infection (Figure 14). The complementation of *prfA* in *Lm* strain XFL7 with the plasmid pAdv34 resulted in the restoration of the *in vitro* invasive properties of the strain and this was comparable to those of the wild type strain.

***Lm*-LLO-PSA causes regression of pre-established PSA expressing tumors in mice.** The efficacy of *Lm*-LLO-PSA in regressing PSA expressing tumors was tested in a mouse model using a PSA-transfected TRAMPC-1 cell line. TrampC-1 cells, originally derived from a murine prostate adenocarcinoma tumor, were stably transfected with the human PSA gene under the control of the human Ubiquitin C promoter. Secretion of PSA by transfected cells was confirmed by ELISA. Four clones were expanded and passaged for 25 generations in the absence of the antibiotic selection marker, blasticidin. Cell culture supernatants were tested for the levels of PSA secretion which were shown to be comparable between the cells at early passage and passage 25 (data not shown). One of these clones retained tumorgenicity *in vivo* and formed solid tumors upon s.c. inoculation in male C57BL/6 mice. An early passage of this clone was used for the tumor studies and it is referred as T-PSA23. Three groups of 8 mice were inoculated s.c. with 5 x 10⁶ T-PSA23 cells and were monitored for tumor growth daily. After 7 days, a solid tumor mass of 4-5 mm in diameter was detected in 7 of 8 mice in each group (Figure 15). At this time mice were immunized with *Lm*-LLO-PSA (1 x 10⁷ CFUs/mouse), Lm-LLO-E7 (1x 10⁸ CFUs/mouse) or left untreated. Primary immunization was followed by two booster doses at 1 week intervals with the same dose as the prime. Mice (7 of 8) in each of the control groups developed slow growing tumors which reached a diameter of 2 cm within 7 weeks post-inoculation (Figure 15), at which point they were sacrificed. One mouse in each group did not show any signs of tumor at any time. Five days after the first immunization, tumor reduction was observed in 7 of 8 of *Lm*-LLO-PSA immunized group and complete tumor regression was observed in 5 of these mice after 1 week. One of the tumors in the *Lm*-LLO-PSA immunized group remained stable until the third immunization. However, one week after the third vaccination, this tumor also regressed and became undetectable. Another mouse in this group developed a slow growing tumor which reached a size of 18-20 mm within 7 weeks and was sacrificed. At the end of the study on week 8 post-tumor inoculation, 7 of 8 mice in the *Lm*-LLO-PSA immunized group were still tumor free. This experiment was repeated twice with similar results. Non-parametric Kruskal-Wallis statistical analysis was performed on the tumor sizes on day 40, before mice in naive and *Lm*-LLO-E7 groups were sacrificed. There were no statistically significant differences among the tumor sizes of the mice in the naive group and the mice immunized with Lm-LLO-E7 (p= 0.613). However, immunization with *Lm*-LLO-PSA was shown to make a significant impact on the T-PSA23 tumor growth (p=0.002). Serum samples from controls and *Lm*-LLO-PSA immunized mice were collected on day 40 post-tumor challenge and tested for the presence of PSA using an anti-human PSA ELISA kit (American Qualex). Significant levels of PSA (5-10 ng/ml) were detected in the serum of control mice and in the mouse in the *Lm*-LLO-PSA group which had tumors. No PSA was found in the serum of *Lm*-LLO-PSA immunized mice after tumor regression.

***Lm*-LLO-PSA elicits cellular immune responses against PSA in both spleens and tumors.** To elucidate whether vaccination with *Lm*-LLO-PSA induced PSA-specific CTLs able to infiltrate the tumor, mice were implanted s.c. with a mixture of T-PSA23 cells + matrigel and immunized with two doses of *Lm*-LLO-PSA, *Lm*-LLO-E7 or none. Spleens and tumors were harvested 6 days after the last immunization and tested for the presence of CD8⁺/PSA₆₅₋₇₄(H2D^{b} restricted) tetramer positive T cells by FACS analysis. No significant anti-PSA responses were observed in spleens of naive (0.05%) or *Lm*-LLO-E7 (0.09%) vaccinated mice (Figure 16, upper panel). However, upon immunization with *Lm*-LLO-PSA, a significant number of PSA specific CD8⁺ T cells were found in the spleens. Interestingly, when we tested the tumors excised from these mice for the presence of TILs, low number of PSA specific CD8⁺ T cells were identified in both naive and *Lm*-LLO-E7 immunized mice (1.5% and 0.51% respectively). Immunization with *Lm*-LLO-PSA caused a considerable increase in this number as 16.1% of all CD8⁺ TILs were shown to be PSA₆₅₋₇₄ specific (Figure 16 lower panel).

**Immunization with *Lm*-LLO-PSA causes a decline in the T regulatory cells in the tumors but not in spleens.** The presence of CD4⁺/CD25⁺/FoxP3⁺ T regulatory (Treg) tumor infiltrating cells have been shown to be associated with increase chance of tumor progression. Therefore, we examined the presence of this T cell population in tumors of *Lm*-LLO-PSA immunized or control mice. Interestingly, immunization with either *Lm*-LLO-PSA or Lm-LLO-E7 caused a considerable decrease in the frequency of Tregs in the tumors as compared to tumors from naive mice (Figure 17). In fact, *Lm*-LLO-PSA had slightly more impact than Lm-LLO-E7 in the frequency of tumor infiltrating Tregs. On the other hand, there were no significant differences between the percentages of CD4⁺/CD25⁺/FoxP3⁺T cells isolated from the spleens of *Lm*-LLO-PSA or control mice.

**Immunization with *Lm*-LLO-PSA results in strong cellular immune responses against PSA.** The immune responses elicited by *Lm* based vaccines have been shown to result in cellular, rather than humoral responses. We investigated the activation and functionality of CD8⁺ T cells induced by *Lm-*LLO-PSA in spleens of immunized mice.

**Cytokine secretion.** Secretion of IFN-γ by activated T cells in response to an *in vitro* stimulation with H2D^{b} PSA peptide was tested by ELISpot and intracellular cytokine staining. Mice were immunized three times with *Lm*-LLO-PSA or *Lm*-LLO-WT-1 (*Lm-*LLO-Wilm's Tumor Antigen as negative control) or were not immunized (naive). Isolated splenocytes were then prepared and incubated with the PSA peptide and the IFN-γ secreting CD8⁺ cells were quantified. In both assays, mice immunized with *Lm*-LLO-PSA showed a significantly higher number of IFN-γ secreting cells in response to peptide pulsing than the negative controls (p< 0.001). The number of IFN-γ secreting cells measured by ELISpot assay, were approximately 11-fold higher than the controls (Figure 18). When tested by intracellular cytokine staining for IFN-γ, a 30-fold increase in the number of CD8⁺CD62L^{low} IFN-γ secreting cells from *Lm*-LLO-PSA immunized mice was detected as compared to the control (4.22% for *Lm*-LLO-PSA vs. 0.15% for naïves) (Figure 19).

**Cytotoxicity (CTL) assay.** In order to determine the functional activity of the T- cells specific for PSA, splenocytes from immunized or naive mice were tested in a CTL assay against cells pulsed with the H2D^{b} PSA peptide. Using fixed peptide concentration (1 µM), splenocytes from *Lm*-LLO-PSA immunized mice showed a maximum specific lysis of 60%, which was reduced in proportion to the E:T ratio (Figure 20A). This outcome was dependent on the peptide concentration in the lysis assay with a maximum value reaching at a peptide concentration of only 0.1 µM (Figure 20B). The SC₅₀ value of the polyclonal population, which is a useful measure of average T cell avidity was approximately 1 pM. The results from this experiment also confirmed that immunization with *Lm-*LLO-PSA generated specific cytolytic T cells of high avidity capable of lysing cells that display the target antigen PSA.

**Comparison of the anti-tumor effects of *Lm*-LLO-PSA with other PSA based vaccine modalities.** Finally to further evaluate *Lm*-LLO-PSA vaccine we compared its anti-tumor efficacy with two other PSA based vaccines that have been described in the literature *i.e.* a recombinant DNA and a vaccinia based vaccine. For this, the complete open reading frame of PSA was cloned into pCDNA3.1 under the control hCMV promoter (pAdv40.1). A similar DNA vaccine was previously described by Roos *et al.* using a similar vector backbone *i.e.* pVax (Invitrogen). The difference between the two vector backbones (pCDNA3.1 and pVax) is only in their antibiotic selection marker. pAdv40.1 was injected i.m. as a mixture with GM-CSF/pCDNA, which has been used as an effective adjuvant for other DNA vaccines. Expression of PSA from PSA/pCDNA plasmid was confirmed by *in vitro* transfection into 293FT cell line (data not shown). Recombinant PSA-vaccinia has been previously described by Hudge *et al..* Mice were implanted with T-PSA23 tumor cells as described above and then immunized twice with each of the vaccines by the optimal dose and route of administration as cited in the literature. Immunization with PSA/vaccinia did not have any impact on the growth of T-PSA23 tumors as all naive and PSA/vaccinia immunized mice developed tumors which reached a size of 20 cm within 7-8 weeks and they had to be sacrificed (Figure 21b). However 1 out of 8 mice immunized with the PSA/DNA vaccine and 3 out of 8 mice vaccinated with *Lm*-LLO-PSA remained tumor free for over 90 days, at the time the study was terminated (Figure 21A and the table insert). This experiment was repeated twice showing similar results. Thus, *Lm*-LLO-PSA was proven to be the most efficacious vaccine of the three PSA-vaccine modalities in regressing established PSA expressing tumors. It has to be noted that in the comparison studies, because of the limited availability of the other two vaccines, only two doses were administered. Therefore, *Lm*-LLO-PSA was shown to be less efficacious (complete tumor regression was observed in only 3 out of 8) than when injected three times (where 7 out of 8 tumors regressed, as seen in Figure 15). This observation was confirmed in several other studies (data not shown).

### COMPARATIVE EXAMPLE 10: LISTERIA-LLO-FOLATE HYDROLASE 1 (FOLH1) AND LISTERIA-LLO-PROSTATE STEM CELL ANTIGEN (PSCA) CONSTRUCTS ELICIT ANTIGEN-SPECIFIC CYTOTOXIC T LYMPHOCYTES

### MATERIALS AND EXPERIMENTAL METHODS

### Growth and storage of bacterial vaccine strains

Recombinant *Listeria-*LLO-FOLH1 and *Listeria-*LLO-PSCA are grown and maintained as described for *Listerin*-PSA in Example 7 above.

### RESULTS

A gene encoding a truncated FOLH1, which contains the complete open reading frame of FOLH1, except for its secretion signal sequence, is fused to a gene encoding a truncated non-hemolytic fragment of Listeriolysin O, in a similar manner to that described for KLK3 in Example 7 above. Similarly, a gene encoding a truncated PSCA, which contains the complete open reading frame of PSCA, except for its secretion signal sequence, is fused to a gene encoding a truncated non-hemolytic fragment of Listeriolysin O. Each gene is cloned into *Listeria* plasmid pGG55 and electroporated into LM XFL-7. LLO-FOLH1 protein and LLO-PSCA protein are thus expressed and secreted episomally from separate recombinant Listeria strains.

To test the immunogenicity of LLO-FOLH1 and LLO-PSCA, mice re-immunized with either Lm-LLO-FOLH1, Lm-LLO-PSCA, or LmWT1A (irrelevant antigen control) or PBS (negative control), as described for LLO-KLK3 in Example 7 above. Following culture with vaccinia-PSA infected stimulator cells with for 5 days, splenocytes from the FOLH1-vaccinated mice are able to recognize and lyse FOLH1-peptide pulsed tumor cells with high efficiency in a CTL assay and splenocytes from the PSCA-vaccinated mice are able to recognize and lyse PSCA-peptide pulsed tumor cells with high efficiency in a CTL assay. In addition, the splenocytes exhibit high levels of IFN-γ secretion, in response to incubation with the FOLH1 peptide or the PSCA peptide, respectively.

Thus, FOLH1-expressing LM strains, PSCA-expressing LM strains, LLO-PSCA fusions and LLO-FOLH1 fusions are efficacious in the induction of antigen-specific CTL that are capable of target cell lysis and IFN-γ secretion. Accordingly, FOLH1-expressing LM strains, PSCA-expressing LM strains, LLO-PSCA fusions and LLO-FOLH1 fusions are efficacious in therapeutic and prophylactic vaccination against PSA-expressing prostate cancer.

### COMPARATIVE EXAMPLE 11: LISTERIA-LLO-FOLH1 CONSTRUCTS PROVIDE TUMOR PROTECTION

*Listeria* vaccines described in the previous Example are used in tumor protection experiments in the orthotopic prostate carcinoma animal model described in Examples 8 and 9 above. Mice are immunized with either Lm-FOLH1, Lm-PSCA, LmWT1A, or PBS, then injected with PC3M-LN4 or 22Rv1 cells. Lm-FOLH1 and Lm-PSCA protect mice from tumor formation.

In additional experiments, mice are first injected with PC-3M prostate cancer cells, as described for Examples 8 and 9 above, vaccinated with Lm-FOLH1, Lm-PSCA, LmWT1A, or PBS 4 days later, and boosted with the same vaccine. Lm-FOLH1 and Lm-PSCA impede prostate metastases.

Thus, FOLH1- and PSCA-producing LM strains and Lm-FOLH1 and Lm-PSCA fusions induce tumor protection.

## Claims

1. A recombinant Listeria strain expressing a fusion peptide of a kallikrein-related peptidase 3 (KLK3) peptide which is operatively linked to an N-terminal non-hemolytic listeriolysin (LLO) peptide, said fusion peptide comprising the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence.

2. The recombinant *Listeria* strain of claim 1, wherein the recombinant *Listeria* strain is an auxotrophic *Listeria* or a recombinant *Listeria monocytogenes* strain.

3. The recombinant *Listeria* strain of claim 2, wherein the auxotrophic *Listeria* strain is a dal/dat mutant or further comprises a deletion in the endogenous ActA gene.

4. The recombinant *Listeria* strain of claim 2, wherein the auxotrophic *Listeria strain* comprises an episomal expression vector comprising a metabolic enzyme that complements the auxotrophy of the auxotrophic *Listeria* strain.

5. The recombinant *Listeria* strain of claim 4 wherein the metabolic enzyme is an alanine racemase enzyme or a D-amino acid transferase enzyme.

6. The recombinant *Listeria* strain of claim 1, wherein the recombinant *Listeria* strain has been passaged through an animal host.

7. A recombinant polypeptide comprising a fusion peptide of a KLK3 peptide which is operatively linked to an N-terminal LLO peptide, wherein said recombinant polypeptide comprises the sequence of SEQ ID NO: 54 or a sequence at least 99% homologous thereto, wherein said N-terminal LLO peptide enhances the immunogenicity of the fusion peptide, and wherein the KLK3 peptide does not contain a signal sequence.

8. A nucleotide molecule encoding the recombinant polypeptide of claim 7.

9. A vaccine comprising the recombinant *Listeria* strain of any one of claims 1 to 6, the recombinant polypeptide of claim 7 or the nucleotide molecule of claim 8, and an adjuvant.

10. A recombinant vaccine vector encoding the recombinant polypeptide of claim 7 or comprising the nucleotide molecule of claim 8.

11. The recombinant *Listeria* strain of any one of claims 1 to 6, an immunogenic composition comprising the recombinant polypeptide of claim 7, or the nucleotide of claim 8, for use in inducing an anti-KLK3 immune response in a subject.

12. The recombinant *Listeria* strain of any one of claims 1 to 6, the recombinant polypeptide of claim 7, or the nucleotide of claim 8, for use in treating a kallikrein-related peptidase 3 (KLK3) protein-expressing prostate cancer in a subject, whereby the subject mounts an immune response against the KLK3 protein-expressing prostate cancer.

13. The recombinant *Listeria* strain of any one of claims 1 to 6, the recombinant polypeptide of claim 7, or the nucleotide of claim 8, for use in inhibiting or suppressing a KLK3 protein-expressing prostate cancer in a subject, whereby the subject mounts an immune response against the KLK3 protein-expressing prostate cancer, thereby inhibiting or suppressing the KLK3 protein-expressing prostate cancer in a subject.

14. The recombinant polypeptide of claim 7 made by a process comprising the step of chemically conjugating a polypeptide comprising the KLK3 peptide to a polypeptide comprising the N-terminal LLO peptide.

15. The recombinant *Listeria* strain of any one of claims 1 to 6, an immunogenic composition comprising the recombinant polypeptide of claim 7, or the nucleotide of claim 8, for use as a medicament.

## Patentansprüche

1. Rekombinanter Listeria-Stamm, der ein Fusionspeptid eines Kallikrein-Related Peptidase 3 (KLK3)-Peptids exprimiert, das in Wirkbeziehung mit einem N-terminalen nicht-hämolytischen Listeriolysin (LLO)-Peptid verbunden ist, wobei das Fusionspeptid die Sequenz der SEQ 10 NO: 54 oder eine mindestens 99 % dazu homologe Sequenz umfasst, wobei das N-terminale LLO-Peptid die Immunogenität des Fusionspeptids verstärkt, und wobei das KLK3-Peptid keine Signalsequenz enthält.

2. Rekombinanter *Listeria*-Stamm nach Anspruch 1, wobei der rekombinante *Listeria*-Stamm ein auxotropher *Listeria-* oder ein rekombinanter *Listeria monocytogenes-Stamm* ist.

3. Rekombinanter *Listeria*-Stamm nach Anspruch 2, wobei der auxotrophe *Listeria*-Stamm eine dal/dat-Mutante ist oder ferner eine Deletion im endogenen ActA-Gen umfasst.

4. Rekombinanter *Listeria*-Stamm nach Anspruch 2, wobei der auxotrophe *Listeria*-Stamm einen episomalen Expressionsvektor umfasst, der ein Stoffwechselenzym umfasst, das die Auxotrophie des auxotrophen *Listeria*-Stamms ergänzt.

5. Rekombinanter *Listeria*-Stamm nach Anspruch 4, wobei das Stoffwechselenzym ein Alanin-Racemase-Enzym oder ein D-Aminosäure-Transferase-Enzym ist.

6. Rekombinanter *Listeria*-Stamm nach Anspruch 1, wobei der rekombinante *Listeria*-Stamm durch einen tierischen Wirt passagiert worden ist.

7. Rekombinantes Polypeptid, umfassend ein Fusionspeptid eines KLK3-Peptids, das in Wirkbeziehung mit einem N-terminalen LLO-Peptid verbunden ist, wobei das rekombinante Polypeptid die Sequenz der SEQ 10 NO: 54 oder eine mindestens 99 % dazu homologe Sequenz umfasst, wobei das N-terminale LLO-Peptid die Immunogenität des Fusionspeptids verstärkt, und wobei das KLK3-Peptid keine Signalsequenz enthält.

8. Nukleotidmolekül, das für das rekombinante Polypeptid nach Anspruch 7 codiert.

9. Vakzin, das den rekombinanten *Listeria*-Stamm nach einem der Ansprüche 1 bis 6, das rekombinante Polypeptid nach Anspruch 7 oder das Nukleotidmolekül nach Anspruch 8 und ein Adjuvans umfasst.

10. Rekombinanter Vakzin-Vektor, der für das rekombinante Polypeptid nach Anspruch 7 codiert, oder das Nukleotidmolekül, nach Anspruch 8 umfasst.

11. Rekombinanter *Listeria*-Stamm nach einem der Ansprüche 1 bis 6, eine immunogene Zusammensetzung, die das rekombinante Polypeptid nach Anspruch 7 oder das Nukleotid nach Anspruch 8 umfasst, zur Verwendung bei der Induktion einer Anti-KLK3-Immunantwort in einem Individuum.

12. Rekombinanter *Listeria*-Stamm nach einem der Ansprüche 1 bis 6, das rekombinante Polypeptid nach Anspruch 7 oder das Nukleotid nach Anspruch 8 zur Verwendung beim Behandeln eines Kallikrein-Related Peptidase 3 (KLK3)-Protein-exprimierenden Prostatakarzinoms in einem Individuum, wobei das Individuum eine Immunantwort gegen das KLK3-Protein-exprimierende Prostatakarzinom entwickelt.

13. Rekombinanter *Listeria*-Stamm nach einem der Ansprüche 1 bis 6, das rekombinante Polypeptid nach Anspruch 7 oder das Nukleotid nach Anspruch 8 zur Verwendung beim Hemmen oder Unterdrücken eines KLK3-Protein-exprimierenden Prostatakarzinoms in einem Subjekt, wobei das Individuum eine Immunantwort gegen das KLK3-Protein-exprimierende Prostatakarzinom entwickelt, wodurch das KLK3-Protein-exprimierende Prostatakarzinom in einem Individuum gehemmt oder unterdrückt wird.

14. Rekombinantes Polypeptid nach Anspruch 7, hergestellt durch ein Verfahren umfassend den Schritt des chemischen Konjugierens eines Polypeptids, das das KLK3-Peptid umfasst, mit einem Polypeptid, das das N-terminale LLO-Peptid umfasst.

15. Rekombinanter *Listeria*-Stamm nach einem der Ansprüche 1 bis 6, eine immunogene Zusammensetzung, die das rekombinante Polypeptid nach Anspruch 7 oder das Nukleotid nach Anspruch 8 umfasst, zur Verwendung als Medikament.

## Revendications

1. Souche recombinée de Listeria exprimant un peptide de fusion d'un peptide peptidase 3 se rapportant à la kallikréine (KLK3) qui est en liaison fonctionnelle avec le peptide N-terminal non hémolytique listériolysine (LLO), ledit peptide de fusion comprenant la séquence de SEQ ID No. : 54 ou une séquence homologue au moins à 99 % par rapport à celle-ci, dans laquelle ledit peptide LLO N-terminal renforce l'immunogénicité du peptide de fusion, et dans laquelle le peptide KLK3 ne contient pas de séquence de signal.

2. Souche recombinée de *Listeria* selon la revendication 1, ladite souche recombinée de *Listeria* étant une souche auxotrophe de *Listeria* ou une souche recombinée de *Listeria monocytogenes.*

3. Souche recombinée de *Listeria* selon la revendication 2, dans laquelle la souche auxotrophe de *Listeria* est un mutant dal/dat ou comprend en outre une délétion dans le gène endogène ActA.

4. Souche recombinée de *Listeria* selon la revendication 2, dans laquelle la souche auxotrophe de *Listeria* comprend un vecteur d'expression épisomique comprenant une enzyme métabolique qui complète l'auxotrophie de la souche auxotrophe de *Listeria.*

5. Souche recombinée de *Listeria* selon la revendication 4, dans laquelle l'enzyme métabolique est une enzyme alanine racémase ou une enzyme acide D-aminé transférase.

6. Souche recombinée de *Listeria* selon la revendication 1, la souche recombinée de *Listeria* ayant subi des passages à travers un hôte animal.

7. Polypeptide recombiné comprenant un peptide de fusion d'un peptide KLK3 qui est en liaison fonctionnelle avec un peptide LLO N-terminal, ledit polypeptide recombiné comprenant la séquence de SEQ ID No. : 54 ou une séquence homologue au moins à 99 % par rapport à celle-ci, dans lequel ledit peptide LLO N-terminal renforce l'immunogénicité du peptide de fusion, et dans lequel le peptide KLK3 ne contient pas de séquence de signal.

8. Molécule de nucléotide codant pour le polypeptide recombiné selon la revendication 7.

9. Vaccin comprenant la souche recombinée de *Listeria* selon l'une quelconque des revendications 1 à 6, le polypeptide recombiné selon la revendication 7 ou la molécule de nucléotide selon la revendication 8, et un adjuvant.

10. Vecteur de vaccin recombiné codant pour le polypeptide recombiné selon la revendication 7 ou comprenant la molécule de nucléotide selon la revendication 8.

11. Souche recombinée de *Listeria* selon l'une quelconque des revendications 1 à 6, composition immunogène comprenant le polypeptide recombiné selon la revendication 7, ou nucléotide selon la revendication 8, destiné à être utilisé pour induire une réponse immunitaire anti-KLK3 chez un sujet.

12. Souche recombinée de *Listeria* selon l'une quelconque des revendications 1 à 6, polypeptide recombiné selon la revendication 7, ou nucléotide selon la revendication 8, destiné à être utilisé pour le traitement d'un cancer de la prostate exprimant une protéine peptidase 3 se rapportant à la kallikréine (KLK3) chez un sujet, moyennant quoi ledit sujet développe une réponse immunitaire vis-à-vis du cancer de la prostate exprimant la protéine KLK3.

13. Souche recombinée de *Listeria* selon l'une quelconque des revendications 1 à 6, polypeptide recombiné selon la revendication 7, ou nucléotide selon la revendication 8, destiné à être utilisé pour inhiber ou supprimer un cancer de la prostate exprimant la protéine KLK3 chez un sujet, moyennant quoi le sujet développe une réponse immunitaire vis-à-vis du cancer de la prostate exprimant la protéine KLK3, ce qui inhibe ou supprime le cancer de la prostate exprimant la protéine KLK3 chez un sujet.

14. Polypeptide recombiné selon la revendication 7, fabriqué par un procédé comprenant l'étape de conjugaison chimique d'un polypeptide comprenant le peptide KLK3 à un polypeptide comprenant le peptide LLO N-terminal.

15. Souche recombinée de *Listeria* selon l'une quelconque des revendications 1 à 6, composition immunogène comprenant le polypeptide recombiné selon la revendication 7, ou nucléotide selon la revendication 8, destiné à être utilisé en tant que médicament.
